# FASCICULE DE BREVET EUROPEEN

(11) **EP 0 955 299 B1**
(45) Date de publication et mention de la délivrance du brevet: **13.03.2002**
(21) Numéro de dépôt: 99401010.6
(22) Date de dépôt: 26.04.1999
(51) Int. Cl.: C07D 333/64, C07D 409/06, C07D 401/06, C07D 409/14, A61K 31/38

(54) **Nouveaux dérivés benzothiophéniques, benzofuraniques et indoliques, leur procédé de préparation et les compositions pharmaceutiques qui les contiennent**
Benzothiophen-, Benzofuran- und Indolderivate, Verfahren zu deren Herstellung und sie enthaltende pharmazeutische Zusammensetzungen
Benzothiophene, benzofurane and indole derivatives, process for their preparation and pharmaceutical compositions containing them

(30) Priorité: 27.04.1998 FR 9805239
(43) Date de publication de la demande: 10.11.1999
(73) Titulaire: LES LABORATOIRES SERVIER, 92200 Neuilly sur Seine (FR)
(72) Inventeur: De Nanteuil, Guillaume, 92150 Suresnes (FR); Lila, Christine, 78220 Viroflay (FR); Verbeuren, Tony, 78540 Vernouillet (FR); Rupin, Alain, 37510 Savonnieres (FR)

(56) Documents cités:
- GB-A- 2 225 012
- M. VEDACHALAM ET AL.: "A facile preparation of 2-alkylindoles-potential intermediates for alkaloids" TETRAHEDRON LETTERS, vol. 24, no. 33, 1983, pages 3531-3532, XP002089157 OXFORD GB
- CHEMICAL ABSTRACTS, vol. 119, no. 5, 2 août 1993 (1993-08-02) Columbus, Ohio, US; abstract no. 49178, SADANANDAN E. V. ET AL.: "2-Alkylindoles via Wittig olefination of indole-2-aldehyde" XP002089158 & INDIAN J. CHEM., SECT. B, vol. 32B, no. 4, 1993, pages 481-483,
- CHEMICAL ABSTRACTS, vol. 117, no. 25, 21 décembre 1992 (1992-12-21) Columbus, Ohio, US; abstract no. 251192, VEDACHALAM M. ET AL.: "Synthesis of 2-alkylindoles" XP002089159 & INDIAN J. CHEM., SECT. B, vol. 31B, no. 10, 1992, pages 685-687,

## Description

La présente invention concerne de nouveaux dérivés benzothiophèniques, benzofuraniques et indoliques, leur procédé de préparation et les compositions pharmaceutiques qui les contiennent. Ces nouveaux composés sont utiles pour leur activité thérapeutique dans le domaine de la fibrinolyse et de la thrombose, grâce à leur propriété inhibitrice de l'activité du PAI-1.

Le PAI-1 est un inhibiteur puissant des activateurs du plasminogène (activateur tissulaire du plasminogène et urokinase). Il provoque, in-vitro et in-vivo, l'inhibition de la lyse des caillots fibrineux formés par l'action de la thrombine sur le fibrinogène. De nombreuses études épidémiologiques ont montré que chez l'homme, des taux élevés de PAI-1 sont associés à la survenue plus fréquente de maladies thromboemboliques. De plus, dans des modèles expérimentaux de thrombose et de thrombolyse, l'inhibition de l'activité du PAI-1 par des anticorps monoclonaux anti-PAI-1 diminue l'incidence des thromboses ou des réocclusions. L'intérêt thérapeutique de molécules possédant la propriété d'inhiber l'activité du PAI-1 au sein du caillot fibrineux formé ou en formation est donc de permettre sa lyse précoce avant sa complexation avec le facteur XIIIa et ainsi de diminuer l'incidence des accidents thromboemboliques chez les patients possédant des taux élevés de PAI-1. De tels composés présentent un intérêt thérapeutique dans toutes les pathologies dont l'origine est la thrombose (tel que l'infarctus du myocarde, l'angor, la claudication intermittente, les accidents vasculaires cérébraux, la thrombose veineuse profonde, ou l'embolie pulmonaire) ainsi que pour les pathologies dans lesquelles les risques thrombotiques sont augmentés (telles que l'hypertension, l'hypercholestérolémie, le diabète, l'obésité, les anomalies génétiques de la coagulation (facteur V leiden, déficit en protéines C et S) ou les anomalies acquises de la coagulation).

Les composés de la présente invention, outre le fait qu'ils soient nouveaux, se sont révélés être des inhibiteurs du PAI-1 plus puissants que ceux décrits dans la littérature, ce qui les rend donc potentiellement utiles pour le traitement de la thrombose, ou des pathologies dont l'origine est la thrombose...

Un certain nombre d'antithrombotiques ont été décrits dans la littérature. C'est le cas, plus particulièrement, des composés décrits dans les brevets WO 97/45424, WO 94/08962, EP 540051 ou GB 2225012.

Plus spécifiquement, la présente invention concerne les composés de formule (I) : 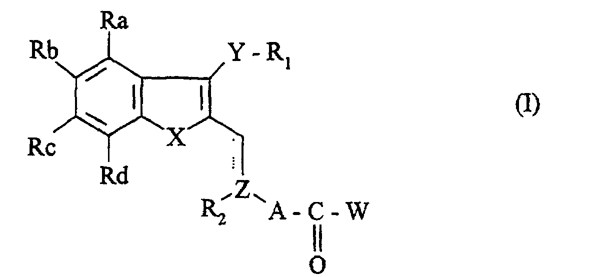 dans laquelle :
- X: représente un atome d'oxygène, de soufre, ou un groupement NR₃ dans lequel R₃ représente un atome d'hydrogène, un groupement alkyle (C₁-C₆) linéaire ou ramifié, acyle (C₁-C₆) linéaire ou ramifié, aryle, arylalkyle (C₁-C₆) linéaire ou ramifié, ou hétéroarylalkyle (C₁-C₆) linéaire ou ramifié,
- Y: représente un atome d'oxygène, de soufre, un groupement NR₃, le groupement R₃ ayant la même définition que précédemment, ou peut représenter une liaison simple quand X représente un groupement NR'₃ dans lequel R'₃ représente un groupement hétéroarylalkyle (C₁-C₆) linéaire ou ramifié,
- Z: représente un atome d'azote quand la liaison qui le relie avec l'atome de carbone voisin est simple (―), un atome de carbone ou un groupement CH selon que la liaison qui le relie avec l'atome de carbone voisin est simple (―) ou double (^{.......}),
- A: représente une liaison simple, un groupement alkylène (C₁-C₆) (éventuellement substitué par un ou plusieurs groupements alkyle (C₁-C₆) linéaire ou ramifié, aryle, arylalkyle (C₁-C₆) linéaire ou ramifié, cycloalkyle ou un hétérocycle), arylène, cycloalkylène, un hétérocycle, ou un groupement -SO₂-R₄- (la partie SO₂ étant reliée à Z) dans lequel R₄ représente un groupement alkylène (C₁-C₆) linéaire ou ramifié, arylène, arylalkylène (C₁-C₆) linéaire ou ramifié, cycloalkylène ou un hétérocycle,
- W: représente un groupement hydroxy, alkoxy (C₁-C₆) linéaire ou ramifié, aryloxy, arylalkoxy (C₁-C₆) linéaire ou ramifié, cycloalkyloxy, un hétérocycle lié à un atome d'oxygène, un groupement amino (lui-même pouvant être substitué par un ou deux groupements, identiques ou différents, indépendamment l'un de l'autre, choisis parmi alkyle (C₁-C₆) linéaire ou ramifié, aryle, arylalkyle (C₁-C₆) linéaire ou ramifié, ou cycloalkyle), ou un groupement hydroxyamino,
- Ra, Rb, Rc, Rd,: identiques ou différents, indépendamment l'un de l'autre, représentent un atome d'hydrogène, d'halogène, un groupement hydroxy, cyano, nitro, alkyle (C₁-C₆) linéaire ou ramifié, alkoxy (C₁-C₆) linéaire ou ramifié, acyle (C₁-C₆) linéaire ou ramifié, carboxy, alkoxycarbonyle (C₁-C₆) linéaire ou ramifié, trihalogénoalkyle (C₁-C₆) linéaire ou ramifié, amino (éventuellement substitué par un ou deux groupements, identiques ou différents, indépendamment l'un de l'autre, alkyle (C₁-C₆) linéaire ou ramifié, aryle, arylalkyle (C₁-C₆) linéaire ou ramifié), aryloxy, arylalkoxy (C₁-C₆) linéaire ou ramifié, hétéroaryloxy, hétéroarylalkoxy (C₁-C₆) linéaire ou ramifié,
- ou Ra+Rb, Rb+Rc ou Rc+Rd: représente un groupement de formule -U₁-V-U₂ (et dans ce cas, les groupements restants Ra, Rb, Rc ou Rd prennent l'une des définitions précédemment définies), dans laquelle :
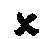 U₁, U₂, identiques ou différents, représentent un atome d'oxygène, de soufre, un groupement NH ou CH₂,
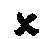 V représente un groupement alkylène (C₁-C₄) linéaire, éventuellement substitué par un ou plusieurs groupements alkyle (C₁-C₆) linéaire ou ramifié, aryle, arylalkyle (C₁-C₆) linéaire ou ramifié, hétéroaryle, hétéroarylalkyle (C₁-C₆) linéaire ou ramifié, ou V représente un groupement de formule 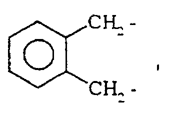
étant entendu que Ra, Rb, Rc et Rd ne peuvent pas représenter simultanément un atome d'hydrogène,
- R₁: représente :
- un groupement aryle substitué par un à cinq substituants, identiques ou différents, indépendamment l'un de l'autre, choisis parmi halogène, hydroxy, cyano, nitro, carboxy, alkyle (C₁-C₆) linéaire ou ramifié, alkoxy (C₁-C₆) linéaire ou ramifié, acyle (C₁-C₆) linéaire ou ramifié, alkoxycarbonyle (C₁-C₆) linéaire ou ramifié, trihalogénoalkyle (C₁-C₆) linéaire ou ramifié (éventuellement substitué par un groupement hydroxy), trihalogénoalkoxy (C₁-C₆) linéaire ou ramifié, amino (éventuellement substitué par un ou deux groupements alkyle (C₁-C₆) linéaire ou ramifié, un desdits groupements alkyles pouvant éventuellement être substitué par un groupement amino, alkylamino (C₁-C₆) linéaire ou ramifié, ou dialkylamino (C₁-C₆) linéaire ou ramifié), aminoalkoxy (C₁-C₆) linéaire ou ramifié (la partie amino pouvant être substitué par un ou deux groupements, identiques ou différents, alkyle (C₁-C₆) linéaire ou ramifié), alkoxycarbonylalkyle (C₁-C₆) linéaire ou ramifié, alkyl(C₁-C₆)carbonylamino linéaire ou ramifié, arylakyle (C₁-C₆) linéaire ou ramifié, aryloxy, arylalkoxy (C₁-C₆) linéaire ou ramifié, arylamino, arylalkylamino (C₁-C₆) linéaire ou ramifié, arylsulfanyl, arylalkyl(C₁-C₆)sulfanyl linéaire ou ramifié, hétéroaryle, hétéroarylalkyle (C₁-C₆) linéaire ou ramifié, hétéroaryloxy, hétéroarylalkoxy (C₁-C₆) linéaire ou ramifié, hétéroarylamino, hétéroarylalkoxy (C₁-C₆)amino linéaire ou ramifié, hétéroarylsulfanyle, hétéroarylalkyl(C₁-C₆)sulfanyl linéaire ou ramifié,
- un groupement de formule, 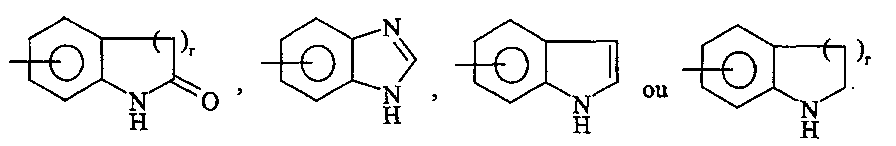 dans lesquelles r est un entier compris entre 1 et 2 inclus,
- un groupement 1-hydroxy-2(1*H*)-pyridinone,
- ou un groupement hétéroaryle éventuellement substitué,
- R₂: représente un atome d'hydrogène, un groupement alkyle (C₁-C₆) linéaire ou ramifié, aryle, arylalkyle (C₁-C₆) linéaire ou ramifié, cycloalkyle, un hétérocycle éventuellement substitué ou un hétérocycle lié à un groupement alkyle (C₁-C₆) linéaire ou ramifié,
leurs isomères ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Par groupement aryle, on entend un groupement phényle, naphtyle, tétrahydronaphtyle, ou dihydronaphtyle, chacun de ces groupements étant éventuellement substitué de façon identique ou différente, par un ou plusieurs atomes d'halogène, groupements hydroxy, cyano, nitro, alkyle (C₁-C₆) linéaire ou ramifié, trihalogénoalkyle (C₁-C₆) linéaire ou ramifié, alkoxy (C₁-C₆) linéaire ou ramifié, acyle (C₁-C₆) linéaire ou ramifié, carboxy, alkoxycarbonyle (C₁-C₆) linéaire ou ramifié, amino (substitué éventuellement par un ou deux groupements, identiques ou différents, alkyle (C₁-C₆) linéaire ou ramifié).

Par groupement cycloalkyle, on entend un groupement mono ou bicyclique, comportant de 3 à 8 atomes de carbone.

Par hétérocycle, on entend un groupement mono ou bicyclique, saturé ou insaturé, à caractère aromatique ou non aromatique, de 5 à 12 chaînons contenant un, deux ou trois hétéroatomes, identiques ou différents, choisis parmi oxygène, azote ou soufre, étant entendu que l'hétérocycle peut être éventuellement substitué, de façon identique ou différente, par un ou plusieurs atomes d'halogène, groupements hydroxy, alkyle (C₁-C₆) linéaire ou ramifié, trihalogénoalkyle (C₁-C₆) linéaire ou ramifié, alkoxy (C₁-C₆) linéaire ou ramifié, aryloxy, arylalkoxy (C₁-C₆) linéaire ou ramifié, amino (substitué éventuellement par un ou deux groupements alkyle (C₁-C₆) linéaire ou ramifié), acyle (C₁-C₆) linéaire ou ramifié, alkoxycarbonyle (C₁-C₆) linéaire ou ramifié, nitro, oxo.

Plus particulièrement, par hétéroaryle, on entend un hétérocycle, éventuellement substitué, mono ou bicyclique insaturé, dont au moins un des cycles possède un caractère aromatique. On peut citer à titre non limitatif les hétéroaryles tels que pyridine, pyrimidine, quinoline, isoquinoline, 1,3-dihydro-2*H*-pyrrolopyridine-2-one, 3*H*-imidazopyridine, 1*H*-pyrrolopyridine, 1,2,3,4-tétrahydronaphtpyridine ou 2,3-dihydro-1*H*-pyrrolopyridine.

Les composés préférés de l'invention sont ceux pour lesquels X représente un atome de soufre ou un groupement NR₃ avec R₃ tel que défini dans la formule (I).

Les composés préférés de l'invention sont ceux pour lesquels Y représente un atome d'oxygène.

Les substituants R₁ préférés selon l'invention sont les groupements choisis parmi phényle éventuellement substitué par un groupement tel que défini dans la formule (I), quinolyle éventuellement substitué, et pyridinyle éventuellement substitué.

Les substituants R₂ préférés selon l'invention sont les groupements choisis parmi aryle et hétérocycle, chacun de ces groupements étant éventuellement substitué. Selon une variante avantageuse, le substituant R₂ préféré est le groupement pyridinyle.

Selon une variante avantageuse de l'invention, les composés préférés sont ceux pour lesquels X représente un atome de soufre et Y représente un atome d'oxygène.

Selon une autre variante particulièrement avantageuse de l'invention, les composés préférés sont ceux pour lesquels :
- X représente un atome de soufre,
- Y représente un atome d'oxygène,
- R₁ représente un groupement phényle éventuellement substitué ou un groupement pyridinyle éventuellement substitué,
- A représente une liaison simple quand Z représente un atome de carbone ou un groupement CH.

Selon une troisième variante avantageuse, les composés préférés de l'invention sont ceux pour lesquels .
- X représente un atome de soufre,
- Y représente un atome d'oxygène,
- R₁ représente un groupement phényle éventuellement substitué par un groupement tel que défini dans la formule (I),
- A représente un groupement alkylène (éventuellement substitué par un groupement alkyle (C₁-C₆) linéaire ou ramifié, aryle, arylalkyle (C₁-C₆) linéaire ou ramifié) ou un groupement arylène quand Z représente un atome d'azote.

Les composés préférés selon l'invention sont :
- l'acide (E)-3-[5,6-diméthoxy-3-(4-méthoxyphénoxy)benzo[*b*]thiophène-2-yl]-2-(4-pyridinyl)-2-propènoïque,
- l'acide (E)-3-[5,6-bis(benzyloxy)-3-(4-méthoxyphénoxy)benzo[*b*]thiophène-2-yl]-2-(4-pyridinyl)-2-propènoïque,
- l'acide (E)-3-[5,6-bis(benzyloxy)-3-(4-benzyloxyphénoxy)benzo[*b*]thiophène-2-yl]-2-(4-pyridinyl)-2-propènoïque,
- l'acide (E)-3-[5,6-bis(benzyloxy)-3-(3-pyridinyloxy)benzo[*b*]thiophène-2-yl]-2-(4-pyridinyl)-2-propènoïque,
- l'acide (E)-3-[5,6-bis(benzyloxy)-3-(4-hydroxyphénoxy)benzo[*b*]thiophène-2-yl]-2-(4-pyridinyl)-2-propènoïque,
- l'acide (E)-3-{5,6-bis(benzyloxy)-3-[(6-méthyl-3-pyridinyl)oxy]benzo[*b*]thiophène-2-yl}-2-(4-pyridinyl)-2-propènoïque,
- l'acide (E)-3-{5-(benzyloxy)-3-[4-(benzyloxy)phénoxy]-6-méthoxy-benzo[*b*]thiophène-2-yl}-2-(4-pyridinyl)-2-propènoïque,
- l'acide (E)-3-[5,6-bis(benzyloxy)-3-(6-quinolinyloxy)benzo[*b*]thiophène-2-yl]-2-(4-pyridinyl)-2-propènoïque,
- l'acide (E)-3-{5,6-bis(benzyloxy)-3-[(6-méthoxy-3-pyridinyl)oxy]-benzo[*b*]thiophèn-2-yl}-2-(4-pyridinyl)-2-propènoïque,
- l'acide (E)-3-{5,6-bis(benzyloxy)-3-[4-(4-pyridinyl)phénoxy-benzo[*b*]thiophène-2-yl} -2-(4-pyridinyl)-2-propènoïque,
- et l'acide (E)-3-[5-benzyloxy-6-méthoxy-3-(3-pyridinyl)oxy-benzo[*b*]thiophène-2-yl]-2-(4-pyridinyl)-2-propènoïque.

Les isomères, ainsi que les sels d'addition à un acide ou à une base pharmaceutiquement acceptable, des composés préférés font partie intégrante de l'invention.

L'invention s'étend également au procédé de préparation des composés de formule (I), caractérisé en ce qu'on utilise comme produit de départ un composé de formule (II) : 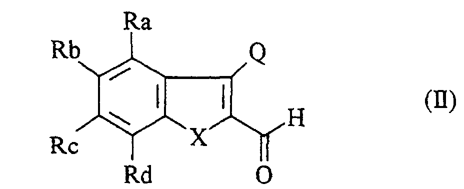 dans laquelle Ra, Rb, Rc, Rd et X ont la même signification que dans la formule (I) et Q représente un atome d'halogène ou un groupement hydroxy et de façon préférée, Q représente un atome d'halogène quand X représente un atome de soufre ou un groupement NR₃ avec R₃ tel que défini dans la formule (I) et Q représente un groupement hydroxy quand X représente un atome d'oxygène,
composé de formule (II) que l'on fait réagir, en condition basique,
- *soit quand Q représente un atome d'halogène :*
   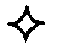 avec un composé de formule (III),

      H - Y₁ - R₁ (III)

      dans laquelle R₁ a la même signification que dans la formule (I) et Y₁ représente un atome d'oxygène, de soufre, ou un groupement NR₃ avec R₃ ayant la même signification que dans la formule (I),
      pour conduire aux composés de formule (IV/a) : 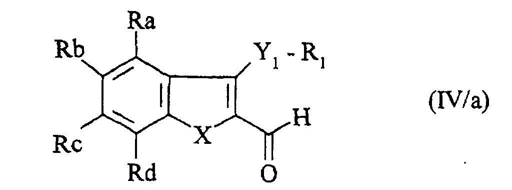 dans laquelle Ra, Rb, Rc, Rd, R₁, X et Y₁ sont tels que définis précédemment,
   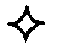 ou avec un composé de formule (V) :

      (HO)₂B - R₁ (V)

      dans laquelle R₁ a la même signification que dans la formule (I), pour conduire aux composés de formule (IV/b) : 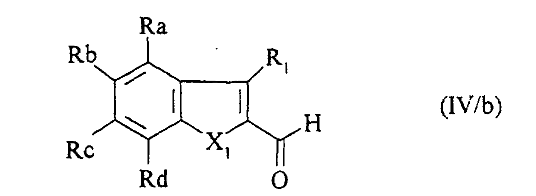 dans laquelle Ra, Rb, Rc, Rd et R₁ sont tels que définis précédemment et X₁ représente un groupement NR₃ dans lequel R₃ représente un groupement hétéroarylalkyle (C₁-C₆) linéaire ou ramifié,
- *soit quand Q représente un groupement hydroxy,* avec un composé de formule (VI),

   Hal - R₁ (VI)

   dans laquelle Hal représente un atome d'halogène et R₁ est tel que défini précédemment, pour conduire aux composés de formule (IV/c) : 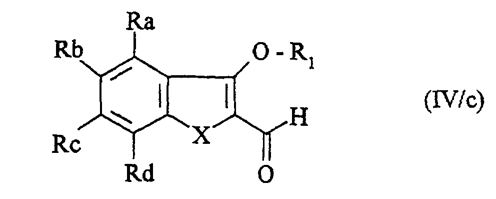 dans laquelle Ra, Rb, Rc, Rd, X et R₁ sont tels que définis précédemment,
   l'ensemble des composés de formule (IV/a), (IV/b) et (IV/c) forment les composés de formule (IV) : 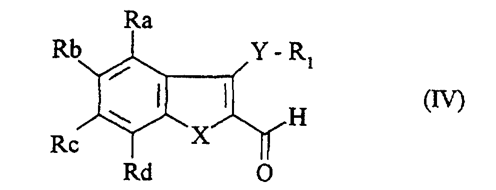 dans laquelle Ra, Rb, Rc, Rd, R₁, X et Y ont la même définition que dans la formule (I),
   composés de formule (IV):
   ◆ que l'on condense, en présence d'anhydride acétique, avec un composé de formule (VII), 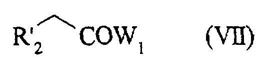 dans laquelle R'₂ a la même signification que R₂ dans la formule (I), excepté que R'₂ ne peut pas représenter un atome d'hydrogène, et W₁ représente un groupement alkoxy (C₁-C₆) linéaire ou ramifié, aryloxy, arylalkoxy (C₁-C₆) linéaire ou ramifié, cycloalkyloxy, un hétérocycle lié à un atome d'oxygène ou un groupement amino (lui-même pouvant être substitué par un ou deux groupements, identiques ou différents, indépendamment l'un de l'autre, choisis parmi alkyle (C₁-C₆) linéaire ou ramifié, aryle, arylalkyle (C₁-C₆) linéaire ou ramifié, ou cycloalkyle),
      pour conduire aux composés de formule (I/a), cas particulier des composés de formule (I) : 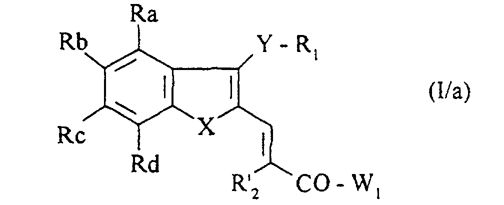 dans laquelle Ra, Rb, Rc, Rd, R₁, R'₂, X, Y et W₁ ont la même définition que précédemment, composés de formule (I/a) que l'on soumet, si on le désire :
      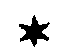 soit à des conditions d'hydrogénation catalytique, en présence de Palladium, pour conduire aux composés de formule (I/b), cas particulier des composés de formule (I) : 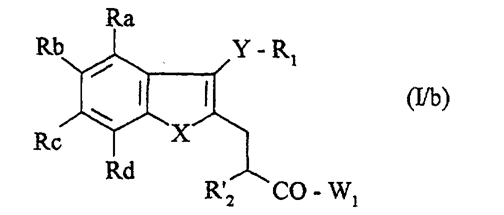 dans laquelle Ra, Rb, Rc, Rd, R₁, R'₂, X, Y et W₁ sont tels que définis précédemment,
      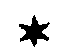 soit à des conditions d'hydrolyse, en milieu basique, pour conduire aux composés de formule (I/c), cas particulier des composés de formule (I): 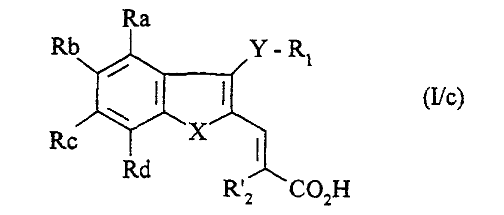 dans laquelle Ra, Rb, Rc, Rd, R₁, R'₂, X et Y ont la même définition que précédemment,
         composés de formule (I/c), dont on réduit, si on le souhaite, la double liaison par hydrogénation catalytique, pour conduire aux composés de formule (I/d), cas particulier des composés de formule (I): 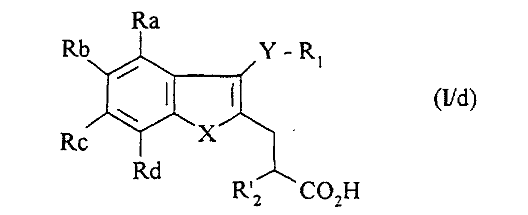 dans laquelle Ra, Rb, Rc, Rd, R₁, R'₂, X et Y sont tels que définis précédemment,
   ◆ ou que l'on soumet, à l'action d'un ylure de phosphore de formule (VIII), 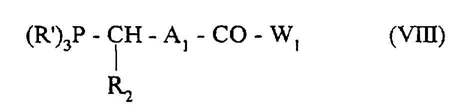 dans laquelle R' représente un groupement alkyle (C₁-C₆) linéaire ou ramifié, ou un groupement phényle, R₂ a la même définition que dans la formule (I), W₁ a la même définition que précédemment et A₁ représente une liaison simple, un groupement alkylène (éventuellement substitué par un ou plusieurs groupements alkyle (C₁-C₆) linéaire ou ramifié, aryle, arylalkyle (C₁-C₆) linéaire ou ramifié, cycloalkyle ou un hétérocycle), un groupement arylène, cycloalkylène ou un hétérocycle,
      pour conduire aux composés de formule (I/e), cas particulier des composés de formule (I) : 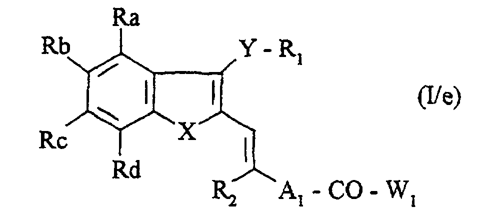 dans laquelle Ra, Rb, Rc, Rd, R₁, R₂, X, Y, A₁ et W₁ ont la même définition que précédemment,
      composés de formule (I/e) que l'on soumet, si on le désire :
      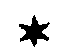 soit à des conditions d'hydrolyse, en condition basique, pour conduire aux composés de formule (I/f), cas particulier des composés de formule (I): 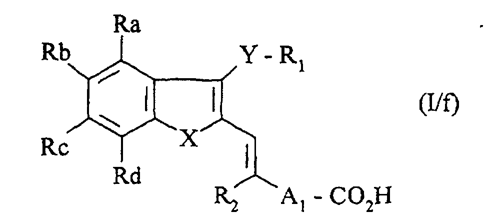 dans laquelle Ra, Rb, Rc, Rd, R₁, R₂, X, Y et A₁ sont tels que définis précédemment,
      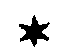 soit à des conditions d'hydrogénation catalytique, pour conduire aux composés de formule (I/g), cas particulier des composés de formule (I) : 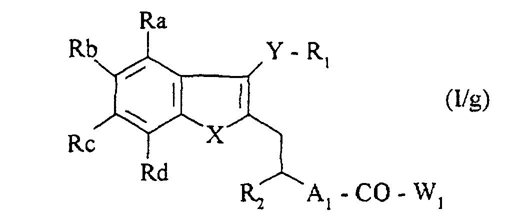 dans laquelle Ra, Rb, Rc, Rd, R₁, R₂, X, Y, A₁ et W₁ sont tels que définis précédemment,
         composés de formule (I/g) que l'on peut traiter dans des conditions d'hydrolyse basique, pour conduire aux composés de formule (I/h), cas particulier des composés de formule (I) : 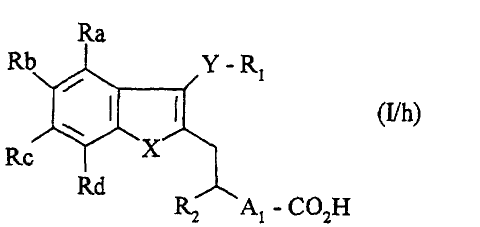 dans laquelle Ra, Rb, Rc, Rd, R₁, R₂, X, Y et A₁ sont tels que définis précédemment,
   ◆ ou, dont on réduit la fonction aldéhyde en alcool primaire, pour conduire aux composés de formule (IX) : 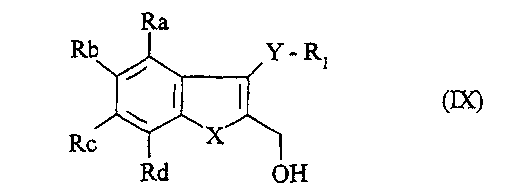 dans laquelle Ra, Rb, Rc, Rd, R₁, X et Y ont la même définition que dans la formule (I),
      composés de formule (IX) dont on substitue l'hydroxy terminal par un halogène, selon des conditions classiques, pour conduire aux composés de formule (X) : 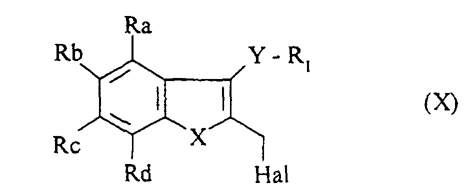 dans laquelle Ra, Rb, Rc, Rd, R₁, X et Y sont tels que définis précédemment, et Hal représente un atome de chlore ou de brome,
      composés de formule (X) :
      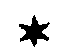 dont on substitue l'atome d'halogène, en condition basique, par un dérivé aminé de formule (XI):

         R₂ - NH - A₁ - CO - W₁ (XI)

         dans laquelle R₂ a la même définition que dans la formule (I) et, A₁, W₁ ont la même signification que précédemment,
         pour conduire aux composés de formule (I/i), cas particulier des composés de formule (I) . 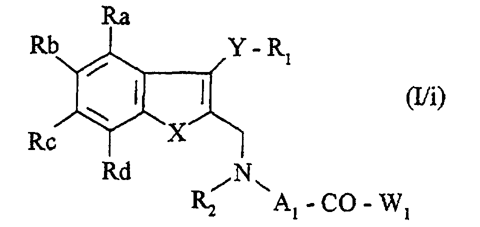 dans laquelle Ra, Rb, Rc, Rd, R₁, R₂, X, Y, A₁ et W₁ sont tels que définis précédemment,
         composés de formule (I/i), dont on hydrolyse en condition basique le groupement carbonyle terminal, pour conduire aux composés de formule (I/j), cas particulier des composés de formule (I): 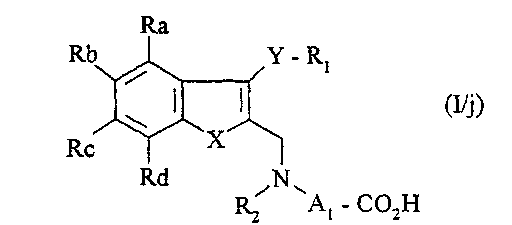 dans laquelle Ra, Rb, Rc, Rd, R₁, R₂, X, Y et A₁ sont tels que définis précédemment,
      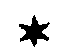 ou que l'on traite par de l'azidure de sodium dans un premier temps, et dont on réduit l'azide obtenue en amine primaire dans des conditions d'hydrogénation catalytique, pour conduire aux composés de formule (XII) : 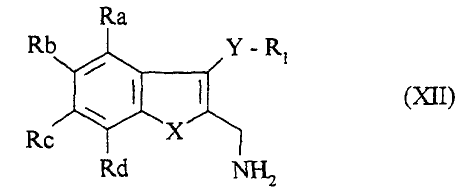 dans laquelle Ra, Rb, Rc, Rd, R₁, X et Y ont la même définition que dans la formule (I),
         composés de formule (XII) que l'on condense, en condition basique, sur un dérivé chlorosulphonyle de formule (XIII) :

         Cl-SO₂-R₄-CO-W₁ (XIII)

         dans laquelle R₄ a la même définition que dans la formule (I), et W₁ est tel que défini précédemment, pour conduire aux composés de formule (I/k), cas particulier des composés de formule (I), 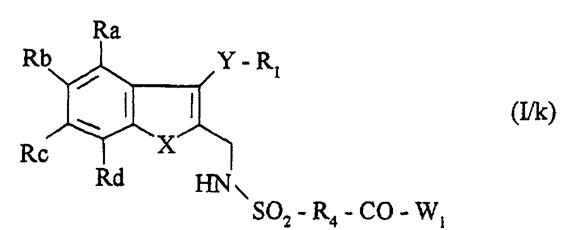 dans laquelle Ra, Rb, Rc, Rd, R₁, R₄, X, Y et W₁ sont tels que définis précédemment,
      composés de formule (I/k) :
      - que l'on soumet, si on le souhaite, à des conditions d'hydrolyse en condition basique, pour conduire aux composés de formule (I/l), cas particulier des composés de formule (I) : 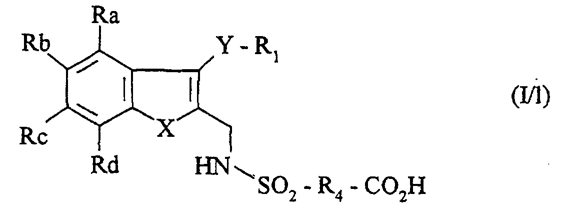 dans laquelle Ra, Rb, Rc, Rd, R₁, R₄, X et Y sont tels que définis précédemment,
      - ou que l'on condense, en milieu basique, avec un composé de formule (XIV) :

         Hal - R'₂ (XIV)

         dans laquelle Hal représente un atome d'halogène tel que l'iode, et R'₂ a la même définition que précédemment,
         pour conduire aux composés de formule (I/m), cas particulier des composés de formule (I) : 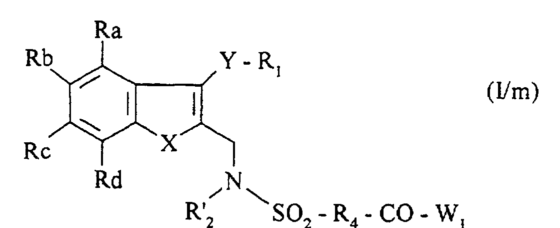 dans laquelle Ra, Rb, Rc, Rd, R₁, R'₂, R₄, X, Y et W₁ sont tels que définis précédemment,
         composés de formule (I/m) que l'on traite par des conditions d'hydrolyse en milieu basique, pour conduire aux composés de formule (I/n), cas particulier des composés de formule (I): 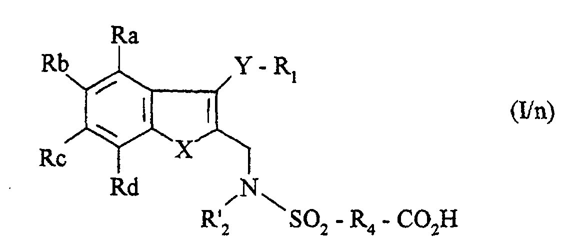 dans laquelle Ra, Rb, Rc, Rd, R₁, R'₂, R₄, X et Y sont tels que définis précédemment,
      l'ensemble des composés de formule (I/c), (I/d), (I/f), (I/h), (I/j), (I/l) et (I/n) formant les composés de formule (I'): 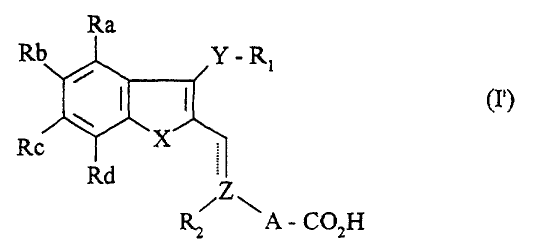 dans laquelle Ra, Rb, Rc, Rd, R₁, R₂, X, Y, Z et A sont tels que définis dans la formule (I),
      composés de formule (I') que l'on met en réaction avec une hydroxylamine-O-substituée, pour conduire, après déprotection de la fonction hydroxylamine, aux composés de formule (I/o), cas particulier des composés de formule (I): 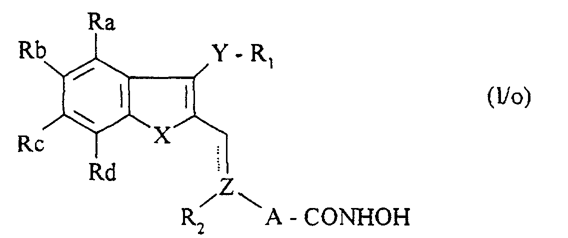 dans laquelle Ra, Rb, Rc, Rd, R₁, R₂, X, Y, Z et A sont tels que définis précédemment,
      les composés (I/a) à (I/o) formant l'ensemble des composés de l'invention, que l'on purifie, le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

Les composés de formule (II) sont obtenus selon des méthodes classiques de synthèse organique. Notamment les composés de formule (II) dans laquelle X représente un atome d'oxygène et Q un groupement hydroxy, sont obtenus à partir de composés de formule (II/A): 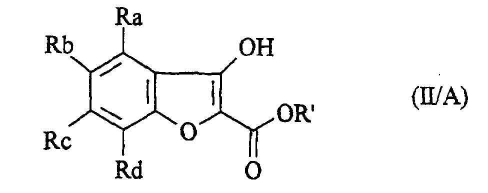 dont le schéma de synthèse est décrit dans *J. Med. Chem.,* 1992, 35, 958-965, et dans laquelle Ra, Rb, Rc, Rd ont les mêmes définitions que dans la formule (I) et R' représente un groupement alkyle (C₁-C₆) linéaire ou ramifié,
et dont on protège la fonction hydroxy, en condition basique, par un groupement trialkylsilyle, puis dont on réduit la fonction ester en fonction alcool primaire par action de LiAlH₄ par exemple, cette dernière étant ensuite oxydée en fonction aldéhyde, puis dont on déprotège la fonction alcool sous l'action de nBu₄NF, permettant d'obtenir les composés particuliers de formule (II) dans laquelle X représente un atome d'oxygène et Q représente un groupement hydroxy.

Les composés particuliers de formule (II) dans laquelle X représente un groupement NR₃ sont obtenus à partir de composés de formule (II/B) : 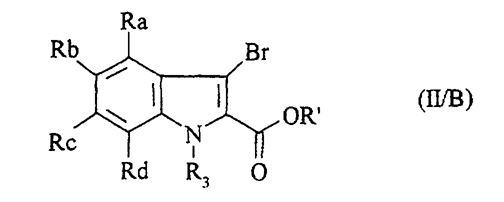 dont le schéma de synthèse est décrit dans *Heterocycles*, 1992, 34 (12), 2349-62 et *Synthesis*, 1984, 862-865, et dans laquelle Ra, Rb, Rc, Rd, R₃ ont les mêmes définitions que dans la formule (I) et R' représente un groupement alkyle (C₁-C₆) linéaire ou ramifié,
et dont la fonction ester est réduite en fonction alcool primaire, celle-ci étant ensuite oxydée sous l'action du dioxyde de Manganèse en fonction aldéhyde, pour conduire aux composés de formule (II) dans laquelle X représente un groupement NR₃ et Q un atome d'halogène.

Les composés particuliers de formule (II) dans laquelle X représente un atome de soufre sont obtenus à partir de composés de formule (II/C): 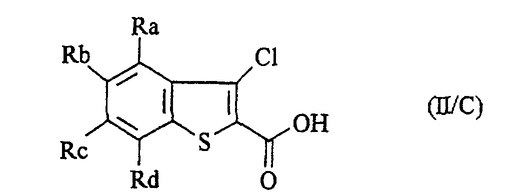 dont le schéma de synthèse est décrit dans *J*. *Heterocyclic. Chem.,* 1971, 8, 711-714, et dans laquelle Ra, Rb, Rc, et Rd ont les mêmes définitions que précédemment, et dont la fonction acide carboxylique est d'abord réduite en alcool primaire puis oxydée en aldéhyde pour conduire aux composés de formule (II) dans laquelle X représente un atome de soufre et Q un atome d'halogène.

Les composés de formule (III), (IV), (VII), (VIII), (XI), (XIII) et (XIV) sont soit des composés commerciaux, soit obtenus selon les méthodes classiques de synthèse organique.

La présente invention a également pour objet les compositions pharmaceutiques renfermant comme principe actif au moins un composé de formule (I), ses isomères optiques ou un de ses sels d'addition à une base ou un acide pharmaceutiquement acceptable, seul ou en combinaison avec un ou plusieurs excipients ou véhicules inertes non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les sachets, les gélules, les tablettes, les suppositoires, les crèmes, les pommades, les gels dermiques, les préparations injectables ou buvables, les aérosols, les gouttes oculaires ou nasales, etc...

La posologie utile varie selon l'âge et le poids du patient, la voie d'administration, la nature et la sévérité de l'affection et la prise de traitements éventuels associés et s'échelonne de 0,1 mg à 1 g en une ou plusieurs prises par jour.

Les exemples suivants illustrent l'invention mais ne la limitent en aucune façon.

Les produits de départ utilisés sont des produits connus ou préparés selon des modes opératoires connus.

Les différents stades de synthèse conduisent à des intermédiaires de synthèse, utiles pour la préparation des composés de l'invention.

Les structures des composés décrits dans les exemples et les stades de synthèse ont été déterminées selon les techniques spectrophotométriques usuelles (infrarouge, RMN, spectromètre de masse, ...).

### EXEMPLE 1 : (E)-3-[5,6-Diméthoxy-3-(4-méthoxyphénoxy)benzo[b]thiophène-2-yl]-2-(4-pyridinyl)-2-propènoate d'éthyle

### Stade A : Chlorure de 3-chloro-5,6-diméthoxy-benzo[b]thiophène-2-carbonyl

A une suspension de 0,25 ml d'acide 3,4-diméthoxycinnamique dans 350 ml de chlorobenzène, sont additionnés successivement à température ambiante 0,025 mol de pyridine, et goutte à goutte 1,27 mol de SOCl₂. Le milieu réactionnel est ensuite porté au reflux pendant 2 jours. Après retour à température ambiante, un précipité se forme. Après filtration, rinçage à l'hexane et séchage, on obtient 53,7 g du produit attendu.
*Point de fusion : 202°C*

### Stade B : Acide 3-chloro-5,6-diméthoxy-benzo[b]thiophène-2-carboxylique

A une solution de 70 mmol du composé du stade A dans 250 ml de dioxanne, est ajouté 40 ml d'eau. Après 20 heures de reflux, puis retour à température ambiante, un précipité se forme. Après filtration, rinçage à l'eau jusqu'à neutralité, le précipité est séché sur P₂O₅ sous pression réduite. On isole ainsi 18,6 g du produit attendu.
*Point de fusion : > 260°C*

### Stade C : (3-Chloro-5,6-diméthoxy-benzo[b]thiophène-2-yl)méthanol

A une solution de 0,1 mol de LiAlH₄ dans 60 ml de tétrahydrofurane, sous atmosphère inerte, est additionné à 5°C une suspension de 68 mmol du composé obtenu dans le stade B dans 150 ml de tétrahydrofurane. Après 4 heures de réaction à température ambiante, la réaction est hydrolysée par addition d'eau puis par une solution aqueuse 2N de soude. Après 12 heures à température ambiante, le mélange réactionnel est filtré sur célite. La phase organique est alors concentrée sous pression réduite, reprise par du dichlorométhane, lavée à l'eau puis par une solution saturée en NaCI. Après séchage de la phase organique sur sulfate de calcium, la solution est concentrée sous pression réduite permettant d'obtenir 15,7 g du produit attendu.
*Point de fusion : 164°C*

### Stade D : 3-Chloro-5,6-diméthoxy-benzo[b]thiophène-2-carbaldéhyde

A une suspension de 60,4 mmol du composé obtenu dans le stade C dans 360 ml de toluène, est additionné à température ambiante sous atmosphère inerte 96 mmol (1,6 équivalents) de MnO₂. Après 6 heures de réaction à 80°C, on ajoute au milieu réactionnel 0,6 équivalent de MnO₂ puis après 6 nouvelles heures, 0,6 équivalent de MnO₂. Le milieu réactionnel est ensuite filtré à chaud sur célite et rinçé au toluène. Après 12 heures à température ambiante, le filtrat précipite. Une filtration du précipité suivie d'un rinçage au toluène puis au pentane permet d'isoler 8,65 g du produit attendu.
*Point de fusion : 280°C*

### Stade E : 5,6-Diméthoxy-3-(4-méthoxyphénoxy)benzo[b]thiophène-2-carbaldéhyde

A une solution de 0,036 mol de 4-méthoxyphénol dans 250 ml de diméthylformamide, sont additionnés à température ambiante et sous atmosphère inerte 1,1 équivalents d'hydrure de sodium, puis 0,033 mol du produit obtenu dans le stade D. Après 12 heures de réaction, le milieu réactionnel est concentré sous pression réduite. Le résidu est alors dilué dans l'acétate d'éthyle, lavé à l'eau puis par une solution aqueuse de NaCl, séché sur sulfate de calcium, filtré et concentré sous pression réduite. Une chromatographie sur gel de silice (Dichlorométhane/Acétate d'éthyle : 98/2) permet d'isoler 8,6 g du produit attendu.
*Point de fusion : 138°C*

### Stade F : (E)-3-[5,6-Diméthoxy-3-(4-méthoxyphénoxy)benzo[b]thiophène-2-yl]-2-(4-pyridinyl)-2-propènoate d'éthyle

Une solution contenant 6 mmol du produit obtenu dans le stade E, 30 mmol de 4-pyridylacétate d'éthyle et 5 ml d'anhydride acétique est portée à 100°C pendant 18 heures. Après retour à température ambiante, la réaction est hydrolysée par une solution saturée en NaHCO₃, extraite à l'acétate d'éthyle. Les phases organiques sont ensuite lavées à l'eau, puis par une solution de NaCl, séchées sur sulfate de calcium, filtrées et concentrées sous pression réduite. Une chromatographie sur gel de silice (dichlorométhane/éthanol : 98/2) permet d'isoler le produit attendu.
*Point de fusion : 164°C*

### EXEMPLE 2 : Acide (E)-3-[5,6-diméthoxy-3-(4-méthoxyphénoxy)benzo[b]thiophène-2-yl]-2-(4-pyridinyl)-2-propènoïque

Une solution contenant 3 mmol du produit de l'exemple 1, 6 ml d'une solution aqueuse IN de soude et 20 ml d'éthanol est portée au reflux pendant 2 heures. Après retour à température ambiante, la réaction est concentrée sous pression réduite, puis le résidu est dilué dans de l'eau, puis repris à l'éther éthylique. La phase organique est alors acidifiée par addition de 6 ml d'une solution d'HCI 1N. Un précipité se forme, celui-ci étant filtré, rincé à l'eau, puis séché sous pression réduite, permettant d'obtenir 1,3 g du composé attendu.
*Point de fusion* : 208°C

### EXEMPLE 3 : (E)-3-[5,6-Diméthoxy-3-(4-méthoxyphénoxy)benzo[b]thiophène-2-yl]-2-(4-pyridinyl)-2-propènoate de sodium

A une suspension de 1,16 g du produit de l'exemple 2 dans 2,5 ml de soude IN est ajoutée de l'eau jusqu'à dilution totale. Une lyophilisation permet d'isoler 1,22 g du produit attendu.
*IR : 1630-1575 cm*^{*-1*} *(v*_{*s*} *(C=O) ; v*_{*s*} *(C=C) ; v*_{*s*} *(C=N))*

### EXEMPLE 4 : (E)-3-{3-[4-(Benzyloxy)phénoxy]-5,6-diméthoxy-benzo[b]thiophène-2-yl}-2-(4-pyridinyl)-2-propènoate de sodium

On procède comme dans l'exemple 1 en utilisant au stade E comme réactif le 4-benzyloxyphénol, puis comme les exemples 2 et 3.
*Spectre de Masse : FAB : [M+H]*^{*+*} *: m/z = 562 ; [M+Na]*^{*+*} *: m/z = 584*

### EXEMPLE 5 : (E)-3-{5,6-Diméthoxy-3-[(6-méthoxy-2-naphtyl)-oxy]benzo[b] thiophène-2-yl}-2-(4-pyridinyl)-2-propènoate de sodium

On procède comme dans l'exemple 4 en utilisant comme réactif au stade E le 6-méthoxy-2-naphtol.

| *Microanalyse élémentaire :* | | | | |
|---|---|---|---|---|
| | *C%* | *H%* | *N%* | *S%* |
| *calculée* | *65,04* | *4,14* | *2,62* | *5,99* |
| *trouvée* | *64,11* | *4,09* | *3,42* | *5,56* |

### EXEMPLE 6 : (E)-3-[5,6-Diméthoxy-3-(4-méthoxyphénoxy)-benzo[b]thiophène-2-yl]-2-phényl-2-propènoate de sodium

On procède comme dans l'exemple 1 en utilisant au stade F, comme réactif, l'acide phényl éthanoïque, puis comme dans l'exemple 3.

| *Microanalyse élémentaire :* | | | |
|---|---|---|---|
| | *C%* | *H%* | *S%* |
| *calculée* | *64,46* | *4,37* | *6,62* |
| *trouvée* | *63,81* | *4,25* | *5,98* |

### EXEMPLE 7 : Acide (E)-3-{5,6-diméthoxy-3-[(6-méthoxy-2-naphtyl)-oxy]benzo[b] thiophène-2-yl]-2-phényl-2-propènoïque

On procède comme dans l'exemple 1 en utilisant au stade E le réactif de l'exemple 5, puis au stade F le réactif de l'exemple 6.
*Point de fusion : 274°C*

| *Microanalyse élémentaire :* | | | |
|---|---|---|---|
| | *C%* | *H%* | *S%* |
| *calculée* | *70,30* | *4,72* | *6,26* |
| *trouvée* | *70,36* | *4,77* | *6,11* |

### EXEMPLE 8 : Acide 3-[5,6-diméthoxy-3-(4-méthoxyphénoxy)-benzo[b]thiophène-2-yl]-2-phényl-propanoïque

On utilise comme substrat le composé obtenu dans l'exemple 6 après le stade F, que l'on traite par un courant d'hydrogène en présence de Pd/C à 10 % dans le méthanol, pendant 24 heures. Une filtration, en fin de réaction, suivie d'une chromatographie sur gel de silice permet d'isoler le produit attendu.

| *Microanalyse élémentaire :* | | | |
|---|---|---|---|
| | *C%* | *H%* | *S%* |
| *calculée* | *67,23* | *5,21* | *6,90* |
| *trouvée* | *67,02* | *5,22* | *6,75* |

### EXEMPLE 9 : (E)-3-[5,6-Diméthoxy-3-(4-méthoxyphénoxy)-benzo[b]thiophène-2-yl]-propènoate d'éthyle

A une suspension de 0,02 mol de bromure de (carboéthoxyméthyl)triphénylphosphonium dans 90 ml de tétrahydrofurane, sous atmosphère inerte, est additionnée goutte à goutte, à 0°C, 20 ml d'une solution de tertio-butylate de potassium 1M dans le tétrahydrofurane. Après addition complète et retour à température ambiante, on ajoute 0,01 mol du composé obtenu dans le stade E de l'exemple 1 dilué dans 30 ml de tétrahydrofurane. Après 12 heures, le milieu réactionnel est hydrolysé par addition de 100 ml d'une solution HCI 1N, puis extrait à l'acétate d'éthyle ; les phases organiques rassemblées sont lavées à l'eau, puis par une solution saturée en NaCI, séchées sur sulfate de sodium, filtrées et concentrées sous pression réduite. Une chromatographie sur gel de silice (Pentane/Acétate d'éthyle : 90/10) permet d'isoler 2,78 g du produit attendu.
*Point de fusion : 110°C*

| *Microanalyse élémentaire :* | | | |
|---|---|---|---|
| | *C%* | *H%* | *S%* |
| *calculée* | *63,75* | *5,35* | *7,74* |
| *trouvée* | *63,76* | *5,42* | *7,33* |

### EXEMPLE 10 : Acide (E)-3-[5,6-diméthoxy-3-(4-méthoxyphénoxy)-benzo[b]thiophène-2-yl]-2-propènoïque

On procède comme dans l'exemple 2 en utilisant comme substrat le produit obtenu dans l'exemple 9

| *Microanalyse élémentaire :* | | | |
|---|---|---|---|
| | *C%* | *H%* | *S%* |
| *calculée* | *62,17* | *4,69* | *8,30* |
| *trouvée* | *62,15* | *4,69* | *8,46* |

### EXEMPLE 11 : (E)-3-[5,6-Diméthoxy-3-(4-méthoxyphénoxy)-benzo[b]thiophène-2-yl] propènoate de sodium

On procède comme dans l'exemple 3 en utilisant comme substrat le produit obtenu dans l'exemple 10.

| *Microanalyse élémentaire :* | | | |
|---|---|---|---|
| | *C%* | *H%* | *S%* |
| *calculée* | *58,82* | *4,20* | *7,85* |
| *trouvée* | *58,86* | *4,25* | *7,38* |

### EXEMPLE 12 : 3-[5,6-Diméthoxy-3-(4-méthoxyphénoxy)-benzo[b]thiophène-2-yl]-propanoate d'éthyle

Une solution contenant 0,8 mmol du produit obtenu dans l'exemple 9 dans 20 ml de méthanol, et 0,2 g de Pd/C 10 % est maintenue pendant 24 heures sous courant d'hydrogène à 40°C. Après filtration et concentration sous pression réduite, une chromatographie sur gel de silice (Cyclohexane/Acétate d'éthyle : 80/20) permet d'isoler le produit attendu.

### EXEMPLE 13 : 3-[5,6-Diméthoxy-3-(4-méthoxyphénoxy)-benzo[b]thiophène-2-yl]-propanoate de sodium

On procède comme dans l'exemple 2, puis comme dans l'exemple 3 en utilisant comme substrat le produit obtenu dans l'exemple 12.

| *Microanalyse élémentaire :* | | | |
|---|---|---|---|
| | *C%* | *H%* | *S%* |
| *calculée* | *58,53* | *4,67* | *7,81* |
| *trouvée* | *58,85* | *4,70* | *7,81* |

### EXEMPLE 14 : 2-({[5,6-Diméthoxy-3-(4-méthoxyphénoxy)benzo[b]thiophène-2-yl] méthyl}anilino)acétate d'éthyle

### Stade G : (5,6-Diméthoxy-3-(4-méthyloxyphénoxy)-benzo[b]thiophène-2-yl)méthanol

A une solution de 24 mmol du composé obtenu dans le stade E de l'exemple 1 dans 100 ml de méthanol est additionné à température ambiante 26 mmol de NaBH₄. Après 2 heures de réaction, un équivalent de NaBH₄ est ajouté au milieu réactionnel. Après 12 heures de réaction, la solution est concentrée, puis diluée à l'acétate d'éthyle, lavée par une solution HCI 1N, puis par de l'eau, puis par une solution saturée en NaCI, puis séchée sur sulfate de calcium, filtrée et concentrée sous pression réduite. Une chromatographie sur gel de silice (Dichlorométhane/Acétate d'éthyle : 95/5) permet d'isoler 6,07 g du produit attendu.
*Point de fusion : 132°C*

### Stade H : [2-Chlorométhyl-5,6-diméthoxy-3-(4-méthyloxyphénoxy)]-benzo[b]thiophène

A 4 mmol du composé du stade G dilué dans 10 ml de dichlorométhane est additionné, goutte à goutte et à 0°C, 0,63 ml de SOCl₂. Après retour à température ambiante, puis chauffage au reflux du dichlorométhane pendant 6 heures, le milieu réactionnel est concentrée sous pression réduite, permettant d'obtenir 1,6 g du produit attendu sous forme d'huile.

| *Microanalyse élémentaire :* | | | | |
|---|---|---|---|---|
| | *C%* | *H%* | *S%* | *Cl%* |
| *calculée* | *59,26* | *4,70* | *8,79* | *9,72* |
| *trouvée* | *58,90* | *4,75* | *8,85* | *11,52* |

### Stade I : 2-({[5,6-Diméthoxy-3-(4-méthoxyphénoxy)benzo[b]thiophène-2-yl] méthyl}anilino) acétate d'éthyle

Une solution contenant 6,5 mmol du composé obtenu dans le stade H, dans 16 ml de diméthylformamide, 1,5 équivalents de N-phénylglycine éthyl ester et 1,5 équivalents de K₂CO₃ est portée à 80°C pendant 18 heures. Après évaporation du solvant, le résidu est dilué à l'acétate d'éthyle et la phase organique est lavée à l'eau, puis par une solution saturée en NaCI, séchée sur sulfate de calcium, filtrée et évaporée sous pression réduite. Une chromatographie sur gel de silice (Toluène/Acétate d'éthyle : 98/2) permet d'isoler 2,56 g du produit attendu sous forme d'huile.

### EXEMPLE 15 : Acide 2-({[5,6-diméthoxy-3-(4-méthyloxyphénoxy)benzo[b]thiophène-2-yl]méthyl}anilino)éthanoïque

On procède comme dans l'exemple 2 en utilisant comme substrat le produit obtenu dans le stade I de l'exemple 14.

| *Microanalyse élémentaire :* | | | | |
|---|---|---|---|---|
| | *C%* | *H%* | *N%* | *S%* |
| *calculée* | *65,12* | *5,25* | *2,92* | *6,69* |
| *trouvée* | *65,53* | *5,47* | *3,02* | *6,33* |

### EXEMPLE 16 : 2-({[5,6-Diméthoxy-3-(4-méthyloxyphénoxy)benzo[b]thiophène-2-yl] méthyl}anilino)acétate de sodium

On procède comme dans l'exemple 3 en utilisant comme substrat le composé obtenu dans l'exemple 15.

| *Microanalyse élémentaire :* | | | | |
|---|---|---|---|---|
| | *C%* | *H%* | *N%* | *S%* |
| *calculée* | *62,03* | *4,77* | *2,89* | *6,12* |
| *trouvée* | *62,27* | *4,82* | *2,79* | *6,39* |

### EXEMPLE 17 : Acide 2-(benzyl{[5,6-diméthoxy-3-(4-méthoxyphénoxy) benzo[b]thiophène-2-yl}amino)acétique

On procède comme dans l'exemple 14 en utilisant au stade I, comme réactif, le N-benzylglycine éthyl ester, puis comme dans l'exemple 15.

### EXEMPLE 18 : 2-(Benzyl{[5,6-diméthoxy-3-(4-méthoxyphénoxy)benzo[b]thiophène-2-yl]}amino)acétate de sodium

On procède comme dans l'exemple 16 en utilisant comme substrat le produit obtenu dans l'exemple 17.

| *Microanalyse élémentaire :* | | | | |
|---|---|---|---|---|
| | *C%* | *H%* | *N%* | *S%* |
| *calculée* | *62,90* | *5,08* | *2,72* | *6,22* |
| *trouvée* | *63,63* | *5,25* | *2,81* | *6,21* |

### EXEMPLE 19: 2-({[5,6-Diméthoxy-3-(4-méthoxyphénoxy)benzo[b]thiophène-2-yl] méthyl}amino)acétate de sodium

On procède comme dans l'exemple 14 en utilisant au stade I, comme réactif, le benzylate de glycine, puis comme dans le protocole de l'exemple 15 et de l'exemple 16.

| *Microanalyse élémentaire :* | | | | |
|---|---|---|---|---|
| | *C%* | *H%* | *N%* | *S%* |
| *calculée* | *56,46* | *4,74* | *3,29* | *7,54* |
| *trouvée* | *56,88* | *4,70* | *3,24* | *6,95* |

### EXEMPLE 20 : 2-({[5,6-Diméthoxy-3-(4-méthoxyphénoxy)benzo[b]thiophène-2-yl] méthyl}amino)benzoate de sodium

On procède comme dans l'exemple 19, en utilisant au stade I, comme réactif, l'anthranilate d'éthyle.
*Point de fusion : 250°C*
*Spectre de masse : ESI* ^{*±*} *: [M+H]*⁺ *= 488 ; [M+Na]*^{*+*} *= 510*

### EXEMPLE 21 : 4-({[5,6-Diméthoxy-3-(4-méthoxyphénoxy)benzo[b]thiophène-2-yl] méthyl}amino)benzoate de sodium

On procède comme dans l'exemple 19, en utilisant au stade I, comme réactif, le 4-aminobenzoate d'éthyle.

| *Microanalyse élémentaire :* | | | | |
|---|---|---|---|---|
| | *C%* | *H%* | *N%* | *S%* |
| *calculée* | *61,59* | *4,55* | *2,87* | *6,58* |
| *trouvée* | *60,95* | *4,31* | *2,99* | *6,54* |

### EXEMPLE 22 : 3-({[5,6-Diméthoxy-3-(4-méthoxyphénoxy)benzo[b]thiophène-2-yl] méthyl}amino)benzoate de sodium

On procède comme dans l'exemple 19, en utilisant au stade I, comme réactif, le 3-aminobenzoate de méthyle.

| *Microanalyse élémentaire :* | | | | |
|---|---|---|---|---|
| | *C%* | *H%* | *N%* | *S%* |
| *calculée* | *61,59* | *4,55* | *2,87* | *6,58* |
| *trouvée* | *62,13* | *4,61* | *2,85* | *5,87* |

### EXEMPLE 23 : 2(S)-({[5,6-Diméthoxy-3-(4-méthoxyphénoxy)benzo[b]thiophène-2-yl] méthyl}amino)-3-phényl propionate de sodium

On procède comme dans l'exemple 19, en utilisant au stade I, comme réactif, le benzyl (S)-phénylalanine.

| *Microanalyse élémentaire :* | | | | |
|---|---|---|---|---|
| | *C%* | *H%* | *N%* | *S%* |
| *calculée* | *62,90* | *5,08* | *2,72* | *6,22* |
| *trouvée* | *62,34* | *5,12* | *2,76* | *5,72* |

### EXEMPLE 24 : 2-[({[5,6-Diméthoxy-3-(4-méthoxyphénoxy)benzo[b]thiophène-2-yl] méthyl}amino)sulfonyl]benzoate de méthyle

### Stade J : [2-Azidométhyl-5,6-diméthoxy-3-(4-méthoxyphénoxy)]-benzo[b]thiophène

Une solution contenant 41 mmol du composé obtenu dans le stade H de l'exemple 14 et 78 mmol d'azidure de sodium dans 80 ml de diméthylformamide est agitée à température ambiante pendant 48 heures. Le milieu réactionnel est ensuite concentré sous pression réduite. Le résidu est dilué à l'acétate d'éthyle, lavé à l'eau puis par une solution saturée en NaCI. La phase organique est ensuite séchée sur sulfate de calcium, filtrée et évaporée, permettant d'obtenir 15,4 g du produit attendu sous forme d'huile.

### Stade K : [2-Aminométhyl-5,6-diméthoxy-3-(4-méthoxyphénoxy)]-benzo[b]thiophène

Une solution contenant 41 mmol du composé obtenu dans le stade J et 1 g de Pd/C dans 6,5 ml de chloroforme et 300 ml de méthanol anhydre est placée sous atmosphère d'hydrogène à température ambiante. Après 12 heures, la réaction est filtrée puis concentrée sous pression réduite permettant d'obtenir 14,5 g du produit attendu.
*Point de fusion : 245°C*

### Stade L : 2-[({[5,6-Diméthoxy-3-(4-méthoxyphénoxy)benzo[b]thiophène-2-yl] méthyl}amino)sulfonyl]benzoate de méthyle

Une solution contenant 6,5 mmol du composé obtenu dans le stade K, 6,5 mmol de 2-(chlorosulfonyl)-benzoate de méthyl, 15,7 mmol de N-méthyl-morpholine, dans 50 ml de dichlorométhane est agitée à température ambiante. Après 12 heures, la réaction est lavée à l'eau, puis par une solution saturée en NaCl, séchée sur sulfate de sodium et concentrée sous pression réduite. Une chromatographie sur gel de silice (Dichlorométhane/Acétate d'éthyle 98/2) permet d'isoler 1,1 g du produit attendu.

### EXEMPLE 25 : Acide 2-[({[5,6-diméthoxy-3-(4-méthoxyphénoxy)benzo[b]thiophène-2-yl]méthyl}amino)sulfonyl]benzoïque

On procède comme dans l'exemple 2, en utilisant comme substrat, le composé obtenu dans le stade L de l'exemple 24.

| *Microanalyse élémentaire :* | | | | |
|---|---|---|---|---|
| | *C%* | *H%* | *N%* | *S%* |
| *calculée* | *50,70* | *4,38* | *2,64* | *12,11* |
| *trouvée* | *56,79* | *4,77* | *2,66* | *11,86* |

### EXEMPLE 26 : 2-[({[5,6-Diméthoxy-3-(4-méthoxyphénoxy)benzo[b]thiophène-2-yl] méthyl}amino)sulfonyl]benzoate d'ammonium

Un passage sur colonne HPLC du composé obtenu dans l'exemple 25 suivi d'une lyophilisation permet d'obtenir le sel 26.

| *Microanalyse élémentaire :* | | | | |
|---|---|---|---|---|
| | *C%* | *H%* | *N%* | *S%* |
| *calculée* | *54,93* | *4,79* | *5,12* | *11,73* |
| *trouvée* | *54,97* | *4,82* | *5,00* | *12,23* |

### EXEMPLE 27: 2-{[{[5,6-Diméthoxy-3-(4-méthoxyphénoxy)benzo[b]thiophène-2-yl] méthyl}(méthyl)amino]sulfonyl}benzoate de méthyle

A une solution de 0,38 g du composé obtenu dans le stade L de l'exemple 24 dans 5 ml de diméthylformamide est ajouté, sous atmosphère inerte à température ambiante, 24 mg d'hydrure de sodium. Après 15 minutes d'agitation, 0,15 g d'iodure de méthyle dilué dans 2 ml de diméthylformamide est additionné. Après 18 heures de réaction à température ambiante, le mélange réactionnel est concentré. Le résidu est dilué dans l'acétate d'éthyle, lavé par une solution saturée en NaHCO₃, puis à l'eau, puis par une solution saturée en NaCl, séchée sur sulfate de calcium, filtrée et évaporée sous pression réduite. Une chromatographie sur gel de silice permet d'isoler le produit attendu.

### EXEMPLE 28 : 2-[({[5,6-Diméthoxy-3-(4-méthoxyphénoxy)benzo[b]thiophène-2-yl] méthyl}amino)sulfonyl]acétate d'éthyle

On procède comme dans l'exemple 24, en utilisant au stade L, comme réactif, le 2-(chlorosulfonyl)-éthanoate d'éthyle.

### EXEMPLE 29 : 2-[({[5,6-Diméthoxy-3-(4-méthoxyphénoxy)benzo[b]thiophène-2-yl] méthyl}amino)sulfonyl]acétate de sodium

On procède comme dans l'exemple 3, en remplaçant l'eau par de l'éthanol, et en utilisant comme substrat le composé obtenu dans l'exemple 28.

| *Microanalyse élémentaire :* | | | | |
|---|---|---|---|---|
| | *C%* | *H%* | *N%* | *S%* |
| *calculée* | *46,97* | *3,74* | *2,74* | *12,54* |
| *trouvée* | *47,71* | *3,80* | *2,81* | *11,86* |

### EXEMPLE 30 : 3-[5,6-Diméthoxy-3-(4-méthoxyphénoxy)benzo[b]thiophène-2-yl]-2-(4-pyridyl)-2-propanoate d'éthyle

On soumet le composé obtenu dans le stade F de l'exemple 1 à la procédure d'hydrogénation décrite dans l'exemple 12.

### EXEMPLE 31 : 3-[5,6-Diméthoxy-3-(4-méthoxyphénoxy)benzo[b]thiophène-2-yl]-2-(4-pyridyl)-2-propanoate de sodium

On procède comme dans l'exemple 2 puis dans l'exemple 3, en utilisant comme substrat le produit de l'exemple 30.

### EXEMPLE 32 : (E)-3-[5,6-Diméthoxy-3-(3-pentylphénoxy)benzo[b]thiophène-2-yl]-2-(4-pyridinyl)-2-propènoate de sodium

On procède comme dans l'exemple 1 en utilisant au stade E, comme réactif, le 3-pentylphénol, puis on suit la procédure de l'exemple 2 et de l'exemple 3.

| *Microanalyse élémentaire :* | | | | |
|---|---|---|---|---|
| | *C%* | *H%* | *N%* | *S%* |
| *calculée* | *66,27* | *5,37* | *2,66* | *6,10* |
| *trouvée* | *66,00* | *5,44* | *2,73* | *5,85* |

### EXEMPLE 33 : Acide (E)-3-[5,6-bis(benzyloxy)-3-(4-méthoxyphénoxy) benzo[b]thiophène-2-yl]-2-(4-pyridinyl)-2-propènoïque

On procède comme dans l'exemple 1 en utilisant comme produit de départ au stade A, l'acide 3,4-dibenzyloxycinnamique, puis comme dans l'exemple 2.

### EXEMPLE 34 : Acide (E)-3-[5,6-dihydroxy-3-(4-méthoxyphénoxy)benzo[b]thiophène-2-yl]-2-(4-pyridinyl)-2-propènoïque

On procède comme dans l'exemple 33 mais entre le stade E et le stade F on réalise l'étape intermédiaire E' suivante : le 5,6-bis(benzyloxy)-3-(4-méthoxyphénoxy)benzo[*b*]thiophène-2-carbaldéhyde (obtenu au stade E) est hydrogénolysé en présence de Pd/C 10 % dans du méthanol. Après 20 heures de réaction à température ambiante, le mélange réactionnel est filtré puis concentré sous pression réduite. Une chromatographie sur gel de silice permet d'isoler le produit attendu, qui est alors soumis au procédé décrit dans le stade F. On procède ensuite selon le protocole de l'exemple 2.

### EXEMPLE 35 : Acide (E)-3-[5,6-bis(benzyloxy)-3-(4-benzyloxyphénoxy) benzo[b]thiophène-2-yl]-2-(4-pyridinyl)-2-propènoïque

On procède comme dans l'exemple 33 en utilisant comme réactif au stade E le 4-benzyloxyphénol, puis comme dans l'exemple 2.

### EXEMPLE 36 : Acide (E)-3-[5-chloro-4,7-diméthoxy-3-(4-méthoxyphénoxy) benzo[b]thiophène-2-yl]-2-(4-pyridinyl)-2-propènoïque

On procède comme dans l'exemple 1, en utilisant comme produit de départ dans le stade A, l'acide 3-chloro-2,5-diméthoxycynnamique, puis on suit le protocole décrit dans l'exemple 2.

### EXEMPLE 37 : Acide (E)-3-[6,7-dichloro-3-(4-méthoxyphénoxy)benzo[b]thiophène-2-yl]-2-(4-pyridinyl)-2-propènoïque

On procède comme dans l'exemple 1, en utilisant comme produit de départ dans le stade A, l'acide 4,5-dichlorocinnamique, puis on suit le protocole décrit dans l'exemple 2.

### EXEMPLE 38 : Acide (E)-3-[5,6-diméthoxy-3-(2,4-difluorophénoxy) benzo[b]thiophène-2-yl]-2-(4-pyridinyl)-2-propènoïque

On procède comme dans l'exemple 1, en utilisant comme réactif au stade E le 2,4-difluorophénol, puis on suit le protocole décrit dans l'exemple 2.

### EXEMPLE 39 : Acide (E)-3-[5,6-diméthoxy-3-(3-pyridinyloxy)benzo[b]thiophène-2-yl]-2-(4-pyridinyl)-2-propènoïque

On procède comme dans l'exemple 1, en utilisant comme réactif au stade E, la 3-hydroxypyridine, puis on suit le protocole décrit dans l'exemple 2.

### EXEMPLE 40 : Acide (E)-3-{3-[(4,6-diméthyl-2-pyrimidinyl)oxy]-6-méthoxy-1-benzofuran-2-yl}-2-(4-pyridinyl)-2-propènoïque

### Stade 1 : 3-[4,6-Diméthyl-2-pyrimidinyl)oxy]-6-méthoxy-1-benzofuran-2-carbaldéhyde

On procède comme dans le stade E de l'exemple 1 en utilisant comme substrat le 3-hydroxy-6-méthoxy-1-benzofuran-2-carbaldéhyde et comme réactif la 2-chloro-4,6-diméthyl-pyrimidine.

### Stade 2 : Acide (E)-3-{3-[(4,6-diméthyl-2-pyrimidinyl)oxy]-6-méthoxy-1-benzofuran-2-yl}-2-(4-pyridinyl)-2-propènoïque

On procède comme dans le stade F de l'exemple 1, puis comme dans l'exemple 2, en utilisant comme substrat le produit obtenu au stade 1 décrit précédemment.

### EXEMPLE 41 : Acide (E)-3-[5,6-diméthoxy-3-(4-méthoxyphénoxy)-1H-2-indolyl]-2-(4-pyridinyl)-2-propènoïque

On procède comme dans l'exemple 1, du stade E au stade F, en utilisant comme substrat au stade E le 3-bromo-5,6-diméthoxy-1H-2-indolecarbaldéhyde, puis on suit le protocole décrit dans l'exemple 2.

### EXEMPLE 42 : Acide (E)-3-[5,6-diméthoxy-3-[(4-méthoxyphényl)sulfanyl] benzo[b]thiophène-2-yl]-2-(4-pyridinyl)-2-propènoïque

On procède comme dans l'exemple 1, en utilisant comme réactif au stade E, le 4-méthoxybenzènethiol, puis on suit le protocole décrit dans l'exemple 2.

### EXEMPLE 43 : (E)-3-[5,6-Bis(benzyloxy)-3-(4-méthoxyphénoxy)benzo[b]thiophène-2-yl]-2-(4-pyridinyl)-2-propènoate de sodium

On procède comme dans l'exemple 3 en utilisant comme substrat le produit de l'exemple 33.
*Point de fusion : > 260°C*

| *Microanalyse élémentaire :* | | | | |
|---|---|---|---|---|
| | *C%* | *H%* | *N%* | *S%* |
| *calculé* | *69,69* | *4,43* | *2,20* | *5,03* |
| *trouvé* | *70,10* | *4,40* | *2,25* | *4,66* |

### EXEMPLE 44 : (E)-3-[5,6-Bis(benzyloxy)-3-(4-benzyloxyphénoxy)benzo[b]thiophène-2-yl]-2-(4-pyridinyl)-2-propènoate de sodium

On procède comme dans l'exemple 3 en utilisant comme substrat le produit de l'exemple 35.
*Produit de fusion :* > *260°C*

| *Microanalyse élémentaire :* | | | | |
|---|---|---|---|---|
| | *C%* | *H%* | *N%* | *S%* |
| *calculé* | *72,36* | *4,52* | *1,96* | *4,49* |
| *trouvé* | *72,51* | *4,54* | *2,04* | *4,25* |

### EXEMPLE 45 : (E)-3-[6,7-Dichloro-3-(4-méthoxyphénoxy)benzo[b]thiophène-2-yl]-2-(4-pyridinyl)-2-propènoate de sodium

On procède comme dans l'exemple 3 en utilisant comme substrat le produit de l'exemple 37.

| *Microanalyse élémentaire :* | | | | | |
|---|---|---|---|---|---|
| | *C%* | *H%* | *N%* | *S%* | *CI%* |
| *calculé* | *55,88* | *2,85* | *2,83* | *6,49* | *14,34* |
| *trouvé* | *56,27* | *2,95* | *2,91* | *6,80* | *14,45* |

### EXEMPLE 46 : (E)-3-[3-(4-Chlorophénoxy)-5,6-diméthoxybenzo[b]thiophène-2-yl]-2-(4-pyridinyl)-2-propènoate de sodium

On procède comme dans l'exemple 1 en utilisant comme réactif au stade E le 4-chlorophénol puis on suit le protocole décrit dans l'exemple 2 puis l'exemple 3.

| *Microanalyse élémentaire :* | | | | | |
|---|---|---|---|---|---|
| | *C%* | *H%* | *N%* | *S%* | *Cl%* |
| *calculé* | *58,84* | *3,50* | *2,86* | *6,54* | *7,24* |
| *trouvé* | *59,33* | *3,53* | *2,93* | *6,28* | *7,60* |

### EXEMPLE 47 : 4-({2-[(E)-2-Sodiumcarboxy-2-(4-pyridinyl)éthenyl]-5,6-diméthoxy-1-benzo[b]thiophène-3-yl}oxy)benzoate de sodium

On procède comme dans l'exemple 1 en utilisant comme réactif au stade E le 4-hydroxy-benzoate de méthyle, puis on suit le protocole décrit dans l'exemple 2 puis l'exemple 3.

| *Microanalyse élémentaire :* | | | | |
|---|---|---|---|---|
| | *C %* | *H%* | *N%* | *S%* |
| *calculé* | *57,58* | *3,29* | *2,69* | *6,15* |
| *trouvé* | *57,20* | *3,07* | *2,65* | *5,63* |

### EXEMPLE 48 : (E)-3-[5-Chloro-4,7-diméthoxy-3-(4-méthoxyphénoxy)-benzo[b]thiophène-2-yl]-2-(4-pyridinyl)-2-propènoate de sodium

On procède comme dans l'exemple 3 en utilisant comme substrat le produit de l'exemple 36.

| *Microanalyse élémentaire :* | | | | | |
|---|---|---|---|---|---|
| | *C%* | *H%* | *N%* | *S%* | *Cl%* |
| *calculé* | *57,75* | *3,68* | *2,69* | *6,82* | *6,17* |
| *trouvé* | *57,93* | *3,61* | *2,67* | *6,84* | *6,10* |

### EXEMPLE 49 : (E)-3-[5,6-Diméthoxy-3-(2,4-difluorophénoxy)benzo[b]thiophène-2-yl]-2-(4-pyridinyl)-2-propénoate de sodium

On procède comme dans l'exemple 3 en utilisant comme substrat le produit de l'exemple 38.

| *Microanalyse élémentaire :* | | | | |
|---|---|---|---|---|
| | *C%* | *H%* | *N%* | *S%* |
| *calculé* | *58,66* | *3,28* | *2,85* | *6,52* |
| *trouvé* | *58,90* | *3,16* | *2,95* | *6,49* |

### EXEMPLE 50 : Acide 3-(E)-{3-[4-(2-(diméthylamino)éthoxy)-phénoxyl]-5,6-diméthoxybenzo[b]thiophène-2-yl}-2-phényl-2-propènoïque

On procède comme dans l'exemple 1 en utilisant comme réactif au stade E, le 2-(diméthylamino)éthoxy-phénol, et au stade F l'acide phényl éthanoique puis on suit le protocole décrit dans l'exemple 2.

| *Microanalyse élémentaire :* | | | | |
|---|---|---|---|---|
| | *C%* | *H%* | *N%* | *S%* |
| *calculé* | *58,10* | *4,70* | *2,17* | *4,97* |
| *trouvé* | *57,63* | *4,60* | *2,23* | *4,84* |

### EXEMPLE 51 : (E)-3-[5,6-Diméthoxy-3-(3-méthoxyphéoxy)benzo[b]thiophène-2-yl]-2-(4-pyridinyl)-2-propènoate de sodium

On procède comme dans l'exemple 1, en utilisant comme réactif au stade E le 3-méthoxy-phénol, puis on suit le protocole décrit dans l'exemple 2, puis l'exemple 3.

| *Microanalyse élémentaire :* | | | | |
|---|---|---|---|---|
| | *C%* | *H%* | *N%* | *S%* |
| *calculé* | *61,85* | *4,15* | *2,89* | *6,60* |
| *trouvé* | *61,66* | *4,21* | *2,89* | *6,36* |

### EXEMPLE 52 : (E)-3-[3-(4-Trifluorométhylphénoxy)-5,6-diméthoxy-benzo[b]thiophène-2-yl]-2-(4-pyridinyl)-2-propènoate de sodium

On procède comme dans l'exemple 1, en utilisant comme réactif au stade E le 4-trifluorométhyl-phénol, puis on suit le protocole décrit dans l'exemple 2, puis l'exemple 3.

| *Microanalyse élémentaire :* | | | | |
|---|---|---|---|---|
| | *C%* | *H%* | *N%* | *S%* |
| *calculé* | *57,36* | *3,27* | *2,68* | *6,13* |
| *trouvé* | *57,43* | *2,78* | *2,72* | *6,33* |

### EXEMPLE 53 : (E)-3-[5,6-Diméthoxy-3-(2-méthoxyphénoxy)benzo[b]thiophène-2-yl]-2-(4-pyridinyl]-2-propènoate de sodium

On procède comme dans l'exemple 1, en utilisant comme réactif au stade E le 2-méthoxy-phénol, puis on suit le protocole décrit dans l'exemple 2, puis l'exemple 3.
*Spectre de masse : FAB : [M-H]*^{*+*} *: m/z = 486 ; [M-Na+2H]*^{*+*} *: m/z = 464 ; [M-H+2Na]*^{*-*} *: m/z = 508*

### EXEMPLE 54 : (E)-3-{3-[4-Sodiumcarboxyméthyl)phénoxy]-5,6-diméthoxy-1-benzo[b] thiophène-2-yl}-2-(4-pyridinyl)-2-propènoate de sodium

On procède comme dans l'exemple 1 en utilisant comme réactif au stade E le 4-hydroxyphényl-éthanoate d'éthyle, puis on suit le protocole décrit dans l'exemple 2, puis l'exemple 3.

| *Microanalyse élémentaire :* | | | | |
|---|---|---|---|---|
| | *C%* | *H%* | *N%* | *S%* |
| *calculé* | *58,32* | *3,58* | *2,62* | *5,99* |
| *trouvé* | *58,38* | *3,65* | *2,65* | *5,80* |

### EXEMPLE 55 : (E)-3-[5,6-Diméthoxy-3-(4-(trifluorométhoxy)phénoxybenzo[b]thiophène-2-yl]-2-(4-pyridinyl)-2-propènoate de sodium

On procède comme dans l'exemple 1, en utilisant comme réactif au stade E, le 4-(trifluorométhoxy)phénol, puis on suit le protocole décrit dans l'exemple 2, puis l'exemple 3.

| *Microanalyse élémentaire :* | | | | |
|---|---|---|---|---|
| | *C%* | *H%* | *N%* | *S%* |
| *calculé* | *55,66* | *3,18* | *2,60* | *5,94* |
| *trouvé* | *56,07* | *3,00* | *2,64* | *6,04* |

### EXEMPLE 56 : (E)-3-{3-[4-(Acétylamino)phénoxy]-5,6-diméthoxybenzo[b]thiophène-2-yl}-2-(4-pyridinyl)-2-propènoate de sodium

On procède comme dans l'exemple 1, en utilisant comme réactif au stade E, le 4-(acétylamino)phénol, puis on suit le protocole décrit dans l'exemple 2, puis l'exemple 3.
*Spectre de masse : FAB : [M+H]*^{*+*} *: m/z = 513 ; [M+Na]*^{*+*} *: m/z = 535*

### EXEMPLE 57 : (E)-3-[5,6-Diméthoxy-3-(4-méthoxyphényl)-1-(4-pyridinyl)méthyl-1H-indol-2-yl]-2-propènoate de sodium

On procède comme dans l'exemple 1, des stades B à D puis stade F, en utilisant comme substrat au stade B, le 5,6-diméthoxy-3-(4-méthoxyphényl)-1-(4-pyridinylméthyl)-2-indoline carboxylate d'éthyle, et comme réactif au stade F, le 2-(triphénylphosphoranylidène)acétate d'éthyle. Puis on suit le protocole décrit dans l'exemple 2 et l'exemple 3.

| *Microanalyse élémentaire :* | | | |
|---|---|---|---|
| | *C%* | *H%* | *N%* |
| *calculé* | *66,95* | *4,97* | *6,01* |
| *trouvé* | *66,18* | *4,76* | *6,01* |

### EXEMPLE 58 : Acide 3-(E)-[5,6-bis(benzyloxy)-3-(pyridinyl-3-oxy)-benzo[b]thiophène-2-yl]-2-(4-pyridinyl)-2-propènoïque

On procède comme dans l'exemple 1, en utilisant comme substrat au stade A l'acide 3,4-bis(benzyloxy)cinnamique comme réactif au stade E, la 3-hydroxy-pyridine, puis on suit le protocole décrit dans l'exemple 2.
*Point de fusion : 256°C*

| *Microanalyse élémentaire :* | | | | |
|---|---|---|---|---|
| | *C%* | *H%* | *N%* | *S%* |
| *calculé* | *71,66* | *4,47* | *4,77* | *5,47* |
| *trouvé* | *71,59* | *4,58* | *4,78* | *5,10* |

### EXEMPLE 59 : Acide 3-(E)-{5,6-bis(benzyloxy)-3-[4-(hydroxy)phénoxy]-benzo[b] thiophène-2-yl}-2-(4-pyridinyl)-2-propènoïque

### Stade 1 : 5,6-Bis(benzyloxy)-3-[4-(pyridinyl-4-oxy)phénoxy]-benzo[b]thiophène-2-carbaldéhyde

On procède comme dans l'exemple 1, des stades A à E en utilisant comme substrat au stade A l'acide 3,4-bis(benzyloxy)cinnamique et comme réactif au stade E, la 4-(pyridinyl-4-oxy)phénol.

### Stade 2 : (E)-3-{3-[4-(Acétyloxy)phénoxy]-5,6-bis(benzyloxy)-benzo[b]thiophène-2-yl}-2-(4-pyridinyl)-2-propènoate d'éthyle

On procède comme dans le stade F de l'exemple 1 en utilisant comme produit de départ celui obtenu dans le stade 1 précédent.

### Stade 3 : Acide 3-(E)-{5,6-bis(benzyloxy-3-[4-(hydroxy)phénoxy]-benzo[b]thiophène-2-yl}-2-(4-pyridinyl)-2-propènoïque

On procède comme dans l'exemple 2 avec le produit obtenu dans le stade 2 précédent.
*Point de fusion : 256°C*

| *Microanalyse élémentaire :* | | | | |
|---|---|---|---|---|
| | *C%* | *H%* | *N%* | *S%* |
| *calculé* | *71,87* | *4,52* | *2,33* | *5,33* |
| *trouvé* | *71,40* | *4,58* | *2,35* | *4,93* |

### EXEMPLE 60 : Acide 3-(E)-{5,6-bis(benzyloxy)-3-[6-(méthyl)pyridinyl-3-oxy]-benzo[b]thiophène-2-yl}-2-(4-pyridinyl)-2-propènoïque

On procède comme dans l'exemple 1, en utilisant comme substrat au stade A l'acide 3,4-bis(benzyloxy)cinnamique comme réactif au stade E, la 2-méthyl-5-hydroxy-pyridine, puis on suit le protocole décrit dans l'exemple 2.
*Point de fusion : 228°C*

| *Microanalyse élémentaire :* | | | | |
|---|---|---|---|---|
| | *C%* | *H%* | *N%* | *S%* |
| *calculé* | *71,98* | *4,70* | *4,66* | *5,34* |
| *trouvé* | *72,19* | *4,81* | *4,70* | *4,94* |

### EXEMPLE 61 : Acide 3-(E)-{6-benzyloxy-3-[4-(benzyloxy)phénoxy]-5-méthoxy-benzo[b]thiophène-2-yl}-2-(4-pyridinyl)-2-propènoïque

On procède comme dans l'exemple 1, en utilisant comme substrat au stade A l'acide 4-benzyloxy-3-méthoxy-cinnamique et comme réactif au stade E le 4-(benzyloxy)phénol, puis on suit le protocole décrit dans l'exemple 2.
*Point de fusion : 216°C*

| *Microanalyse élémentaire :* | | | | |
|---|---|---|---|---|
| | *C%* | *H%* | *N%* | *S%* |
| *calculé* | *72,18* | *4,75* | *2,27* | *5,21* |
| *trouvé* | *71,92* | *4,56* | *2,32* | *5,10* |

### EXEMPLE 62 : Acide 3-(E)-{5-benzyloxy-3-[4-{benzylory)phénoxy]-6-méthoxy-benzo[b]thiophène-2-yl}-2-(4-pyridinyl)-2-propènoïque

On procède comme dans l'exemple 61 en utilisant comme produit de départ l'acide 3-benzyloxy-4-méthoxy-cinnamique.
*Point de fusion : 210°C*

### EXEMPLE 63 : (E)-3-[5,6-Bis(benzyloxy)-3-(6-quinolinyloxy)benzo[b]thiophène-2-yl]-2-(4-pyridinyl)-2-propènoate de sodium

### Stade 1 : Acide 3-(E)-[5,6-Bis(benzyloxy)-3-(6-quinolinyloxy)benzo[b]thiophène-2-yl]-2-(4-pyridinyl)-2-propènoïque

On procède comme dans l'exemple 1 en utilisant comme substrat au stade A l'acide 3,4-bis(benzyloxy)-cinnamique et comme réactif au stade E la 6-hydroxy-quinoléine, puis on suit le protocole décrit dans l'exemple 2.

### Stade 2 : (E)-3-[5,6-Bis(benzyloxy)-3-(6-quinolinyloxy)benzo[b]thiophène-2-yl]-2-(4-pyridinyl)-2-propènoate de sodium

On procède comme dans l'exemple 3 en utilisant comme substrat le produit obtenu dans le stade 2 précédent.

| *Microanalyse élémentaire :* | | | | |
|---|---|---|---|---|
| | *C%* | *H %* | *N%* | *S%* |
| *calculé* | *71,11* | *4,13* | *4,25* | *4,87* |
| *trouvé* | *71,36* | *4,22* | *4,31* | *4,46* |

### EXEMPLE 64 : (E)-3-{5,6-Bis-(benzyloxy)-3-[4-(1H-imidazol-1-yl)phénoxy] benzo[b]thiophène-2-yl}-2-(4-pyridinyl)-2-propènoate de sodium

On procède comme dans l'exemple 1 en utilisant comme substrat au stade A l'acide 3,4-bis(benzyloxy)cinnamique et comme réactif au stade E le 4-(1*H*-imidazol-1-yl)phénol, puis on suit le protocole décrit dans l'exemple 2 puis l'exemple 3.

| *Microanalyse élémentaire :* | | | | |
|---|---|---|---|---|
| | *C%* | *H%* | *N%* | *S%* |
| *calculé* | *69,53* | *4,19* | *6,24* | *4,76* |
| *trouvé* | *69,27* | *4,25* | *6,29* | *4,62* |

### EXEMPLE 65 : Acide (E)-3-{5,6-bis(benzyloxy)-3-[(6-méthoxy-3-pyridinyl)oxy] benzo[b]thiophène-2-yl}-2-(4-pyridinyl)-2-propènoïque

On procède comme dans l'exemple 1, en utilisant comme substrat au stade A, l'acide 3,4-bis(benzyloxy)cinnamique et comme réactif au stade E, la 6-méthoxy-3-pyridinol, puis on suit le protocole décrit dans l'exemple 2.
*Point de fusion : 188°C*

| *Microanalyse élémentaire :* | | | | |
|---|---|---|---|---|
| | *C%* | *H%* | *N%* | *S%* |
| *calculé* | *70,12* | *4,58* | *4,34* | *5,20* |
| *trouvé* | *69,77* | *4,67* | *4,58* | *4,82* |

### EXEMPLE 66 : Acide (E)-3-{5,6-bis(benzyloxy)-3-[4-(4-pyridinyl)phénoxy-benzo[b] thiophène-2-yl}-2-(4-pyridinyl)-2-propènoïque

On procède comme dans l'exemple 65 en utilisant comme réactif au stade E le 4-(4-pyridinyl)phénol.
*Spectre de masse : FAB : [M+H]*^{*+*} *: m/z = 663*

### EXEMPLE 67 : (E)-3-[5-Benzyloxy-6-méthoxy-3-(3-pyridinyl)oxy-benzo[b]thiophène-2-yl]-2-(4-pyridinyl)-2-propènoate de sodium

### Stade 1 : Acide (E)-3-[5-benzyloxy-6-méthoxy-3-(3-pyridinyl)oxy-benzo[b]thiophène-2-yl]-2-(4-pyridinyl)-2-propènoïque

On procède comme dans l'exemple 1 en utilisant comme substrat au stade A l'acide 3-benzyloxy-4-méthoxy-cinnamique et comme réactif au stade E la 3-pyridinol, puis on suit le protocole décrit dans l'exemple 2.

### Stade 2 : (E)-3-[5-Benzyloxy-6-méthoxy-3-(3-pyridinyl)oxy-benzo[b]thiophène-2-yl]-2-(4-pyridinyl)-2-propènoate de sodium

On procède comme dans l'exemple 3 en utilisant comme substrat le produit obtenu dans le stade 2 précédent.

| *Microanalyse élémentaire :* | | | | |
|---|---|---|---|---|
| | *C%* | *H%* | *N%* | *S%* |
| *calculé* | *65,41* | *3,97* | *5,26* | *6,02* |
| *trouvé* | *65,25* | *3,99* | *5,24* | *5,84* |

### EXEMPLE 68 : Acide (E)-3-{3-[(1-hydroxy-2-oxo-1,2-dihydro-4-pyridinyl)oxy]-5,6-diméthoxy-benzo[b]thiophène-2-yl}-2-(4-pyridinyl)-2-propènoïque

On procède comme dans l'exemple 1 en utilisant comme réactif au stade E la 1,4-dihydroxy-2-oxo-1,2-dihydro-pyridine, puis on suit le protocole décrit dans l'exemple 2.

### EXEMPLE 69 : Acide 3-(E)-{5,6-diméthoxy-3-[(2-oxo-2,3-dihydro-1H-indol-5-yl)oxy] benzo[b]thiophène-2-yl}-2-(4-pyridinyl)-2-propènoïque

On procède comme dans l'exemple 1 en utilisant comme réactif au stade E le 5-hydroxy-1,3-dihydro-2*H*-indol-2-one.

### EXEMPLE 70 : Acide 3-(E)-[3-(1H-benzimidazol-5-yloxy)-5,6-diméthoxy-benzo [b]thiophène-2-yl]-2-(4-pyridinyl)-2-propènoïque

On procède comme dans l'exemple 1 en utilisant comme réactif au stade E le 5-hydroxy-1*H*-benzimidazol, puis on suit le protocole de l'exemple 2.

### EXEMPLE 71 : Acide 3-(E)-[3-(1H-indol-5-yloxy)-5,6-diméthoxy-benzo[b]thiophène-2-yl]-2-(4-pyridinyl)-2-propènoïque

On procède comme dans l'exemple 1 en utilisant comme réactif au stade E le 5-hydroxy-1*H*-indole, puis on suit le protocole de l'exemple 2.

### EXEMPLE 72 : Acide 3-(E)-[5,6-diméthoxy-3-(1,2,3,4-tétrahydro-6-quinolinyloxy)-benzo[b]thiophène-2-yl]-2-(4-pyridinyl)-2-propènoïque

On procède comme dans l'exemple 1 en utilisant comme réactif au stade E la 1,2,3,4-tétrahydro-6-quinolinol, puis on suit le protocole de l'exemple 2.

### EXEMPLE 73 : (E)-3-[5,6-Diméthoxy-3-(4-méthoxyanilino)benzo[b]thiophène-2-yl]-2-(4-pyridinyl)-2-propènoate d'éthyle

On procède comme dans l'exemple 1 en utilisant comme réactif au stade E la *p*-anisidine.

### EXEMPLE 74 : (E)-3-[5,6-Bis(benzyloxy)-3-(4-méthoxyanilino)benzo[b]thiophène-2-yl]-2-(4-pyridinyl)-2-propènoate d'éthyle

On procède comme dans l'exemple 1 en utilisant comme substrat au stade A l'acide 3,4-bis(benzyloxy)-cinnamique et comme réactif au stade E la *p*-anisidine.

### EXEMPLE 75 : (E)-3-[5,6-Diméthoxy-3-(4-méthoxyphénoxy)benzo[b]thiophène-2-yl]-2-(4-méthylphényl)-2-propènoate d'éthyle

On procède comme dans l'exemple 1, en utilisant comme réactif au stade F, l'éthyl 4-méthylphénylacétate.

### EXEMPLE 76 : (E)-3-[5,6-Diméthoxy-3-[4-méthoxyphénoxy)-benzo[b]thiophène-2-yl]-2-(3-thiényl)-2-propènoate d'éthyle

On procède comme dans l'exemple 1, en utilisant comme réactif au stade F l'éthyl 3-thiophèneacétate.

### EXEMPLE 77 : (E)-3-[5,6-(1,3,4,6-Tétrahydro-2,5-benzodioxin-3,4-diyl)-3-(4-méthoxy-phénoxy)-benzo[b]thiophène-2-yl]-2-(4-pyridinyl)-2-propènoate d'éthyle

On procède comme dans l'exemple 1, en utilisant comme substrat au stade A l'acide 3,4-(1,3,4,6-tétrahydro-2,5-benzodioxin-3,4-diyl)cinnamique, ce dernier étant obtenu par addition du dibromo-*o*-xylène sur l'acide 3,4-dihydroxycinnamique.

### EXEMPLE 78 : (E)-3-[3-Phényl-9-(4-méthoxyphénoxy)-3,4-dihydro-2H-thiéno[2,3-h][1,5] benzodioxépin-8-yl]-2-(4-pyridinyl)-2-propènoate d'éthyle

On procède comme dans l'exemple 1 en utilisant comme substrat au stade A, l'acide (E)-3-(3-phényl-3,4-dihydro-2*H*-1,5-benzodioxépin-7-yl)-2-propènoïque.

### EXEMPLE 79 : (E)-3-[8-(4-méthoxyphénoxy)-2,3-diphényl-2,3-dihydrothiéno[2,3-g][1,4] benzodioxin-7-yl]-2-(4-pyridinyl)-2-propènoate d'éthyle

On procède comme dans l'exemple 1 en utilisant comme substrat au stade A, l'acide (E)-3-(2,3-diphényl-2,3-dihydro-1,4-benzodioxin-6-yl)-2-propènoïque.

### ETUDE PHARMACOLOGIQUE DES COMPOSES DE L'INVENTION

### EXEMPLE 80 : Inhibition de l'activité du PAI-1

L'inhibition de l'activité du PAI-1 a été réalisée in-vitro dans des puits de microplaque dans lesquels la formation puis la lyse d'un caillot de fibrine est suivie en continu en turbidimétrie grâce à un spectrophotomètre. Pour ce faire, en utilisant comme diluant un tampon phosphate 50 mM pH 7,4 contenant 0,05% de sérum albumine bovine, 50 µl de l'inhibiteur est mis en présence avec 50 µl d'une solution de PAI-1 actif recombinant humain à 2 nM pendant 5 minutes à température ambiante. 50 µl d'une solution d'activateur tissulaire du plasminogène à 0,42 nM, 50 µl d'une solution de plasminogène humain à 800 nM et 50 µl d'une solution de fibrinogène à 2g/l sont ensuite additionnés et la fibrinoformation déclenchée par l'ajout de 50 µl de thrombine humaine purifiée à 14 nM. En l'absence de produit, l'inhibition de la lyse deux heures après le début de la fibrinoformation, est mesurée par l'absorbance du caillot et représente 100% de l'activité PAI-1. En l'absence de produit et de PAI-1, la lyse est mesurée par l'absorbance du caillot lysé et représente 0% de l'activité PAI-1. La concentration du produit inhibant 50 % de l'activité du PAI-1 est recherchée en mesurant l'absorbance du caillot deux heures après la fibrinoformation en présence de PAI-1 et de concentration croissante du produit. Les IC₅₀ des composés de l'invention ainsi que celle de la substance référence, le XR 5082, sont présentées dans le tableau 1. Les résultats démontrent l'activité fibrinolytique supérieure des composés de l'invention.

**Tableau 1 :**

| **Activité fibrinolytique des composés de l'invention** | |
|---|---|
| ***Exemple*** | ***IC50 (µM)*** |
| 43 | 13 |
| 44 | 7 |
| 66 | 2,2 |
| 67 | 5 |
| ***XR 5082*** | ***190*** |

### EXEMPLE 81 : Composition pharmaceutique

Formule de préparation pour 1000 comprimés dosés à 10 mg :

| | |
|---|---|
| Composé de l'exemple | 10 g |
| Hydroxypropylcellulose | 2 g |
| Polyvinylpyrrolidone | 2 g |
| Amidon de blé | 10 g |
| Lactose | 100 g |
| Stéarate de magnésium | 3 g |

## Revendications

1. Composés de formule (I): 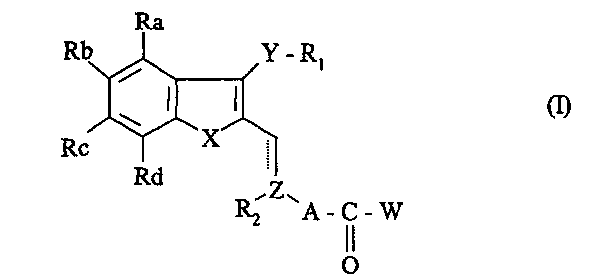 dans laquelle :
X représente un atome d'oxygène, de soufre, ou un groupement NR₃ dans lequel R₃ représente un atome d'hydrogène, un groupement alkyle (C₁-C₆) linéaire ou ramifié, acyle (C₁-C₆) linéaire ou ramifié, aryle, arylalkyle (C₁-C₆) linéaire ou ramifié, ou hétéroarylalkyle (C₁-C₆) linéaire ou ramifié,
Y représente un atome d'oxygène, de soufre, un groupement NR₃, le groupement R₃ ayant la même définition que précédemment, ou peut représenter une liaison simple quand X représente un groupement NR'₃ dans lequel R'₃ représente un groupement hétéroarylalkyle (C₁-C₆) linéaire ou ramifié,
Z représente un atome d'azote quand la liaison qui le relie avec l'atome de carbone voisin est simple (―), un atome de carbone ou un groupement CH selon que la liaison qui le relie avec l'atome de carbone voisin est simple (―) ou double (^{......}),
A représente une liaison simple, un groupement alkylène (C₁-C₆) éventuellement substitué par un ou plusieurs groupements choisis parmi alkyle (C₁-C₆) linéaire ou ramifié, aryle, arylalkyle (C₁-C₆) linéaire ou ramifié, cycloalkyle et un hétérocycle, arylène, cycloalkylène, un hétérocycle, ou un groupement -SO₂-R₄- dans lequel R₄ représente un groupement alkylène (C₁-C₆) linéaire ou ramifié, arylène, arylalkylène (C₁-C₆) linéaire ou ramifié, cycloalkylène ou un hétérocycle,
W représente un groupement hydroxy, alkoxy (C₁-C₆) linéaire ou ramifié, aryloxy, arylalkoxy (C₁-C₆) linéaire ou ramifié, cycloalkyloxy, un hétérocycle lié à un atome d'oxygène, un groupement hydroxyamino, ou un groupement amino lui-même pouvant être substitué par un ou deux groupements, identiques ou différents, indépendamment l'un de l'autre, choisis parmi alkyle (C₁-C₆) linéaire ou ramifié, aryle, arylalkyle (C₁-C₆) linéaire ou ramifié, ou cycloalkyle,
Ra, Rb, Rc, Rd, identiques ou différents, indépendamment l'un de l'autre, représentent un atome d'hydrogène, d'halogène, un groupement hydroxy, cyano, nitro, alkyle (C₁-C₆) linéaire ou ramifié, alkoxy (C₁-C₆) linéaire ou ramifié, acyle (C₁-C₆) linéaire ou ramifié, carboxy, alkoxycarbonyle (C₁-C₆) linéaire ou ramifié, trihalogénoalkyle (C₁-C₆) linéaire ou ramifié, aryloxy, arylalkoxy (C₁-C₆) linéaire ou ramifié, hétéroaryloxy, hétéroarylalkoxy (C₁-C₆) linéaire ou ramifié, amino éventuellement substitué par un ou deux groupements, identiques ou différents, indépendamment l'un de l'autre, alkyle (C₁-C₆) linéaire ou ramifié, aryle, arylalkyle (C₁-C₆) linéaire ou ramifié,
ou Ra+Rb, Rb+Rc ou Rc+Rd représente un groupement de formule -U₁-V-U₂ dans laquelle :
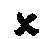 U₁, U₂, identiques ou différents, représentent un atome d'oxygène, de soufre, un groupement NH ou CH₂,
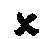 V représente un groupement alkylène (C₁-C₄) linéaire, éventuellement substitué par un ou plusieurs groupements alkyle (C₁-C₆) linéaire ou ramifié, aryle, arylalkyle (C₁-C₆) linéaire ou ramifié, hétéroaryle, hétéroarylalkyle (C₁-C₆) linéaire ou ramifié, ou V représente un groupement de formule 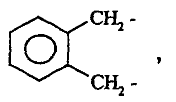
étant entendu que Ra, Rb, Rc et Rd ne peuvent pas représenter simultanément un atome d'hydrogène,
R₁ représente:
- un groupement aryle substitué par un à cinq substituants, identiques ou différents, indépendamment l'un de l'autre, choisis parmi halogène, hydroxy, cyano, nitro, carboxy, alkyle (C₁-C₆) linéaire ou ramifié, alkoxy (C₁-C₆) linéaire ou ramifié, acyle (C₁-C₆) linéaire ou ramifié, alkoxycarbonyle (C₁-C₆) linéaire ou ramifié, trihalogénoalkyle (C₁-C₆) linéaire ou ramifié éventuellement substitué par un groupement hydroxy ; trihalogénoalkoxy (C₁-C₆) linéaire ou ramifié, aminoalkoxy (C₁-C₆) linéaire ou ramifié, la partie amino pouvant être substitué par un ou deux groupements, identiques ou différents, alkyle (C₁-C₆) linéaire ou ramifié ; alkoxycarbonylalkyle (C₁-C₆) linéaire ou ramifié, alkyl(C₁-C₆)carbonylamino linéaire ou ramifié, arylakyle (C₁-C₆) linéaire ou ramifié, aryloxy, arylalkoxy (C₁-C₆) linéaire ou ramifié, arylamino, arylalkylamino (C₁-C₆) linéaire ou ramifié, arylsulfanyl, arylalkyl(C₁-C₆)sulfanyl linéaire ou ramifié, hétéroaryle, hétéroarylalkyle (C₁-C₆) linéaire ou ramifié, hétéroaryloxy, hétéroarylalkoxy (C₁-C₆) linéaire ou ramifié, hétéroarylamino, hétéroarylalkyl(C₁-C₆)amino linéaire ou ramifié, hétéroarylsulfanyle, hétéroarylalkyl(C₁-C₆)sulfanyl linéaire ou ramifié, amino éventuellement substitué par un ou deux groupements alkyle (C₁-C₆) linéaire ou ramifié, un desdits groupements alkyles pouvant éventuellement être substitué par un groupement amino, alkylamino (C₁-C₆) linéaire ou ramifié, ou dialkylamino (C₁-C₆) linéaire ou ramifié;
- un groupement de formule, 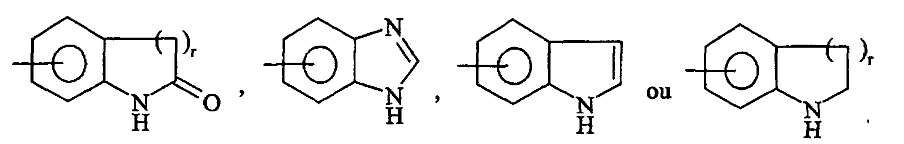 dans lesquelles r est un entier compris entre 1 et 2 inclus,
- un groupement 1-hydroxy-2(1*H*)-pyridinone,
- ou un groupement hétéroaryle éventuellement substitué,
R₂ représente un atome d'hydrogène, un groupement alkyle (C₁-C₆) linéaire ou ramifié, aryle, arylalkyle (C₁-C₆) linéaire ou ramifié, cycloalkyle, un hétérocycle éventuellement substitué ou un hétérocycle lié à un groupement alkyle (C₁-C₆) linéaire ou ramifié,
leurs isomères ainsi que leurs sels d'addition à un acide ou à une base pharmaceutiquement acceptable,
étant entendu que par :
- groupement aryle, on entend un groupement phényle, naphtyle, tétrahydronaphtyle, ou dihydronaphtyle, chacun de ces groupements étant éventuellement substitué de façon identique ou différente, par un ou plusieurs atomes d'halogène, groupements hydroxy, cyano, nitro, alkyle (C₁-C₆) linéaire ou ramifié, trihalogénoalkyle (C₁-C₆) linéaire ou ramifié, alkoxy (C₁-C₆) linéaire ou ramifié, acyle (C₁-C₆) linéaire ou ramifié, carboxy, alkoxycarbonyle (C₁-C₆) linéaire ou ramifié, amino substitué éventuellement par un ou deux groupements, identiques ou différents, alkyle (C₁-C₆) linéaire ou ramifié,
- groupement cycloalkyle, on entend un groupement mono ou bicyclique, comportant de 3 à 8 atomes de carbone,
- hétérocycle, on entend un groupement mono ou bicyclique, saturé ou insaturé, à caractère aromatique ou non aromatique, de 5 à 12 chaînons contenant un, deux ou trois hétéroatomes, identiques ou différents, choisis parmi oxygène, azote ou soufre, étant entendu que l'hétérocycle peut être éventuellement substitué, de façon identique ou différente, par un ou plusieurs atomes d'halogène, groupements hydroxy, alkyle (C₁-C₆) linéaire ou ramifié, trihalogénoalkyle (C₁-C₆) linéaire ou ramifié, alkoxy (C₁-C₆) linéaire ou ramifié, aryloxy, arylalkoxy (C₁-C₆) linéaire ou ramifié, acyle (C₁-C₆) linéaire ou ramifié, alkoxycarbonyle (C₁-C₆) linéaire ou ramifié, nitro, oxo, amino substitué éventuellement par un ou deux groupements alkyle (C₁-C₆) linéaire ou ramifié,
- hétéroaryle, plus précisément, on entend un hétérocycle, tel que défini ci-dessus, éventuellement substitué, mono ou bicyclique insaturé, dont au moins un des cycles possède un caractère aromatique.

2. Composés de formule (I) selon la revendication 1 **caractérisés en ce que** X représente un atome de soufre ou un groupement NR₃ avec R₃ tel que défini dans la formule (I), leurs isomères ainsi que leurs sels d'addition à un acide ou à une base pharmaceutiquement acceptable.

3. Composés de formule (I) selon la revendication 1 **caractérisés en ce que** Y représente un atome d'oxygène, leurs isomères ainsi que leurs sels d'addition à un acide ou à une base pharmaceutiquement acceptable.

4. Composés de formule (I) selon l'une quelconque des revendications 1 à 3 **caractérisés en ce que** X représente un atome de soufre et Y représente un atome d'oxygène, leurs isomères ainsi que leurs sels d'addition à un acide ou à une base pharmaceutiquement acceptable.

5. Composés de formule (I) selon la revendication 1 **caractérisés en ce que** R₁ représente un groupement phényle éventuellement substitué, pyridinyle éventuellement substitué ou quinolyle éventuellement substitué, leurs isomères ainsi que leurs sels d'addition à une acide ou à une base pharmaceutiquement acceptable.

6. Composés de formule (I) selon la revendication 1 **caractérisés en ce que** R₂ représente un groupement aryle, éventuellement substitué ou un hétérocycle éventuellement substitué, leurs isomères ainsi que leurs sels d'addition à un acide ou à une base pharmaceutiquement acceptable.

7. Composés de formule (I) selon l'une quelconque des revendications 1 et 6 **caractérisés en ce que** R₂ représente un groupement pyridinyle, leurs isomères ainsi que leurs sels d'addition à un acide ou à une base pharmaceutiquement acceptable.

8. Composés de formule (I) selon la revendication 1 **caractérisés en ce que** :
- X représente un atome de soufre,
- Y représente un atome d'oxygène,
- R₁ représente un groupement phényle éventuellement substitué ou un groupement pyridinyle éventuellement substitué,
- A représente une liaison simple quand Z représente un atome de carbone ou un groupement CH,
leurs isomères ainsi que leurs sels d'addition à un acide ou à une base pharmaceutiquement acceptable.

9. Composés de formule (I) selon la revendication 1 **caractérisés en ce que** :
- X représente un atome de soufre,
- Y représente un atome d'oxygène,
- R₁ représente un groupement phényle éventuellement substitué par un groupement tel que défini dans la formule (I),
- A représente un groupement alkylène (C₁-C₆) éventuellement substitué par un groupement choisi parmi alkyle (C₁-C₆) linéaire ou ramifié, aryle, et arylalkyle (C₁-C₆) linéaire ou ramifié, ou un groupement arylène quand Z représente un atome d'azote,
leurs isomères ainsi que leurs sels d'addition à un acide ou à une base pharmaceutiquement acceptable.

10. Composé de formule (I) selon la revendication 1 qui est l'acide (E)-3-[5,6-diméthoxy-3-(4-méthoxyphénoxy)benzo[*b*]thiophène-2-yl]-2-(4-pyridinyl)-2-propènoïque et ses sels d'addition à un acide ou à une base pharmaceutiquement acceptable.

11. Composé de formule (I) selon la revendication 1 qui est l'acide (E)-3-[5,6-bis(benzyloxy)-3-(4-méthoxyphénoxy)benzo[*b*]thiophène-2-yl]-2-(4-pyridinyl)-2-propènoïque, et ses sels d'addition à un acide ou à une base pharmaceutiquement acceptable.

12. Composé de formule (I) selon la revendication 1 qui est l'acide (E)-3-[5,6-bis(benzyloxy)-3-(4-benzyloxyphénoxy)benzo[*b*]thiophène-2-yl]-2-(4-pyridinyl)-2-propènoïque, et ses sels d'addition à un acide ou à une base pharmaceutiquement acceptable.

13. Composé de formule (I) selon la revendication 1 qui est l'acide (E)-3-[5,6-bis(benzyloxy)-3-(3-pyridinyloxy)benzo[*b*]thiophène-2-yl]-2-(4-pyridinyl)-2-propènoïque, et ses sels d'addition à un acide ou à une base pharmaceutiquement acceptable.

14. Composé de formule (I) selon la revendication 1 qui est l'acide (E)-3-[5,6-bis(benzyloxy)-3-(4-hydroxyphénoxy)benzo[*b*]thiophène-2-yl]-2-(4-pyridinyl)-2-propènoïque, et ses sels d'addition à un acide ou à une base pharmaceutiquement acceptable.

15. Composé de formule (I) selon la revendication 1 qui est l'acide (E)-3-{5,6-bis(benzyloxy)-3-[(6-méthyl-3-pyridinyl)oxy]benzo[*b*]thiophène-2-yl}-2-(4-pyridinyl)-2-propènoïque, et ses sels d'addition à un acide ou à une base pharmaceutiquement acceptable.

16. Composé de formule (I) selon la revendication 1 qui est l'acide (E)-3-{5-(benzyloxy)-3-[4-(benzyloxy)phénoxy]-6-méthoxy-benzo[*b*]thiophène-2-yl}-2-(4-pyridinyl)-2-propènoïque, et ses sels d'addition à un acide ou à une base pharmaceutiquement acceptable.

17. Composé de formule (I) selon la revendication 1 qui est l'acide (E)-3-[5,6-bis(benzyloxy)-3-(6-quinolinyloxy)benzo[*b*]thiophène-2-yl]-2-(4-pyridinyl)-2-propènoïque, et ses sels d'addition à un acide ou à une base pharmaceutiquement acceptable.

18. Composé de formule (I) selon la revendication 1 qui est l'acide (E)-3-{5,6-bis(benzyloxy)-3-[(6-méthoxy-3-pyridinyl)oxy]benzo[*b*]thiophène-2-yl}-2-(4-pyridinyl)-2-propènoïque, et ses sels d'addition à un acide ou à une base pharmaceutiquement acceptable.

19. Composé de formule (I) selon la revendication 1 qui est l'acide (E)-3-{5,6-bis(benzyloxy)-3-[4-(4-pyridinyl)phénoxy-benzo[*b*]thiophène-2-yl}-2-(4-pyridinyl)-2-propènoïque, et ses sels d'addition à un acide ou à une base pharmaceutiquement acceptable.

20. Composé de formule (I) selon la revendication 1 qui est l'acide (E)-3-[5-benzyloxy-6-méthoxy-3-(3-pyridinyl)oxy-benzo[*b*]thiophène-2-yl]-2-(4-pyridinyl)-2-propènoïque, et ses sels d'addition à un acide ou à une base pharmaceutiquement acceptable.

21. Procédé de préparation des composés de formule (I) selon la revendication 1 **caractérisé en ce que** l'on utilise comme produit de départ un composé de formule (II): 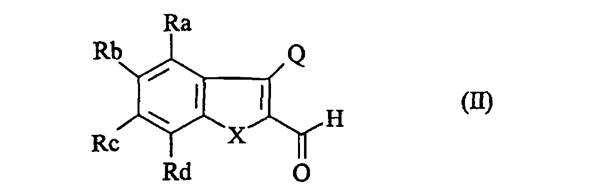 dans laquelle Ra, Rb, Rc, Rd et X ont la même signification que dans la formule (I) et Q représente un atome d'halogène ou un groupement hydroxy et de façon préférée, Q représente un atome d'halogène quand X représente un atome de soufre ou un groupement NR₃ avec R₃ tel que défini dans la formule (I) et Q représente un groupement hydroxy quand X représente un atome d'oxygène,
composé de formule (II) que l'on fait réagir, en condition basique,
- *soit quand Q représente un atome d'halogène :*
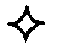 avec un composé de formule (III),
H - Y₁ - R₁ (III)
dans laquelle R₁ a la même signification que dans la formule (I) et Y₁ représente un atome d'oxygène, de soufre, ou un groupement NR₃ avec R₃ ayant la même signification que dans la formule (I),
pour conduire aux composés de formule (IV/a) : 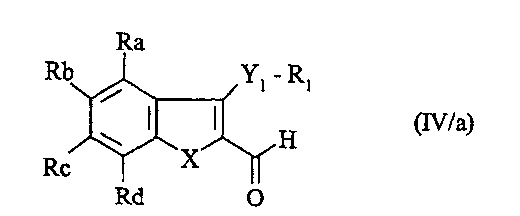 dans laquelle Ra, Rb, Rc, Rd, R₁, X et Y₁ sont tels que définis précédemment,
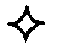 ou avec un composé de formule (V):
(HO)₂B - R₁ (V)
dans laquelle R₁ a la même signification que dans la formule (I),
pour conduire aux composés de formule (IV/b) 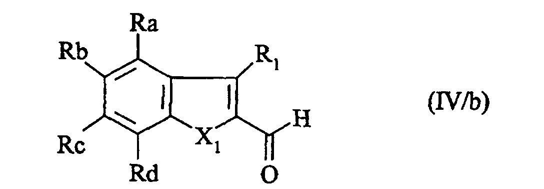 dans laquelle Ra, Rb, Rc, Rd et R₁ sont tels que définis précédemment et X₁ représente un groupement NR₃ dans lequel R₃ représente un groupement hétéroarylalkyle (C₁-C₆) linéaire ou ramifié,
- *soit quand Q représente un groupement hydroxy*, avec un composé de formule (VI),
Hal - R₁ (VI)
dans laquelle Hal représente un atome d'halogène et R₁ est tel que défini précédemment,
pour conduire aux composés de formule (IV/c) : 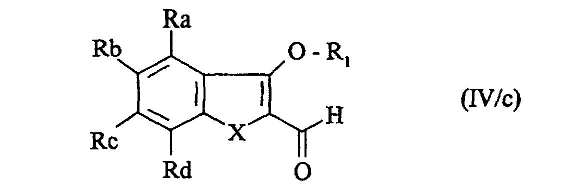 dans laquelle Ra, Rb, Rc, Rd, X et R₁ sont tels que définis précédemment,
l'ensemble des composés de formule (IV/a), (IV/b) et (IV/c) forment les composés de formule (IV): 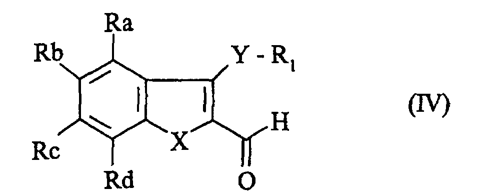 dans laquelle Ra, Rb, Rc, Rd, R₁, X et Y ont la même définition que dans la formule (I), composés de formule (IV) :
◆ que l'on condense, en présence d'anhydride acétique, avec un composé de formule (VII), 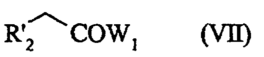 dans laquelle R'₂ a la même signification que R₂ dans la formule (I), excepté que R'₂ ne peut pas représenter un atome d'hydrogène, et W₁ représente un groupement alkoxy (C₁-C₆) linéaire ou ramifié, aryloxy, arylalkoxy (C₁-C₆) linéaire ou ramifié, cycloalkyloxy, un hétérocycle lié à un atome d'oxygène ou un groupement amino (lui-même pouvant être substitué par un ou deux groupements, identiques ou différents, indépendamment l'un de l'autre, choisis parmi alkyle (C₁-C₆) linéaire ou ramifié, aryle, arylalkyle (C₁-C₆) linéaire ou ramifié, ou cycloalkyle),
pour conduire aux composés de formule (I/a), cas particulier des composés de formule (I): 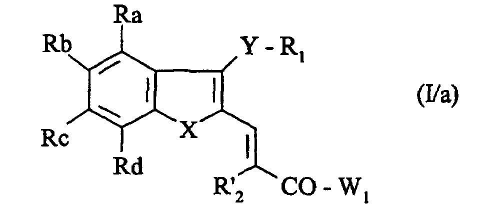 dans laquelle Ra, Rb, Rc, Rd, R₁, R'₂, X, Y et W₁ ont la même définition que précédemment, composés de formule (I/a) que l'on soumet, si on le désire :
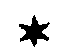 soit à des conditions d'hydrogénation catalytique, en présence de Palladium, pour conduire aux composés de formule (I/b), cas particulier des composés de formule (I): 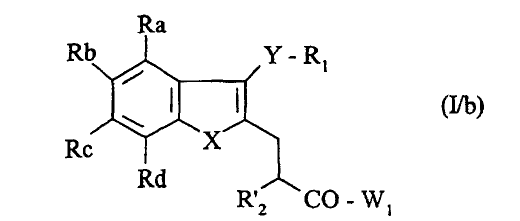 dans laquelle Ra, Rb, Rc, Rd, R₁, R'₂, X, Y et W₁ sont tels que définis précédemment,
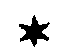 soit à des conditions d'hydrolyse, en milieu basique, pour conduire aux composés de formule (I/c), cas particulier des composés de formule (I): 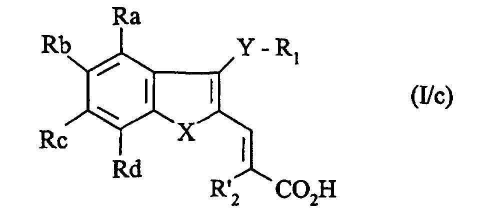 dans laquelle Ra, Rb, Rc, Rd, R₁, R'₂, X et Y ont la même définition que précédemment,
composés de formule (I/c), dont on réduit, si on le souhaite, la double liaison par hydrogénation catalytique, pour conduire aux composés de formule (I/d), cas particulier des composés de formule (I): 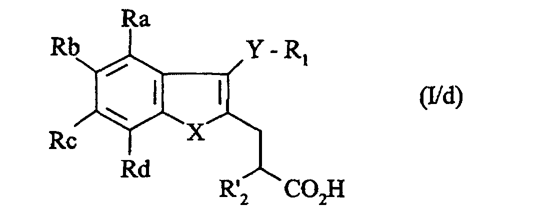 dans laquelle Ra, Rb, Rc, Rd, R₁, R'₂, X et Y sont tels que définis précédemment,
◆ ou que l'on soumet, à l'action d'un ylure de phosphore de formule (VIII), 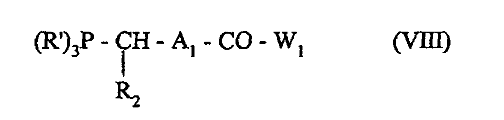 dans laquelle R' représente un groupement alkyle (C₁-C₆) linéaire ou ramifié, ou un groupement phényle, R₂ a la même définition que dans la formule (I), W₁ a la même définition que précédemment et A₁ représente une liaison simple, un groupement alkylène (éventuellement substitué par un ou plusieurs groupements alkyle (C₁-C₆) linéaire ou ramifié, aryle, arylalkyle (C₁-C₆) linéaire ou ramifié, cycloalkyle ou un hétérocycle), un groupement arylène, cycloalkylène ou un hétérocycle,
pour conduire aux composés de formule (I/e), cas particulier des composés de formule (I) : 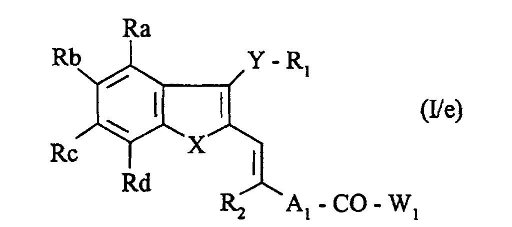 dans laquelle Ra, Rb, Rc, Rd, R₁, R₂, X, Y, A₁ et W₁ ont la même définition que précédemment,
composés de formule (I/e) que l'on soumet, si on le désire :
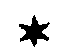 soit à des conditions d'hydrolyse, en condition basique, pour conduire aux composés de formule (I/f), cas particulier des composés de formule (I): 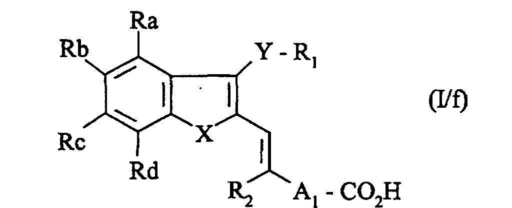 dans laquelle Ra, Rb, Rc, Rd, R₁, R₂, X, Y et A₁ sont tels que définis précédemment,
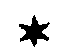 soit à des conditions d'hydrogénation catalytique, pour conduire aux composés de formule (I/g), cas particulier des composés de formule (I): 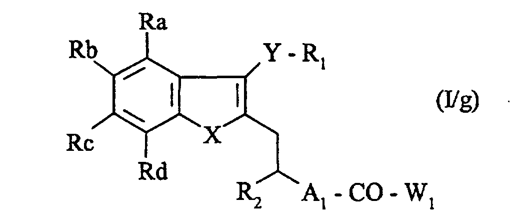 dans laquelle Ra, Rb, Rc, Rd, R₁, R₂, X, Y, A₁ et W₁ sont tels que définis précédemment, composés de formule (I/g) que l'on peut traiter dans des conditions d'hydrolyse basique, pour conduire aux composés de formule (I/h), cas particulier des composés de formule (I): 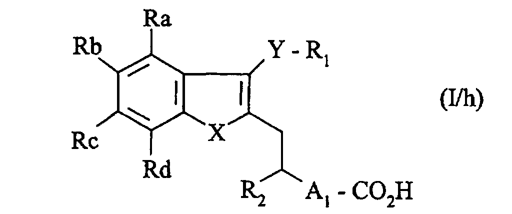 dans laquelle Ra, Rb, Rc, Rd, R₁, R₂, X, Y et A₁ sont tels que définis précédemment,
◆ ou, dont on réduit la fonction aldéhyde en alcool primaire, pour conduire aux composés de formule (IX) : 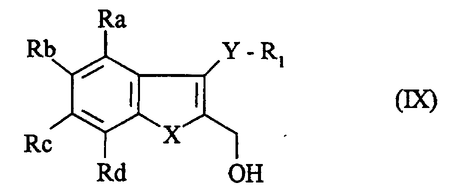 dans laquelle Ra, Rb, Rc, Rd, R₁, X et Y ont la même définition que dans la formule (I),
composés de formule (IX) dont on substitue l'hydroxy terminal par un halogène, selon des conditions classiques, pour conduire aux composés de formule (X) : 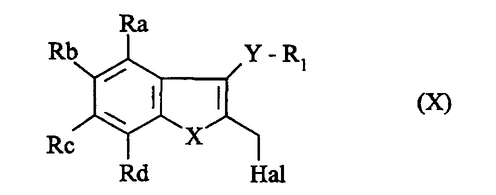 dans laquelle Ra, Rb, Rc, Rd, R₁, X et Y sont tels que définis précédemment, et Hal représente un atome de chlore ou de brome,
composés de formule (X):
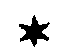 dont on substitue l'atome d'halogène, en condition basique, par un dérivé aminé de formule (XI):
R₂ - NH - A₁ - CO - W₁ (XI)
dans laquelle R₂ a la même définition que dans la formule (I) et, A₁, W₁ ont la même signification que précédemment,
pour conduire aux composés de formule (I/i), cas particulier des composés de formule (I) : 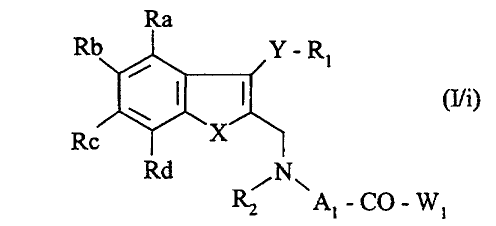 dans laquelle Ra, Rb, Rc, Rd, R₁, R₂, X, Y, A₁ et W₁ sont tels que définis précédemment,
composés de formule (I/i), dont on hydrolyse en condition basique le groupement carbonyle terminal, pour conduire aux composés de formule (I/j), cas particulier des composés de formule (I): 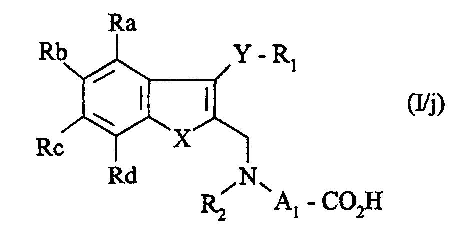 dans laquelle Ra, Rb, Rc, Rd, R₁, R₂, X, Y et A₁ sont tels que définis précédemment,
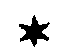 ou que l'on traite par de l'azidure de sodium dans un premier temps, et dont on réduit l'azide obtenue en amine primaire dans des conditions d'hydrogénation catalytique, pour conduire aux composés de formule (XII): 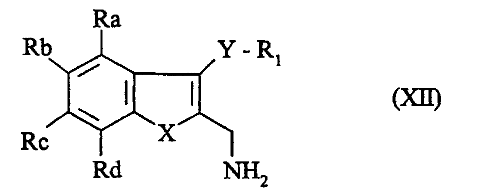 dans laquelle Ra, Rb, Rc, Rd, R₁, X et Y ont la même définition que dans la formule (I),
composés de formule (XII) que l'on condense, en condition basique, sur un dérivé chlorosulphonyle de formule (XIII) :
Cl - SO₂ - R₄ - CO - W₁ (XIII)
dans laquelle R₄ a la même définition que dans la formule (I), et W₁ est tel que défini précédemment, pour conduire aux composés de formule (I/k), cas particulier des composés de formule (I), 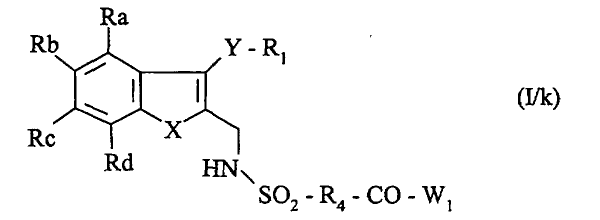 dans laquelle Ra, Rb, Rc, Rd, R₁, R₄, X, Y et W₁ sont tels que définis précédemment,
composés de formule (I/k):
- que l'on soumet, si on le souhaite, à des conditions d'hydrolyse en condition basique, pour conduire aux composés de formule (I/l), cas particulier des composés de formule (I) : 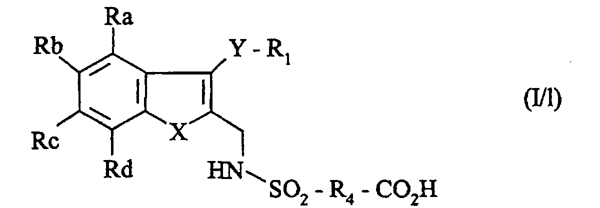 dans laquelle Ra, Rb, Rc, Rd, R₁, R₄, X et Y sont tels que définis précédemment,
- ou que l'on condense, en milieu basique, avec un composé de formule (XIV) :
Hal - R'₂ (XIV)
dans laquelle Hal représente un atome d'halogène tel que l'iode, et R'₂ a la même définition que précédemment,
pour conduire aux composés de formule (I/m), cas particulier des composés de formule (I): 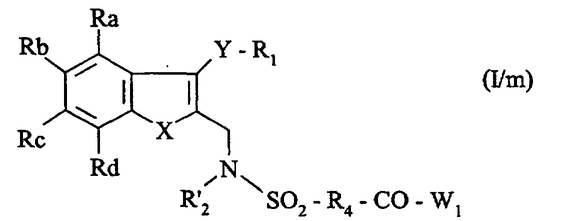 dans laquelle Ra, Rb, Rc, Rd, R₁, R'₂, R₄, X, Y et W₁ sont tels que définis précédemment,
composés de formule (I/m) que l'on traite par des conditions d'hydrolyse en milieu basique, pour conduire aux composés de formule (I/n), cas particulier des composés de formule (I): 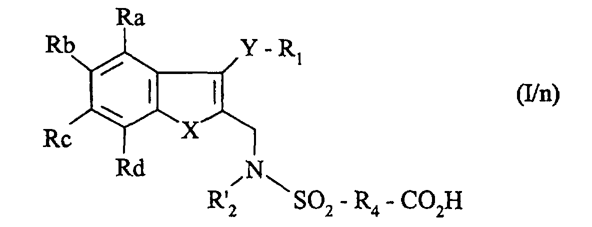 dans laquelle Ra, Rb, Rc, Rd, R₁, R'₂, R₄, X et Y sont tels que définis précédemment,
l'ensemble des composés de formule (I/c), (I/d), (I/f), (I/h), (I/j), (I/l) et (I/n) formant les composés de formule (I') : 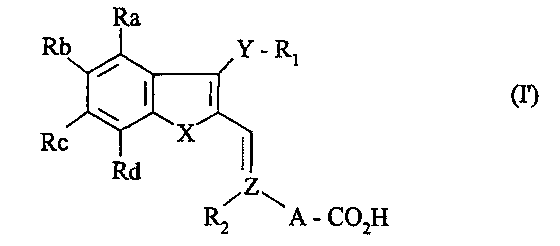 dans laquelle Ra, Rb, Rc, Rd, R₁, R₂, X, Y, Z et A sont tels que définis dans la formule (I), composés de formule (I') que l'on met en réaction avec une hydroxylamine-O-substituée, pour conduire, après déprotection de la fonction hydroxylamine, aux composés de formule (I/o), cas particulier des composés de formule (I): 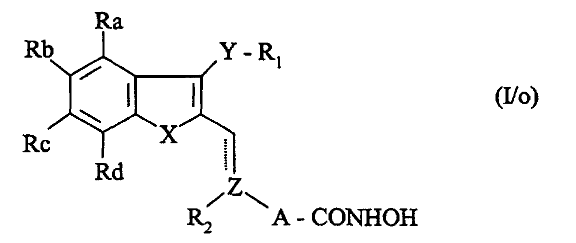 dans laquelle Ra, Rb, Rc, Rd, R₁, R₂, X, Y, Z et A sont tels que définis précédemment,
les composés (I/a) à (I/o) formant l'ensemble des composés de l'invention, que l'on purifie, le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

22. Compositions pharmaceutiques contenant comme principe actif au moins un composé de formule (I) selon l'une quelconque des revendications 1 à 20, seul ou en combinaison avec un ou plusieurs excipients ou véhicules inertes, non toxiques, pharmaceutiquement acceptable.

23. Compositions pharmaceutiques selon la revendication 22 contenant au moins un principe actif selon l'une quelconque des revendications 1 à 20, utiles dans le traitement de la thrombose, des pathologies dont l'origine est la thrombose et des pathologies entraînant une augmentation des risques thrombotiques.

## Patentansprüche

1. Verbindungen der Formel (I): 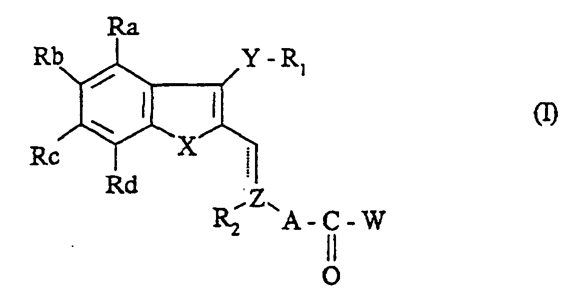 in der:
X ein Sauerstoffatom, ein Schwefelatom oder eine Gruppe NR₃ darstellt, worin R₃ ein Wasserstoffatom, eine geradkettige oder verzweigte (C₁-C₆)-Alkylgruppe, geradkettige oder verzweigte (C₁-C₆)-Acylgruppe, Arylgruppe, geradkettige oder verzweigte Aryl-(C₁-C₆)-alkylgruppe oder geradkettige oder verzweigte Heteroaryl-(C₁-C₆)-alkylgruppe bedeutet,
Y ein Sauerstoffatom, ein Schwefelatom oder eine Gruppe NR₃ darstellt, worin die Gruppe R₃ die oben angegebenen Bedeutungen besitzt, oder eine Einfachbindung bedeuten kann, wenn X eine Gruppe NR'₃ darstellt, worin R'₃ eine geradkettige oder verzweigte Heteroaryl-(C₁-C₆)-alkylgruppe bedeutet,
Z ein Stickstoffatom, wenn die Bindung, welche diese Gruppe mit dem benachbarten Kohlenstoffatom bindet, einfach (―) ist, ein Kohlenstoffatom oder eine Gruppe CH in Abhängigkeit davon, ob die Bindung, mit der diese Gruppe mit dem benachbarten Kohlenstoffatom verbunden ist, einfach (―) oder doppelt 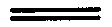 ) ist, bedeutet,
A eine Einfachbindung, eine (C₁-C₆)-Alkylengruppe, die gegebenenfalls durch eine oder mehrere Gruppen ausgewählt aus geradkettigem oder verzweigtem (C₁-C₆)-Alkyl, Aryl, geradkettigem oder verzweigtem Aryl-(C₁-C₆)-alkyl, Cycloalkyl und einem Heterocyclus substituiert ist, eine Arylengruppe, eine Cycloalkylengruppe, einen Heterocyclus oder eine Gruppe -SO₂-R₄- darstellt, worin R₄ eine geradkettige oder verzweigte (C₁-C₆)-Alkylengruppe, eine Arylengruppe, eine geradkettige oder verzweigte Aryl-(C₁-C₆)-alkylengruppe, eine Cycloalkylengruppe oder einen Heterocyclus bedeutet,
W eine Hydroxygruppe, eine geradkettige oder verzweigte (C₁-C₆)-Alkoxygruppe, eine Aryloxygruppe, eine geradkettige oder verzweigte Aryl-(C₁-C₆)-alkoxygruppe, eine Cycloalkyloxygruppe, einen Heterocyclus, der an ein Sauerstoffatom gebunden ist, eine Hydroxyaminogruppe oder eine Aminogruppe, welche ihrerseits durch eine oder zwei gleichartige oder verschiedenartige Gruppen substituiert sein kann, die unabhängig voneinander aus geradkettigem oder verzweigtem (C₁-C₆)-Alkyl, Aryl, geradkettigem oder verzweigtem Aryl-(C₁-C₆)-alkyl oder Cycloalkyl ausgewählt sind, bedeutet,
Ra, Rb, Rc und Rd, die gleichartig oder verschieden sind, unabhängig voneinander Wasserstoffatome, Halogenatome, Hydroxygruppen, Cyanogruppen. Nitrogruppen, geradkettige oder verzweigte (C₁-C₆)-Alkylgruppen, geradkettige oder verzweigte (C₁-C₆)-Alkoxygruppen, geradkettige oder verzweigte (C₁-C₆)-Acylgruppen, Carboxygruppen, geradkettige oder verzweigte (C₁-C₆)-Alkoxycarbonylgruppen, geradkettige oder verzweigte (C₁-C₆)-Trihalogenalkylgruppen, Aryloxygruppen, geradkettige oder verzweigte Aryl-(C₁-C₆)-alkoxygruppen, Heteroaryloxygruppen, geradkettige oder verzweigte Heteroaryl-(C₁-C₆)-alkoxygruppen oder Aminogruppen, die gegebenenfalls durch eine oder zwei gleichartige oder verschiedenartige Gruppen substituiert sind, die unabhängig voneinander aus geradkettigem oder verzweigtem (C₁-C₆)-Alkyl, Aryl, geradkettigem oder verzweigtem Aryl-(C₁-C₆)-alkyl ausgewählt sind, bedeuten,
oder Ra + Rb, Rb + Rc oder Rc + Rd eine Gruppe der Formel -U₁-V-U₂ bedeuten, worin:
* U₁ und U₂, die gleichartig oder verschieden sind, Sauerstoffatome, Schwefelatome oder Gruppen NH oder CH₂ bedeuten,
* V eine geradkettige (C₁-C₄)-Alkylengruppe, die gegebenenfalls durch eine oder mehrere geradkettige oder verzweigte (C₁-C₆)-Alkylgruppen, Arylgruppen, geradkettige oder verzweigte Aryl-(C₁-C₆)-alkylgruppen, Heteroarylgruppen, geradkettige oder verzweigte Heteroaryl-(C₁-C₆)-alkylgruppen substituiert ist, oder V eine Gruppe der Formel 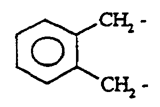 bedeutet,
wobei es sich versteht, daß Ra, Rb, Rc und Rd nicht gleichzeitig Wasserstoffatome bedeuten,
R₁:
- eine Arylgruppe, die durch eins bis fünf gleichartige oder verschiedene Substituenten substituiert ist, die unabhängig voneinander ausgewählt sind aus Halogen, Hydroxy, Cyano, Nitro, Carboxy, geradkettigem oder verzweigtem (C₁-C₆)-Alkyl, geradkettigem oder verzweigtem (C₁-C₆)-Alkoxy, geradkettigem oder verzweigtem (C₁-C₆)-Acyl, geradkettigem oder verzweigtem (C₁-C₆)-Alkoxycarbonyl, geradkettigem oder verzweigtem (C₁-C₆)-Trihalogenalkyl, welches gegebenenfalls durch eine Hydroxygruppe substituiert ist; geradkettigem oder verzweigtem Trihalogen-(C₁-C₆)-alkoxy, geradkettigem oder verzweigtem (C₁-C₆)-Aminoalkoxy, wobei der Aminorest durch eine oder zwei gleichartige oder verschiedene, geradkettige oder verzweigte (C₁-C₆)-Alkylgruppen substituiert sein kann; geradkettigem oder verzweigtem Alkoxycarbonyl-(C₁-C₆)-alkyl, geradkettigem oder verzweigtem (C₁-C₆)-Alkyl-carbonylamino, geradkettigem oder verzweigtem Aryl-(C₁-C₆)-alkyl, Aryloxy, geradkettigem oder verzweigtem Aryl-(C₁-C₆)-alkoxy, Arylamino, geradkettigem oder verzweigtem Aryl-(C₁-C₆)-alkylamino, Arylsulfanyl, geradkettigem oder verzweigtem Aryl-(C₁-C₆)-alkyl-sulfanyl, Heteroaryl, geradkettigem oder verzweigtem Heteroaryl-(C₁-C₆)-alkyl, Heteroaryloxy, geradkettigem oder verzweigtem Heteroaryl-(C₁-C₆)-alkoxy. Heteroarylamino, geradkettigem oder verzweigtem Heteroaryl-(C₁-C₆)-alkylamino, Heteroarylsulfanyl, geradkettigem oder verzweigtem Heteroaryl-(C₁-C₆)-alkylsulfanyl, Amino, welches gegebenenfalls durch eine oder zwei geradkettige oder verzweigte (C₁-C₆)-Alkylgruppen substituiert ist, wobei eine dieser Alkylgruppen gegebenenfalls durch eine Aminogruppe, eine geradkettige oder verzweigte (C₁-C₆)-Alkylaminogruppe oder eine geradkettige oder verzweigte (C₁-C₆)-Dialkylaminogruppe substituiert sein kann;
- eine Gruppe der Formel 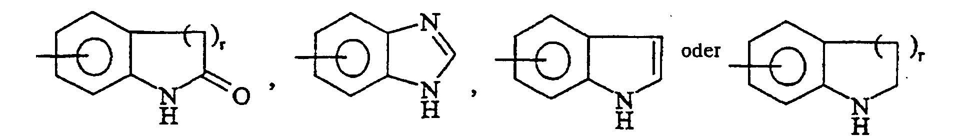 worin r eine ganze Zahl mit einem Wert zwischen 1 und 2 einschließlich darstellt,
- eine 1-Hydroxy-2(1*H*)-pyrdinongruppe oder
- eine gegebenenfalls substituierte Heteroarylgruppe bedeutet, und
R₂ ein Wasserstoffatom, eine geradkettige oder verzweigte (C₁-C₆)-Alkylgruppe, eine Arylgruppe, eine geradkettige oder verzweigte Aryl-(C₁-C₆)-Alkylgruppe, eine Cycloalkylgruppe, einen gegebenenfalls substituierten Heterocyclus oder einen Heterocyclus, der an eine geradkettige oder verzweigte (C₁-C₆)-Alkylgruppe gebunden ist, darstellt,
deren Isomere sowie deren Additionssalze mit einer pharmazeutisch annehmbaren Säure oder Base,
wobei es sich versteht, daß:
- unter einer Arylgruppe eine Phenyl-, Naphthyl-, Tetrahydronaphthyl- oder Dihydronaphthylgruppe zu verstehen ist, wobei jede dieser Gruppen gegebenenfalls in identischer oder verschiedenartiger Weise durch ein oder mehrere Halogenatome, Hydroxygruppen, Cyanogruppen, Nitrogruppen, geradkettige oder verzweigte (C₁-C₆)-Alkylgruppen, geradkettige oder verzweigte (C₁-C₆)-Trihalogenalkylgruppen, geradkettige oder verzweigte (C₁-C₆)-Alkoxygruppen, geradkettige oder verzweigte (C₁-C₆)-Acylgruppen, Carboxygruppen, geradkettige oder verzweigte (C₁-C₆)-Alkoxycarbonylgruppen, Aminogruppen, die gegebenenfalls durch eine oder zwei gleichartige oder verschiedene, geradkettige oder verzweigte (C₁-C₆)-Alkylgruppen substituiert sind, substituiert sein kann,
- unter einer Cycloalkylgruppe eine mono- oder bicyclische Gruppe zu verstehen ist, die 3 bis 8 Kohlenstoffatome aufweist,
- unter einem Heterocyclus eine gesättigte oder ungesättigte mono- oder bicyclische Gruppe zu verstehen ist mit aromatischem oder nichtaromatischem Kern und 5 bis 12 Kettengliedern, welche ein, zwei oder drei gleichartige oder verschiedene Heteroatome ausgewählt aus Sauerstoff, Stickstoff oder Schwefel enthält, wobei es sich versteht, daß der Heterocyclus gegebenenfalls in identischer oder verschiedenartiger Weise substituiert sein kann durch ein oder mehrere Halogenatome, Hydroxygruppen, geradkettige oder verzweigte (C₁-C₆)-Alkylgruppen, geradkettige oder verzweigte (C₁-C₆)-Trihalogenalkylgruppen, geradkettige oder verzweigte (C₁-C₆)-Alkoxygruppen, Aryloxygruppen, geradkettige oder verzweigte Aryl-(C₁-C₆)-alkoxygruppen, geradkettige oder verzweigte (C₁-C₆)-Acylgruppen geradkettige oder verzweigte (C₁-C₆)-Alkoxycarbonylgruppen, Nitrogruppen, Oxogruppen oder gegebenenfalls durch eine oder zwei geradkettige oder verzweigte (C₁-C₆)-Alkylgruppen substituierte Aminogruppen.
- genauer unter Heteroaryl ein Heterocyclus zu verstehen ist, wie er oben definiert ist, der gegebenenfalls substituiert ist, mono- oder bicyclisch ist und ungesättigt ist, wobei mindestens einer der Ringe einen aromatischen Charakter besitzt.

2. Verbindungen der Formel (I) nach Anspruch 1, **dadurch gekennzeichnet, daß** X ein Schwefelatom oder eine Gruppe NR₃ bedeutet, worin R₃ die bezüglich der Formel (I) angegebenen Bedeutungen besitzt, deren Isomere sowie deren Additionssalze mit einer pharmazeutisch annehmbaren Säure oder Base.

3. Verbindungen der Formel (I) nach Anspruch 1, **dadurch gekennzeichnet, daß** Y ein Sauerstoffatom bedeutet, deren Isomere sowie deren Additionssalze mit einer pharmazeutisch annehmbaren Säure oder Base.

4. Verbindungen der Formel (I) nach einem der Ansprüche 1 bis 3, **dadurch gekennzeichnet, daß** X ein Schwefelatom und Y ein Sauerstoffatom bedeuten, deren Isomere sowie deren Additionssalze mit einer pharmazeutisch annehmbaren Säure oder Base.

5. Verbindungen der Formel (I) nach Anspruch 1, **dadurch gekennzeichnet, daß** R₁ eine gegebenenfalls substituierte Phenylgruppe, eine gegebenenfalls substituierte Pyridinylgruppe oder eine gegebenenfalls substituierte Chinolylgruppe bedeutet, deren Isomere sowie deren Additionssalze mit einer pharmazeutisch annehmbaren Säure oder Base.

6. Verbindungen der Formel (I) nach Anspruch 1, **dadurch gekennzeichnet, daß** R₂ eine gegebenenfalls substituierte Arylgruppe oder einen gegebenenfalls substituierten Heterocyclus bedeutet, deren Isomere sowie deren Additionssalze mit einer pharmazeutisch annehmbaren Säure oder Base.

7. Verbindungen der Formel (I) nach einem der Ansprüche 1 bis 6, **dadurch gekennzeichnet, daß** R₂ eine Pyridinylgruppe bedeutet, deren Isomere sowie deren Additionssalze mit einer pharmazeutisch annehmbaren Säure oder Base.

8. Verbindungen der Formel (I) nach Anspruch 1, **dadurch gekennzeichnet, daß**:
- X ein Schwefelatom,
- Y ein Sauerstoffatom.
- R₁ eine gegebenenfalls substituierte Phenylgruppe oder eine gegebenenfalls substituierte Pyridinylgruppe und
- A eine Einfachbindung, wenn Z ein Kohlenstoffatom oder eine CH-Gruppe darstellt,
bedeuten, deren Isomere sowie deren Additionssalze mit einer pharmazeutisch annehmbaren Säure oder Base.

9. Verbindungen der Forme (I) nach Anspruch 1, **dadurch gekennzeichnet, daß**:
- X ein Schwefelatom,
- Y ein Sauerstoffatom,
- R₁ eine Phenylgruppe, die gegebenenfalls durch eine Gruppe, wie sie bezüglich der Formel (I) definiert worden ist, substituiert ist,
- A eine (C₁-C₆)-Alkylengruppe, die gegebenenfalls durch eine Gruppe ausgewählt aus geradkettigem oder verzweigtem (C₁-C₆)-Alkyl und geradkettigem oder verzweigtem Aryl-(C₁-C₆)-alkyl substituiert ist, oder eine Arylengruppe, wenn Z ein Stickstoffatom darstellt,
bedeuten, deren Isomere sowie deren Additionssalze mit einer pharmazeutisch annehmbaren Säure oder Base.

10. Verbindung der Formel (I) nach Anspruch 1, nämlich (E)-3-(5,6-Dimethoxy-3-(4-methoxyphenoxy)-benzo[*b*]thiophen-2-yl]-2-(4-pyridinyl)-2-propensäure und deren Additionssalze mit einer pharmazeutisch annehmbaren Säure oder Base.

11. Verbindung der Formel (I) nach Anspruch 1, nämlich (E)-3-[5,6-Bis(benzyloxy)-3-(4-methoxyphenoxy)-benzo[*b*]thiophen-2-yl]-2-(4-pyridinyl)-2-propensäure und deren Additionssalze mit einer pharmazeutisch annehmbaren Säure oder Base.

12. Verbindung der Formel (I) nach Anspruch 1, nämlich (E)-3-[5,6-Bis(benzyloxy)-3-(4-benzyloxyphenoxy)-benzo[*b*]thiophen-2-yl]-2-(4-pyridinyl)-2-propensäure und deren Additionssalze mit einer pharmazeutisch annehmbaren Säure oder Base.

13. Verbindun der Formel (I) nach Anspruch 1, nämlich (E)-3-[5,6-Bis(benzyloxy)-3-(3-pyridinyloxy)-benzo[*b*]thiophen-2-yl]-2-(4-pyridinyl)-2-propensäure und deren Additionssalze mit einer pharmazeutisch annehmbaren Säure oder Base.

14. Verbindung der Formel (I) nach Anspruch 1, nämlich (E)-3-[5,6-Bis(benzyloxy)-3-(4-hydroxyphenoxy)-benzo[*b*]thiophen-2-yl]-2-(4-pyridinyl)-2-propensäure und deren Additionssalze mit einer pharmazeutisch annehmbaren Säure oder Base.

15. Verbindung der Formel (I) nach Anspruch 1, nämlich (E)-3-{5,6-Bis(benzyloxy)-3-[(6-methyl-3-pyridinyl)-oxyl-benzo[*b*]thiophen-2-yl}-2-(4-pyridinyl)-2-propensäure und deren Additionssalze mit einer pharmazeutisch annehmbaren Säure oder Base.

16. Verbindung der Formel (I) nach Anspruch 1, nämlich (E)-3-{5-(Benzyloxy)-3-[4-(benzyloxy)-phenoxy]-6-methoxy-benzo[*b*]thiophen-2-yl}-2-(4-pyridinyl)-2-propensäure und deren Additionssalze mit einer pharmazeutisch annehmbaren Säure oder Base.

17. Verbindung der Formel (I) nach Anspruch 1, nämlich (E)-3-[5,6-Bis(benzyloxy)-3-(6-chinolinyloxy)-benzo[*b*]thiophen-2-yl]-2-(4-pyridinyl)-2-propensäure und deren Additionssalze mit einer pharmazeutisch annehmbaren Säure oder Base.

18. Verbindung der Formel (I) nach Anspruch 1, nämlich (E)-3-{5,6-Bis(benzyloxy)-3 -[(6-methoxy-3-pyridinyl)-oxy]-benzo[*b*]thiophen-2-yl}-2-(4-pyridinyl)-2-propensäure und deren Additionssalze mit einer pharmazeutisch annehmbaren Säure oder Base.

19. Verbindung der Formel (I) nach Anspruch 1, nämlich (E)-3-{5,6-Bis(benzyloxy)-3-[4-(4-pyridinyl)-phenoxy-benzo[*b*]thiophen-2-yl}-2-(4-pyridinyl)-2-propensäure und deren Additionssalze mit einer pharmazeutisch annehmbaren Säure oder Base.

20. Verbindung der Formel (I) nach Anspruch 1, nämlich (E)-3-[5-Benzyloxy-6-methoxy-3-(3-pyridinyl)-oxy-benzo[*b*]thiophen-2-yl]-2-(4-pyridinyl)-2-propensäure und deren Additionssalze mit einer pharmazeutisch annehmbaren Säure oder Base.

21. Verfahren zur Herstellung der Verbindungen der Formel (I) nach Anspruch 1, **dadurch gekennzeichnet, daß** man als Ausgangsprodukt eine Verbindung der Formel (II) verwendet: 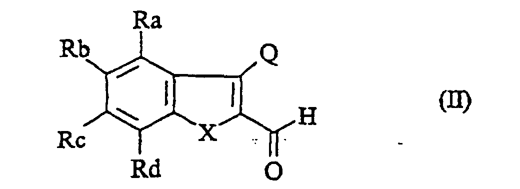 in der Ra, Rb, Rc, Rd und X die bezüglich der Formel (I) angegebenen Bedeutungen besitzen und Q ein Halogenatom oder eine Hydroxygruppe bedeutet und Q vorzugsweise ein Halogenatom bedeutet, wenn X ein Schwefelatom oder eine Gruppe NR₃ darstellt, worin R₃ die bezüglich der Formel (I) angegebenen Bedeutungen besitzt und Q eine Hydroxygruppe darstellt, wenn X ein Sauerstoffatom bedeutet,
welche Verbindung der Formel (II) man unter basischen Bedingungen
- *wenn Q ein Halogenatom bedeutet:*
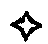 mit einer Verbindung der Formel (III) umsetzt:
H - Y₁ - R₁ (III)
in der R₁ die bezüglich der Formel (I) angegebenen Bedeutungen besitzt und Y₁ ein Sauerstoffatom, ein Schwefelatom oder eine Gruppe NR₃ bedeutet, worin R₃ die bezüglich der Formel (I) angegebenen Bedeutungen besitzt, zur Bildung der Verbindungen der Formel (IV/a): 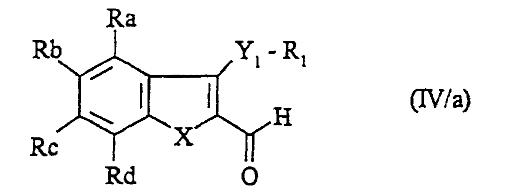 in der Ra, Rb, Rc, Rd, R₁, X und Y₁ die oben angegebenen Bedeutungen besitzen,
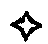 oder mit einer Verbindung der Formel (V) umsetzt:
(HO)₂B - R₁ (V)
in der R₁ die bezüglich der Formel (I) angegebenen Bedeutungen besitzt, zur Bildung der Verbindungen der Formel (IV/b): 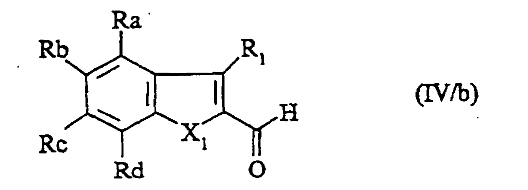 in der Ra, Rb, Rc, Rd und R₁ die oben angegebenen Bedeutungen besitzen und X₁ eine Gruppe NR₃ darstellt, worin R₃ eine geradkettige oder verzweigte Heteroaryl-(C₁-C₆)-alkylgruppe bedeutet,
- *oder, wenn Q eine Hydroxygruppe darstellt,* mit einer Verbindung der Formel (VI) umsetzt:
Hal - R₁ (VI)
in der Hal ein Halogenatom darstellt und R₁ die oben angegebenen Bedeutungen besitzt,
zur Bildung der Verbindungen der Formel (IV/c): 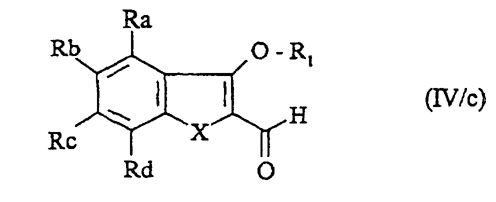 in der Ra, Rb, Rc, Rd, X und R₁ die oben angegebenen Bedeutungen besitzen, wobei die Gesamtheit der Verbindungen der Formeln (IV/a), (IV/b) und (IV/c) die Verbindungen der Formel (IV) bildet: 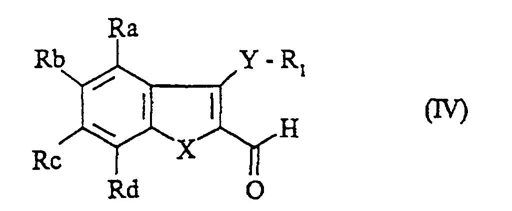 in der Ra, Rb, Rc, Rd, R₁, X und Y die bezüglich der Formel (I) angegebenen Bedeutungen besitzen,
welche Verbindungen der Formel (IV):
◆ man in Gegenwart von Essigsäureanhydrid mit einer Verbindung der Formel (VII) kondensiert: 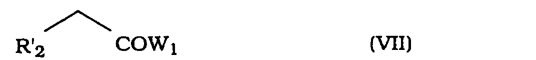 in der R'₂ die gleichen Bedeutungen wie R₂ bezüglich der Formel (I) besitzt mit der Ausnahme, daß R'₂ kein Wasserstoffatom bedeuten kann und W₁ eine geradkettige oder verzweigte (C₁-C₆)-Alkoxygruppe, Aryloxygruppe, geradkettige oder verzweigte Aryl-(C₁-C₆)-alkoxygruppe, Cycloalkyloxygruppe, einen an ein Sauerstoffatom gebundenen Heterocyclus oder eine Aminogruppe (die ihrerseits unabhängig voneinander durch eine oder zwei gleichartige oder verschiedene Gruppen ausgewählt aus geradkettigem oder verzweigtem (C₁-C₆)-Alkyl, Aryl, geradkettigem oder verzweigtem Aryl-(C₁-C₆)-alkyl oder Cycloalkyl substituiert ist) bedeutet,
zur Bildung der Verbindungen der Formel (I/a), einem Sonderfall der Verbindungen der Formel (I): 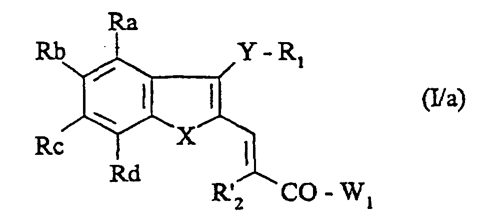 in der Ra, Rb, Rc, Rd, R₁, R'₂, X, Y und W₁ die oben angegebenen Bedeutungen besitzen,
welche Verbindungen der Formel (I/a) man gewünschtenfalls:
* entweder den Bedingungen der katalytischen Hydrierung in Gegenwart von Palladium unterwirft zur Bildung der Verbindungen der Formel (I/b), einem Sonderfall der Verbindungen der Formel (I): 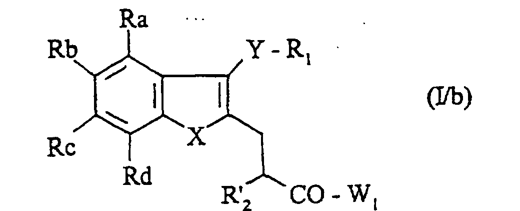 in der Ra, Rb, Rc, Rd, R₁, R'₂, X, Y und W₁ die oben angegebenen Bedeutungen besitzen,
* oder den Bedingungen der Hydrolyse in basischem Medium unterwirft zur Bildung der Verbindungen der Formel (I/c), einem Sonderfall der Verbindungen der Formel (I): 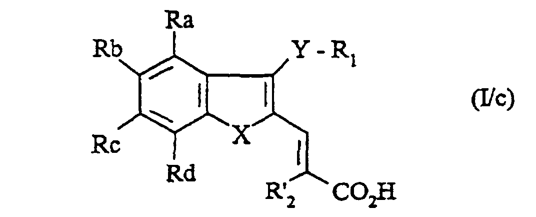 in der Ra, Rb, Rc, Rd, R₁, R'₂, X und Y die oben angegebenen Bedeutungen besitzen,
bei welchen Verbindungen der Formel (I/c) man gewünschtenfalls die Doppelbindung durch katalytische Hydrierung reduziert, zur Bildung der Verbindungen der Formel (I/d), einem Sonderfall der Verbindungen der Formel (I): 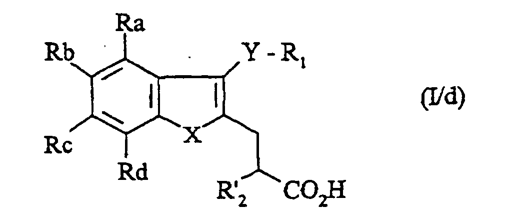 in der Ra, Rb, Rc, Rd, R₁, R'₂, X und Y die oben angegebenen Bedeutungen besitzen,
◆ oder man der Einwirkung eines Phosphorylids der Formel (VIII) unterwirft: 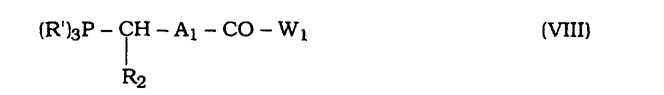 in der R' eine geradkettige oder verzweigte (C₁-C₆)-Alkylgruppe oder eine Phenylgruppe bedeutet, R₂ die bezüglich der Formel (I) angegebenen Bedeutungen besitzt, W₁ die oben angegebenen Bedeutungen besitzt und A₁ eine Einfachbindung, eine (gegebenenfalls durch eine oder mehrere, geradkettige oder verzweigte (C₁-C₆)-Alkylgruppen, Arylgruppen, geradkettige oder verzweigte Aryl-(C₁-C₆)-alkylgruppen, Cycloalkylgruppen oder Heterocyclen substituierte) Alkylengruppe, eine Arylengruppe, eine Cycloalkylengruppe oder einen Heterocyclus bedeutet,
zur Bildung der Verbindungen der Formel (I/e), einem Sonderfall der Verbindungen der Formel (I): 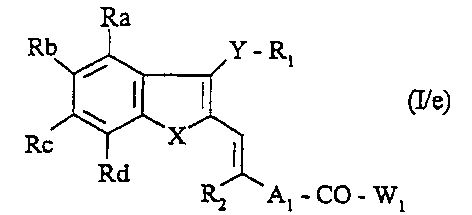 in der Ra, Rb, Rc, Rd, R₁, R₂, X, Y, A₁ und W₁ die oben angegebenen Bedeutungen besitzen,
welche Verbindungen der Formel (I/e) man gewünschtenfalls:
* entweder den Bedingungen der Hydrolyse unter basischen Bedingungen unterwirft zur Bildung der Verbindungen der Formel (I/f), einem Sonderfall der Verbindungen der Formel (I): 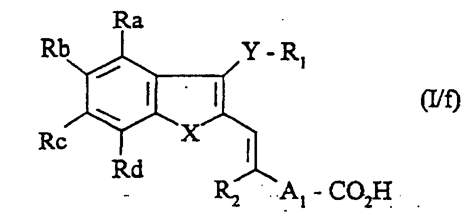 in der Ra, Rb, Rc, Rd, R₁, R₂, Y, X und A₁ die oben angegebenen Bedeutungen besitzen,
* oder den Bedingungen der katalytischen Hydrierung unterwirft zur Bildung der Verbindungen der Formel (I/g), einem Sonderfall der Verbindungen der Formel (I): 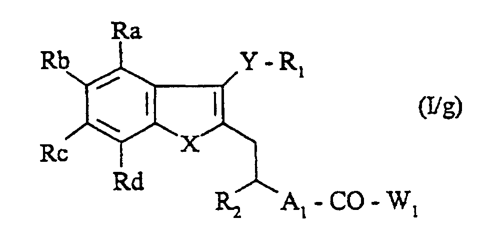 in der Ra, Rb, Rc, Rd, R₁, R₂, X, Y, A₁ und W₁ die oben angegebenen Bedeutungen besitzen,
welche Verbindungen der Formel (I/g) man unter den Bedingungen der basischen Hydrolyse behandeln kann, zur Bildung der Verbindungen der Formel (I/h), einem Sonderfall der Verbindungen der Formel (I): 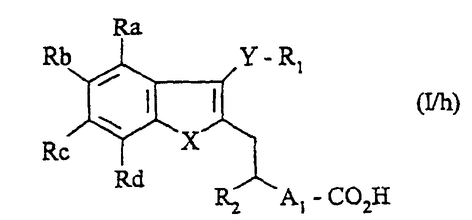 in der Ra, Rb, Rc, Rd, R₁, R₂, X, Y und A₁ die oben angegebenen Bedeutungen besitzen,
◆ oder deren Aldehydfunktion man mit einem primären Alkohol reduziert zur Bildung der Verbindungen der Formel (IX): 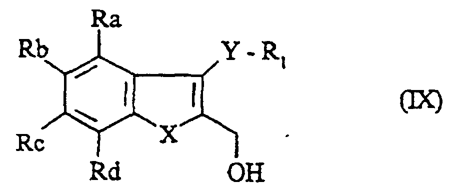 in der Ra, Rb, Rc, Rd, R₁, X und Y die bezüglich der Formel (I) angegebenen Bedeutungen besitzen,
bei welchen Verbindungen der Formel (IX) man die endständige Hydroxygruppe unter Anwendung klassischer Bedingungen durch ein Halogen substituiert, zur Bildung der Verbindungen der Formel (X): 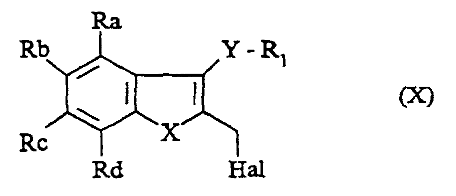 in der Ra, Rb, Rc, Rd, R₁, X und Y die oben angegebenen Bedeutungen besitzen und Hal ein Chlor- oder Bromatom bedeutet,
bei welchen Verbindungen der Formel (X):
* man das Halogenatom unter basischen Bedingungen mit einem Aminderivat der Formel (XI) substituiert:
R₂ - NH - A₁ - CO - W₁ (XI)
in der R₂ die bezüglich der Formel (I) angegebenen Bedeutungen besitzt und A₁ und W₁ die oben angegebenen Bedeutungen besitzen,
zur Bildung der Verbindungen der Formel (I/i), einem Sonderfall der Verbindungen der Formel (I): 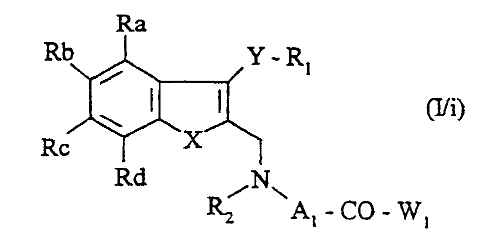 in der Ra, Rb, Rc, Rd, R₁, R₂, X, Y, A₁ und W₁ die oben angegebenen Bedeutungen besitzen,
bei welchen Verbindungen der Formel (I/i) man die endständige Carbonylgruppe unter basischen Bedingungen hydrolysiert, zur Bildung der Verbindungen der Formel (I/j), einem Sonderfall der Verbindungen der Formel (I): 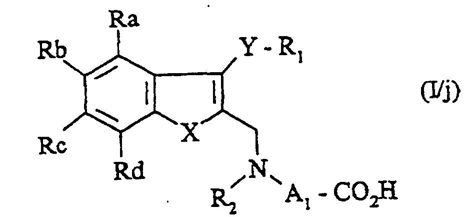 in der Ra, Rb, Rc, Rd, R₁, R₂, X, Y und A₁ die oben angegebenen Bedeutungen besitzen.
* oder welche man in einer ersten Stufe mit Natriumazid behandelt und das erhaltene Azid unter den Bedingungen der katalytischen Hydrierung zu einem primären Amin reduziert, zur Bildung der Verbindungen der Formel (XII): 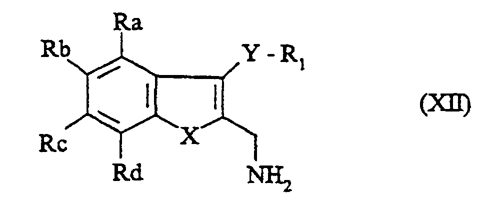 in der Ra, Rb, Rc. Rd, R₁, X und Y die bezüglich der Formel (I) angegebenen Bedeutungen besitzen,
welche Verbindungen der Formel (XII) man unter basischen Bedingungen mit einem Chlorsulfonylderivat der Formel (XIII) kondensiert:
Cl- SO₂ - R₄ - CO - W₁ (XIII)
in der R₄ die bezüglich der Formel (I) angegebenen Bedeutungen besitzt und W₁ die oben angegebenen Bedeutungen aufweist, zur Bildung der Verbindungen der Formel (I/k), einem Sonderfall der Verbindungen der Formel (I): 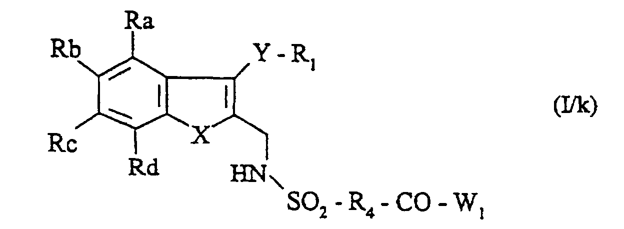 in der Ra. Rb, Rc, Rd, R₁, R₄, X, Y und W₁ die oben angegebenen Bedeutungen besitzen,
welche Verbindungen der Formel (I/k):
- man gewünschtenfalls den Bedingungen der Hydrolyse unter basischen Bedingungen unterwirft, zur Bildung der Verbindungen der Formel (I/l), einem Sonderfall der Verbindungen der Formel (I): 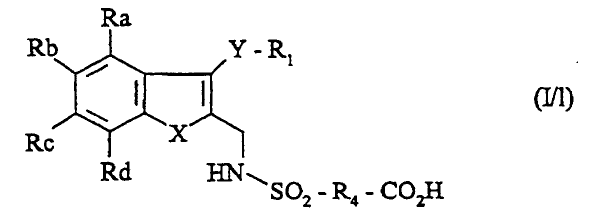 in der Ra, Rb, Rc, Rd, R₁, R₄, X und Y die oben angegebenen Bedeutungen besitzen,
- oder man in basischem Medium mit einer Verbindung der Formel (XIV) kondensiert:
Hal - R'₂ (XIV)
in der Hal ein Halogenatom, wie ein Iodatom bedeutet, und R'₂ die oben angegebenen Bedeutungen besitzt,
zur Bildung der Verbindungen der Formel (I/m), einem Sonderfall der Verbindungen der Formel (I): 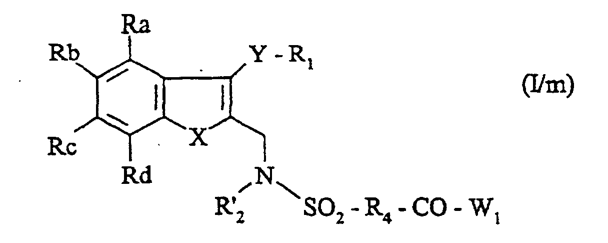 in der Ra, Rb, Rc, Rd, R₁, R'₂, R₄, X, Y und W₁ die oben angegebenen Bedeutungen besitzen,
welche Verbindungen der Formel (I/m) man in basischem Medium bei den Bedingungen der Hydrolyse behandelt, zur Bildung der Verbindungen der Formel (I/n), einem Sonderfall der Verbindungen der Formel (I): 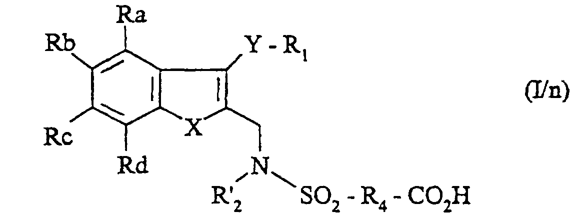 in der Ra, Rb, Rc, Rd, R₁, R'₂, R₄, X und Y die oben angegebenen Bedeutungen besitzen,
wobei die Gesamtheit der Verbindungen der Formeln (I/c), (I/d), (I/f), (I/h), (I/ j), (I/l) und (I/n) die Verbindungen der Formel (I') bilden: 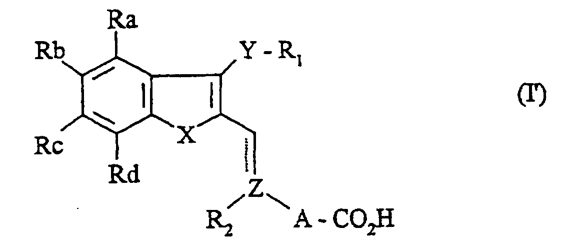 in der Ra, Rb, Rc, Rd, R₁, R₂, X, Y, Z und A die bezüglich der Formel (I) angegebenen Bedeutungen besitzen,
welche Verbindungen der Formel (I') man mit einem O-substituierten Hydroxylamin umsetzt, so daß man nach der Abspaltung der Schutzgruppe der Hydroxylaminfunktion die Verbindungen der Formel (I/o) erhält, einem Sonderfall der Verbindungen der Formel (I): 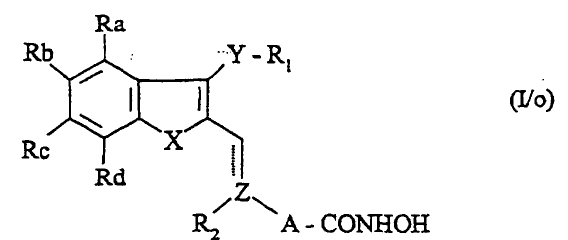 in der Ra, Rb, Rc, Rd, R₁, R₂, X, Y, Z und A die oben angegebenen Bedeutungen besitzen,
welche Verbindungen der Formeln (I/a) bis (I/o), welche die Gesamtheit der erfindungsgemäßen Verbindungen darstellen, man gewünschtenfalls mit Hilfe einer klassischen Reinigungsmethode reinigt, welche gewünschtenfalls mit Hilfe einer klassischen Trennmethode in ihre verschiedenen Isomeren aufgetrennt werden können und welche man gegebenenfalls mit einer pharmazeutisch annehmbaren Säure oder Base in ihre Additionssalze umwandeln kann.

22. Pharmazeutische Zubereitungen enthaltend als Wirkstoff mindestens eine Verbindung der Formel (I) nach einem der Ansprüche 1 bis 20, allein oder in Kombination mit einem oder mehreren inerten, nichttoxischen, pharmazeutisch annehmbaren Trägermaterialien oder Bindemitteln.

23. Pharmazeutische Zubereitungen nach Anspruch 22 enthaltend mindestens einen Wirkstoff nach einem der Ansprüche 1 bis 20, zur Behandlung von Thrombosen, pathologischen Zuständen mit der Thrombose als Ursprung und pathologischen Zuständen, die zu einer Erhöhung von Thromboserisiken führen.

## Claims

1. Compounds of formula (I) : 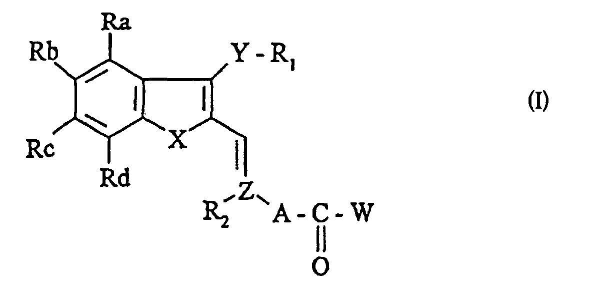 wherein:
X represents an oxygen atom, a sulphur atom, or an NR₃ group wherein R₃ represents a hydrogen atom, a linear or branched (C₁-C₆)alkyl group, a linear or branched (C₁-C₆)acyl group, an aryl group, an aryl-(C₁-C₆)alkyl group in which the alkyl moiety is linear or branched, or a heteroaryl-(C₁-C₆)alkyl group in which the alkyl moiety is linear or branched,
Y represents an oxygen atom, a sulphur atom, an NR₃ group, the R₃ group being as defined above, or may represent a single bond when X represents an NR'₃ group wherein R'₃ represents a heteroaryl-(C₁-C₆)alkyl group in which the alkyl moiety is linear or branched,
Z represents a nitrogen atom when the bond that links it to the adjacent carbon atom is single (―), a carbon atom or a CH group depending on whether the bond that links it to the adjacent carbon atom is single (―) or double ( 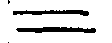 ),
A represents a single bond, a (C₁-C₆)alkylene group optionally substituted by one or more groups selected from linear or branched (C₁-C₆)alkyl, aryl, aryl-(C₁-C₆)alkyl in which the alkyl moiety is linear or branched, cycloalkyl and a heterocycle, an arylene group, a cycloalkylene group, a heterocycle, or an -SO₂-R₄- group wherein R₄ represents a linear or branched (C₁-C₆)alkylene group, an arylene group, an aryl-(C₁-C₆)alkylene group in which the alkylene moiety is linear or branched, a cycloalkylene group or a heterocycle,
W represents a hydroxy group, a linear or branched (C₁-C₆)alkoxy group, an aryloxy group, an aryl-(C₁-C₆)alkoxy group in which the alkoxy moiety is linear or branched, a cycloalkyloxy group, a heterocycle bonded to an oxygen atom, a hydroxyamino group, or an amino group which may itself be substituted by one or two identical or different groups, each independently of the other selected from linear or branched (C₁-C₆)alkyl, aryl, aryl-(C₁-C₆)-alkyl in which the alkyl moiety is linear or branched, and cycloalkyl,
Ra, Rb, Rc, Rd, which may be identical or different, each independently of the others represent a hydrogen atom, a halogen atom, a hydroxy group, a cyano group, a nitro group, a linear or branched (C₁-C₆)alkyl group, a linear or branched (C₁-C₆)alkoxy group, a linear or branched (C₁-C₆)acyl group, a carboxy group, a linear or branched (C₁-C₆)alkoxycarbonyl group, a linear or branched (C₁-C₆)trihaloalkyl group, an aryloxy group, an aryl-(C₁-C₆)alkoxy group in which the alkoxy moiety is linear or branched, a heteroaryloxy group, a heteroaryl-(C₁-C₆)alkoxy group in which the alkoxy moiety is linear or branched, or an amino group optionally substituted by one or two identical or different groups, each independently of the other selected from linear or branched (C₁-C₆)alkyl, aryl and aryl-(C₁-C₆)alkyl in which the alkyl moiety is linear or branched,
or Ra+Rb, Rb+Rc or Rc+Rd represents a grouping of formula -U₁-V-U₂ wherein :
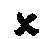 U₁ and U₂, which may be identical or different, represent an oxygen atom, a sulphur atom, or an NH or CH₂ group,
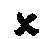 V represents a linear (C₁-C₄)alkylene group, optionally substituted by one or more groups: linear or branched (C₁-C₆)alkyl, aryl, aryl-(C₁-C₆)alkyl in which the alkyl moiety is linear or branched, heteroaryl, heteroaryl-(C₁-C₆)alkyl in which the alkyl moiety is linear or branched, or V represents
a grouping of formula 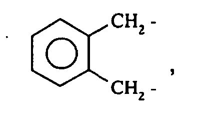
with the proviso that Ra, Rb, Rc and Rd cannot simultaneously represent a hydrogen atom,
R₁ represents :
- an aryl group substituted by from one to five identical or different substituents, each independently of the others selected from halogen, hydroxy, cyano, nitro, carboxy, linear or branched (C₁-C₆)alkyl, linear or branched (C₁-C₆)alkoxy, linear or branched (C₁-C₆)-acyl, linear or branched (C₁-C₆)alkoxy-carbonyl, trihalo-(C₁-C₆)alkyl (in which the alkyl moiety is linear or branched and is optionally substituted by a hydroxy group), trihalo-(C₁-C₆)alkoxy in which the alkoxy moiety is linear or branched, amino-(C₁-C₆)alkoxy in which the alkoxy moiety is linear or branched, the amino moiety of which may be substituted by one or two, identical or different, linear or branched (C₁-C₆)alkyl groups; (C₁-C₆)alkoxycarbonyl-(C₁-C₆)alkyl in which the alkoxy and alkyl moieties are each linear or branched, linear or branched (C₁-C₆)alkyl-carbonylamino, aryl-(C₁-C₆)alkyl in which the alkyl moiety is linear or branched, aryloxy, aryl-(C₁-C₆)alkoxy in which the alkoxy moiety is linear or branched, arylamino, aryl-(C₁-C₆)alkylamino in which the alkyl moiety is linear or branched, arylsulphanyl, aryl-(C₁-C₆)alkylsulphanyl in which the alkyl moiety is linear or branched, heteroaryl, heteroaryl-(C₁-C₆)alkyl in which the alkyl moiety is linear or branched, heteroaryloxy, heteroaryl-(C₁-C₆)alkoxy in which the alkoxy moiety is linear or branched, heteroarylamino, heteroaryl-(C₁-C₆)alkylamino in which the alkyl moiety is linear or branched, heteroarylsulphanyl, heteroaryl-(C₁-C₆)alkylsulphanyl in which the alkyl moiety is linear or branched, amino optionally substituted by one or two linear or branched (C₁-C₆)alkyl groups, one of which alkyl groups may be optionally substituted by an amino group, by a linear or branched (C₁-C₆)alkylamino group, or by a di-(C₁-C₆)-alkylamino group in which the alkyl moieties are each linear or branched,
- a group of formula, 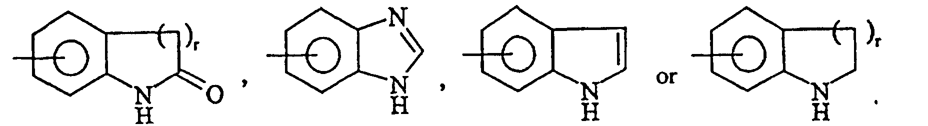 wherein r is an integer 1 or 2,
- a 1-hydroxy-2(1*H*)-pyridinone group, or
- an optionally substituted heteroaryl group,
R₂ represents a hydrogen atom, a linear or branched (C₁-C₆)alkyl group, an aryl group, an aryl-(C₁-C₆)alkyl group in which the alkyl moiety is linear or branched, a cycloalkyl group, an optionally substituted heterocycle or a heterocycle bonded to a linear or branched (C₁-C₆)-alkyl group,
their isomers and addition salts thereof with a pharmaceutically acceptable acid or base,
it being understood that:
- "aryl group" is understood to be a phenyl, naphthyl, tetrahydronaphthyl or dihydronaphthyl group, each of those groups being optionally substituted, identically or differently, by one or more substituents selected from halogen atoms and hydroxy, cyano, nitro, linear or branched (C₁-C₆)alkyl, linear or branched (C₁-C₆)trihaloalkyl, linear or branched (C₁-C₆)alkoxy, linear or branched (C₁-C₆)acyl, carboxy, linear or branched (C₁-C₆)alkoxycarbonyl and amino groups (amino optionally being substituted by one or two identical or different linear or branched (C₁-C₆)alkyl groups).
- "cycloalkyl group" is understood to be a mono- or bi-cyclic group, containing from 3 to 8 carbon atoms.
- "heterocycle" is understood to be a mono- or bi-cyclic, saturated or unsaturated, aromatic or non-aromatic, group having from 5 to 12 ring members, containing one, two or three identical or different hetero atoms selected from oxygen, nitrogen and sulphur, it being understood that the heterocycle may be optionally substituted, identically or differently, by one or more substituents selected from halogen atoms and hydroxy, linear or branched (C₁-C₆)alkyl, linear or branched (C₁-C₆)trihaloalkyl, linear or branched (C₁-C₆)alkoxy, aryloxy, aryl-(C₁-C₆)alkoxy in which the alkoxy moiety is linear or branched, linear or branched (C₁-C₆)acyl, linear or branched (C₁-C₆)-alkoxycarbonyl, nitro, oxo and amino groups (amino optionally being substituted by one or two linear or branched (C₁-C₆)alkyl groups),
- "heteroaryl" is understood, more precisely, to be an optionally substituted, unsaturated mono- or bi-cyclic heterocycle, as defined above, at least one of the rings of which is aromatic.

2. Compounds of formula (I) according to claim 1 **characterised in that** X represents a sulphur atom or an NR₃ group wherein R₃ is as defined for formula (I), their isomers and addition salts thereof with a pharmaceutically acceptable acid or base.

3. Compounds of formula (I) according to claim 1 **characterised in that** Y represents an oxygen atom, their isomers and addition salts thereof with a pharmaceutically acceptable acid or base.

4. Compounds of formula (I) according to any one of claims 1 to 3, **characterised in that** X represents a sulphur atom and Y represents an oxygen atom, their isomers and addition salts thereof with a pharmaceutically acceptable acid or base.

5. Compounds of formula (I) according to claim 1 **characterised in that** R₁ represents an optionally substituted phenyl group, an optionally substituted pyridyl group or an optionally substituted quinolyl group, their isomers and addition salts thereof with a pharmaceutically acceptable acid or base.

6. Compounds of formula (I) according to claim 1 **characterised in that** R₂ represents an optionally substituted aryl group or an optionally substituted heterocycle, their isomers and addition salts thereof with a pharmaceutically acceptable acid or base.

7. Compounds of formula (I) according to any one of claims 1 and 6, **characterised in that** R₂ represents a pyridyl group, their isomers and addition salts thereof with a pharmaceutically acceptable acid or base.

8. Compounds of formula (I) according to claim 1 **characterised in that** :
- X represents a sulphur atom,
- Y represents an oxygen atom,
- R₁ represents an optionally substituted phenyl group or an optionally substituted pyridyl group,
- A represents a single bond when Z represents a carbon atom or a CH group,
their isomers and addition salts thereof with a pharmaceutically acceptable acid or base.

9. Compounds of formula (I) according to claim 1 **characterised in that**:
- X represents a sulphur atom,
- Y represents an oxygen atom,
- R₁ represents a phenyl group optionally substituted by a group as defined for formula (I),
- A represents a (C₁-C₆)alkylene group optionally substituted by a group selected from linear or branched (C₁-C₆)alkyl, aryl and aryl-(C₁-C₆)alkyl in which the alkyl moiety is linear or branched, or an arylene group when Z represents a nitrogen atom,
their isomers and addition salts thereof with a pharmaceutically acceptable acid or base.

10. Compound of formula (I) according to claim 1 which is (E)-3-[5,6-dimethoxy-3-(4-methoxyphenoxy)benzo[*b*]thiophen-2-yl]-2-(4-pyridyl)-2-propenoic acid and its addition salts with a pharmaceutically acceptable acid or base.

11. Compound of formula (I) according to claim 1 which is (E)-3-[5,6-bis(benzyloxy)-3-(4-methoxyphenoxy)benzo[*b*]thiophen-2-yl]-2-(4-pyridyl)-2-propenoic acid and its addition salts with a pharmaceutically acceptable acid or base.

12. Compound of formula (I) according to claim 1 which is (E)-3-[5,6-bis(benzyloxy)-3-(4-benzyloxyphenoxy)benzo[*b*]thiophen-2-yl]-2-(4-pyridyl)-2-propenoic acid and its addition salts with a pharmaceutically acceptable acid or base.

13. Compound of formula (I) according to claim 1 which is (E)-3-[5,6-bis(benzyloxy)-3-(3-pyridyloxy)benzo[*b*]thiophen-2-yl]-2-(4-pyridyl)-2-propenoic acid and its addition salts with a pharmaceutically acceptable acid or base.

14. Compound of formula (I) according to claim 1 which is (E)-3-[5,6-bis(benzyloxy)-3-(4-hydroxyphenoxy)benzo[*b*]thiophen-2-yl]-2-(4-pyridyl)-2-propenoic acid and its addition salts with a pharmaceutically acceptable acid or base.

15. Compound of formula (I) according to claim 1 which is (E)-3-{5,6-bis(benzyloxy)-3-[(6-methyl-3-pyridyl)oxy]benzo[*b*]thiophen-2-yl}-2-(4-pyridyl)-2-propenoic acid and its addition salts with a pharmaceutically acceptable acid or base.

16. Compound of formula (I) according to claim 1 which is (E)-3-{5-(benzyloxy)-3-[4-(benzyloxy)phenoxy]-6-methoxy-benzo[*b*]thiophen-2-yl}-2-(4-pyridyl)-2-propenoic acid and its addition salts with a pharmaceutically acceptable acid or base.

17. Compound of formula (I) according to claim 1 which is (E)-3-[5,6-bis(benzyloxy)-3-(6-quinolyloxy)benzo[*b*]thiophen-2-yl]-2-(4-pyridyl)-2-propenoic acid and its addition salts with a pharmaceutically acceptable acid or base.

18. Compound of formula (I) according to claim 1 which is (E)-3-{5,6-bis(benzyloxy)-3-[(6-methoxy-3-pyridyl)oxy]benzo[*b*]thiophen-2-yl}-2-(4-pyridyl)-2-propenoic acid and its addition salts with a pharmaceutically acceptable acid or base.

19. Compound of formula (I) according to claim 1 which is (E)-3-{5,6-bis(benzyloxy)-3-[4-(4-pyridyl)phenoxy-benzo[*b*]thiophen-2-yl}-2-(4-pyridyl)-2-propenoic acid and its addition salts with a pharmaceutically acceptable acid or base.

20. Compound of formula (I) according to claim 1 which is (E)-3-[5-benzyloxy-6-methoxy-3-(3-pyridyl)oxy-benzo[b]thiophen-2-yl]-2-(4-pyridyl)-2-propenoic acid and its addition salts with a pharmaceutically acceptable acid or base.

21. Process for the preparation of compounds of formula (I) according to claim 1 **characterised in that** there is used as starting material a compound of formula (II) : 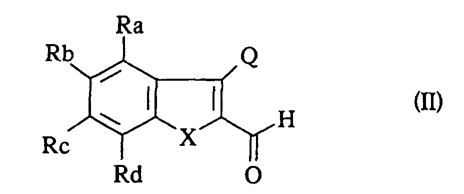 wherein Ra, Rb, Rc, Rd and X are as defined for formula (I) and Q represents a halogen atom or a hydroxy group and, preferably, Q represents a halogen atom when X represents a sulphur atom or an NR₃ group wherein R₃ is as defined for formula (I) and Q represents a hydroxy group when X represents an oxygen atom,
which compound of formula (II) is reacted, under basic conditions,
- *when Q represents a halogen atom :*
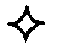 with a compound of formula (III),
H - Y₁ - R₁ (III)
wherein R₁ is as defined for formula (I) and Y₁ represents an oxygen atom, a sulphur atom, or an NR₃ group wherein R₃ is as defined for formula (I),
to yield the compounds of formula (IV/a) : 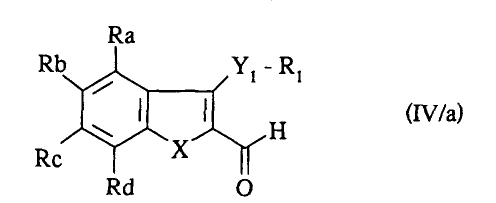 wherein Ra, Rb, Rc, Rd, R₁, X and Y₁ are as defined hereinabove,
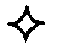 or with a compound of formula (V):
(HO)₂B - R₁ (V)
wherein R₁ is as defined for formula (I),
to yield the compounds of formula (IV/b) : 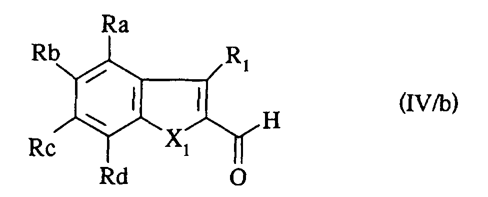 wherein Ra, Rb, Rc, Rd and R₁ are as defined hereinabove and X₁ represents an NR₃ group wherein R₃ represents a heteroaryl-(C₁-C₆)alkyl group in which the alkyl moiety is linear or branched,
- *when Q represents a hydroxy group,* with a compound of formula (VI),
Hal - R₁ (VI)
wherein Hal represents a halogen atom and R₁ is as defined hereinabove,
to yield the compounds of formula (IV/c) : 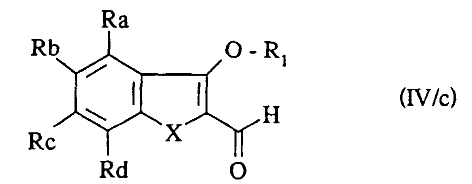 wherein Ra, Rb, Rc, Rd, X and R₁ are as defined hereinabove,
the totality of the compounds of formulae (IV/a), (IV/b) and (IV/c) constituting the compounds of formula (IV): 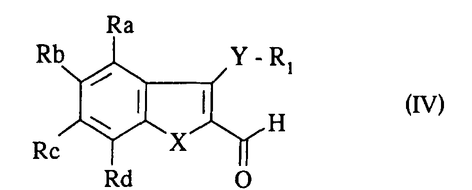 wherein Ra, Rb, Rc, Rd, R₁, X and Y are as defined for formula (I),
which compounds of formula (IV) :
◆ are condensed, in the presence of acetic anhydride, with a compound of formula (VII), 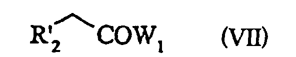 wherein R'₂ has the same definition as R₂ for formula (I), with the exception that R'₂ cannot represent a hydrogen atom, and W₁ represents a linear or branched (C₁-C₆)alkoxy group, an aryloxy group, an aryl-(C₁-C₆)alkoxy group in which the alkoxy moiety is linear or branched, a cycloalkyloxy group, a heterocycle bonded to an oxygen atom, or an amino group (which may itself be substituted by one or two identical or different groups, each independently of the other selected from linear or branched (C₁-C₆)alkyl, aryl, aryl-(C₁-C₆)alkyl in which the alkyl moiety is linear or branched, and cycloalkyl),
to yield the compounds of formula (I/a), a particular case of the compounds of formula (I) 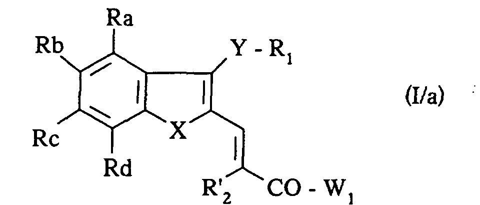 wherein Ra, Rb, Rc, Rd, R₁, R'₂, X, Y and W₁ are as defined hereinabove,
which compounds of formula (I/a) are, if desired, subjected to :
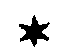 conditions of catalytic hydrogenation, in the presence of palladium, to yield the compounds of formula (I/b), a particular case of the compounds of formula (I): 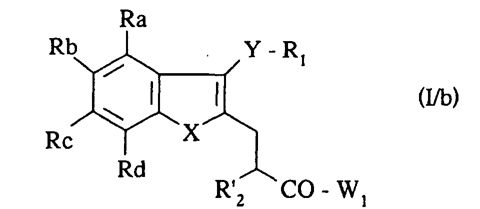 wherein Ra, Rb, Rc, Rd, R₁, R'₂, X, Y and W₁ are as defined hereinabove,
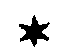 or to conditions of hydrolysis, in a basic medium, to yield the compounds of formula (I/c), a particular case of the compounds of formula (I) : 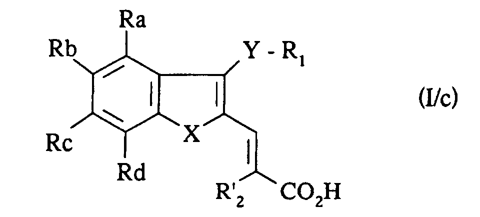 wherein Ra, Rb, Rc, Rd, R₁, R'₂, X and Y are as defined hereinabove,
the double bond of which compounds of formula (I/c) is, if desired, reduced by catalytic hydrogenation, to yield the compounds of formula (I/d), a particular case of the compounds of formula (I): 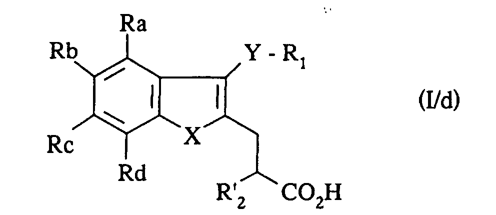 wherein Ra, Rb, Rc, Rd, R₁, R'₂, X and Y are as defined hereinabove,
◆ or are subjected to the action of a phosphorus ylid of formula (VIII), 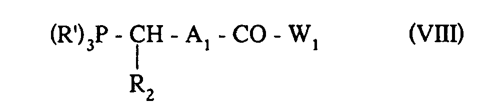 wherein R' represents a linear or branched (C₁-C₆)alkyl group, or a phenyl group, R₂ is as defined for formula (I), W₁ is as defined hereinabove and A₁ represents a single bond, an alkylene group (optionally substituted by one or more groups: linear or branched (C₁-C₆)-alkyl, aryl, aryl-(C₁-C₆)alkyl in which the alkyl moiety is linear or branched, cycloalkyl, or a heterocycle), an arylene group, a cycloalkylene group or a heterocycle,
to yield the compounds of formula (I/e), a particular case of the compounds of formula (I): 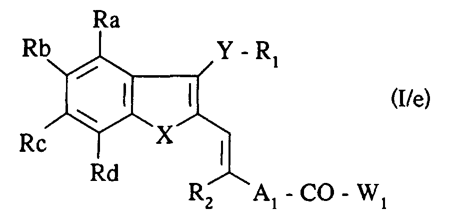 wherein Ra, Rb, Rc, Rd, R₁, R₂, X, Y, A₁ and W₁ are as defined hereinabove,
which compounds of formula (I/e) are, if desired, subjected to :
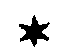 conditions of hydrolysis, under basic conditions, to yield the compounds of formula (I/f), a particular case of the compounds of formula (I): 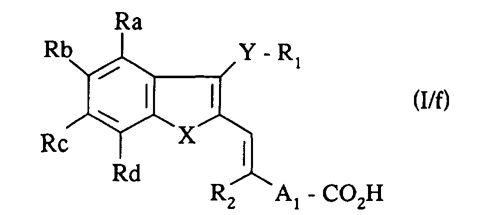 wherein Ra, Rb, Rc, Rd, R₁, R₂, X, Y and A₁ are as defined hereinabove,
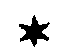 or to conditions of catalytic hydrogenation, to yield the compounds of formula (I/g), a particular case of the compounds of formula (I) : 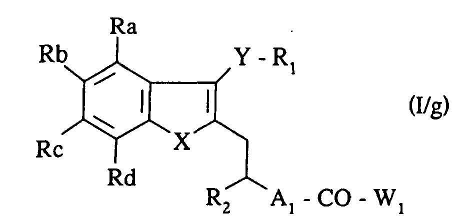 wherein Ra, Rb, Rc, Rd, R₁, R₂, X, Y, A₁ and W₁ are as defined hereinabove,
which compounds of formula (I/g) may be treated under conditions of basic hydrolysis to yield the compounds of formula (I/h), a particular case of the compounds of formula (I): 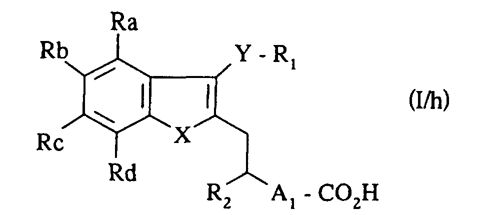 wherein Ra, Rb, Rc, Rd, R₁, R₂, X, Y and A₁ are as defined hereinabove,
◆ or the aldehyde function of which compounds of formula (IV) is reduced to the primary alcohol, to yield the compounds of formula (IX) : 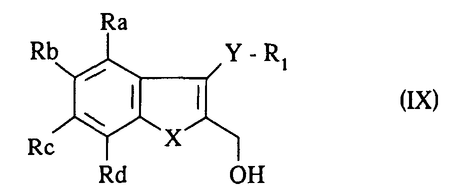 wherein Ra, Rb, Rc, Rd, R₁, X and Y are as defined for formula (I),
the terminal hydroxy of which compounds of formula (IX) is replaced by a halogen atom, according to conventional conditions, to yield the compounds of formula (X) : 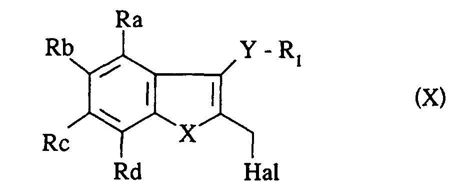 wherein Ra, Rb, Rc, Rd, R₁, X and Y are as defined hereinabove, and Hal represents a chlorine or bromine atom,
in which compounds of formula (X) :
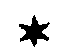 the halogen atom is replaced, under basic conditions, by an aminated compound of formula (XI) :
R₂ - NH - A₁ - CO - W₁ (XI)
wherein R₂ is as defined for formula (I) and A₁ and W₁ are as defined hereinabove, to yield the compounds of formula (I/i), a particular case of the compounds of formula (I): 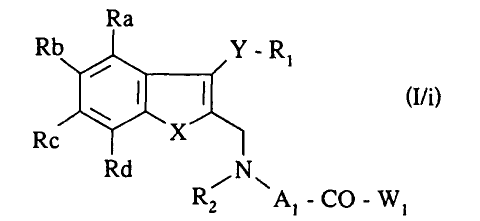 wherein Ra, Rb, Rc, Rd, R₁, R₂, X, Y, A₁ and W₁ are as defined hereinabove,
the terminal carbonyl group of which compounds of formula (I/i) is hydrolysed under basic conditions, to yield the compounds of formula (I/j), a particular case of the compounds of formula (I): 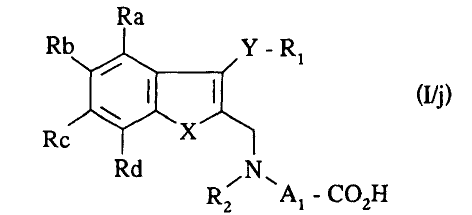 wherein Ra, Rb, Rc, Rd, R₁, R₂, X, Y and A₁ are as defined hereinabove,
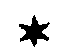 or which compounds of formula (X) are initially treated with sodium azide, the resulting azide being reduced to the primary amine under conditions of catalytic hydrogenation, to yield the compounds of formula (XII): 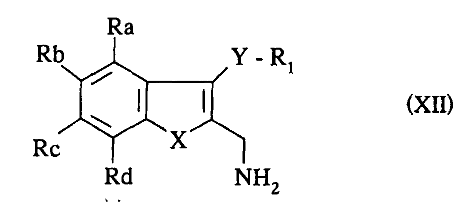 wherein Ra, Rb, Rc, Rd, R₁, X and Y are as defined for formula (I),
which compounds of formula (XII) are condensed, under basic conditions, with a chlorosulphonyl compound of formula (XIII):
Cl - SO₂ - R₄ - CO - W₁ (XIII)
wherein R₄ is as defined for formula (I), and W₁ is as defined hereinabove, to yield the compounds of formula (I/k), a particular case of the compounds of formula (I), 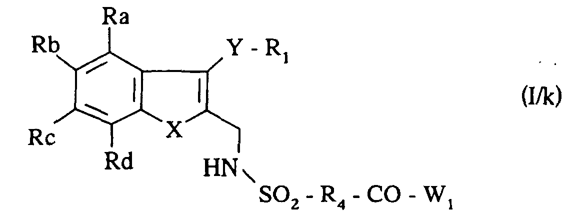 wherein Ra, Rb, Rc, Rd, R₁, R₄, X, Y and W₁ are as defined hereinabove,
which compounds of formula (I/k) :
- are subjected, if desired, to conditions of hydrolysis under basic conditions, to yield the compounds of formula (I/l), a particular case of the compounds of formula (I): 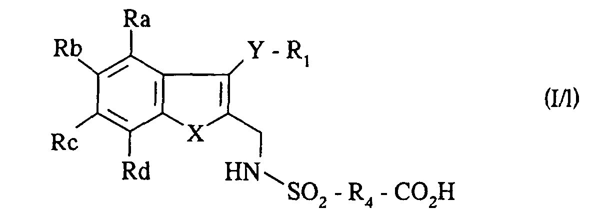 wherein Ra, Rb, Rc, Rd, R₁, R₄, X and Y are as defined hereinabove,
- or condensed, in a basic medium, with a compound of formula (XIV):
Hal - R'₂ (XIV)
wherein Hal represents a halogen atom, such as iodine, and R'₂ is as defined hereinabove, to yield the compounds of formula (I/m), a particular case of the compounds of formula (I): 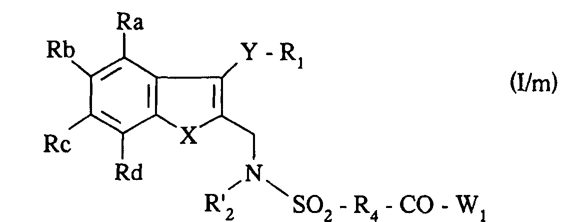 wherein Ra, Rb, Rc, Rd, R₁, R'₂, R₄, X, Y and W₁ are as defined hereinabove,
which compounds of formula (I/m) are treated by conditions of hydrolysis in a basic medium, to yield the compounds of formula (I/n), a particular case of the compounds of formula (I): 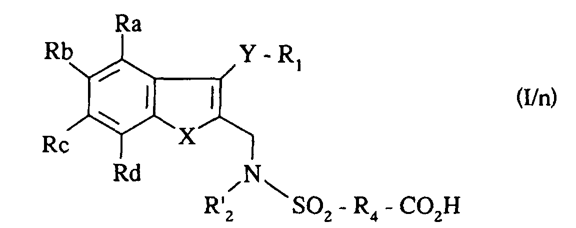 wherein Ra, Rb, Rc, Rd, R₁, R'₂, R₄, X and Y are as defined hereinabove,
the totality of the compounds of formulae (I/c), (I/d), (I/f), (I/h), (I/j), (I/l) and (I/n) constituting the compounds of formula (I'): 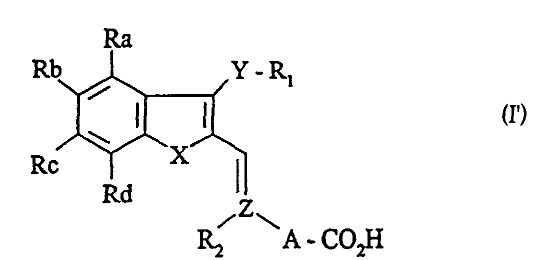 wherein Ra, Rb, Rc, Rd, R₁, R₂, X, Y, Z and A are as defined for formula (I),
which compounds of formula (I') are reacted with an O-substituted hydroxylamine, to yield, after deprotection of the hydroxylamine function, the compounds of formula (I/o), a particular case of the compounds of formula (I): 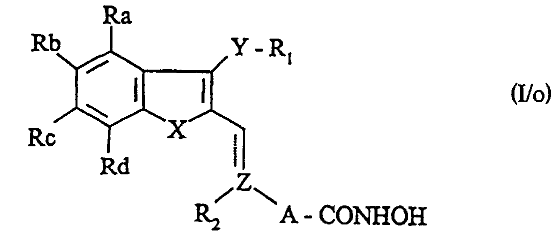 wherein Ra, Rb, Rc, Rd, R₁, R₂, X, Y, Z and A are as defined hereinabove,
the compounds (I/a) to (I/o) constituting the totality of the compounds of the invention, which are purified, if necessary, according to a conventional purification technique, which may be separated, if desired, into their different isomers according to a conventional separation technique, and which are converted, where appropriate, into addition salts thereof with a pharmaceutically acceptable acid or base.

22. Pharmaceutical compositions comprising as active ingredient at least one compound of formula (I) according to any one of claims 1 to 20, on its own or in combination with one or more inert, non-toxic pharmaceutically acceptable excipients or carriers.

23. Pharmaceutical compositions according to claim 22 comprising at least one active ingredient according to any one of claims 1 to 20, for use in the treatment of thrombosis, pathologies for which the origin is thrombosis and pathologies causing an increase in risk of thrombosis.
